# EUROPEAN PATENT APPLICATION

(11) **EP 4 778 943 A1**
(43) Date of publication of application: **22.07.2026**
(21) Application number: 24864665.5
(22) Date of filing: 11.09.2024
(51) Int. Cl.: C07K 19/00, A61K 39/395, A61K 38/29, C07K 16/24, A61P 19/10

(54) **LONG-ACTING PARATHYROID HORMONE RECEPTOR AGONIST FUSION PROTEIN AND USE THEREOF**

(30) Priority: 11.09.2023 CN 202311166451; 06.09.2024 CN 202411252600
(71) Applicant: Shanghai Btd-Bio Technology Co., Ltd., Shanghai 201506 (CN)
(72) Inventor: SONG, Liping, Shanghai 201318 (CN); ZHANG, Huanhuan, Shanghai 201318 (CN); HUANG, Xiaohui, Shanghai 201318 (CN); MU, Jun, Shanghai 201318 (CN); XU, Hong, Shanghai 201318 (CN); FAN, Yi, Shanghai 201318 (CN); WANG, Hongyan, Shanghai 201318 (CN)
(74) Representative: Dehns
(86) International application number: PCT/CN2024/118317
(87) International publication number: WO 2025/055951

(57) **Abstract**

A long-acting parathyroid hormone receptor agonist fusion protein and the use thereof. The fusion protein can inhibit the activity of RANKL and promote PTHR-mediated bone anabolism, and contains a first arm and a second arm. The first arm contains a functional region targeting RANKL and an Fc region, and the second arm contains a functional region capable of activating PTHR and an Fc region. The long-acting parathyroid hormone receptor agonist fusion protein has multiple advantages, *e.g*., better drug efficacy, longer lasting effect, better safety, higher structural stability and higher expression level, and the overall performance thereof is superior to that of an existing or potential treatment means, *e.g*., teriparatide, PEG-PTH, anti-RANKL monoclonal antibody, romosozumab, or the control sample synPTH-αRANKL-1.

## Description

The present application claims the priorities of Chinese patent application 2023111664518 filed on September 11, 2023, and Chinese patent application 2024112526007 filed on September 6, 2024. The contents of the Chinese patent applications are incorporated herein by reference in their entireties.

### TECHNICAL FIELD

The present disclosure belongs to the technical field of biomedicines, and particularly relates to a long-acting parathyroid hormone receptor agonist fusion protein and use thereof.

### BACKGROUND

Osteoporosis is a common degenerative skeletal disease, which is mainly characterized by bone loss and bone structure degeneration, and caused by an imbalance between bone formation of osteoblasts and bone resorption of osteoclasts (Dirckx N, Van Hul M, Maes C. Osteoblast recruitment to sites of bone formation in skeletal development, homeostasis, and regeneration. Birth Defects Res C Embryo Today. 2013; 99(3):170-91.). When the rate of bone resorption exceeds that of bone formation, it will lead to decreased bone mineral density and increased risk of fractures, thus inducing osteoporosis (Egermann M, Schneider E, Evans C H, et al, The potential of gene therapy for fracture union in osteoporosis [J]. Osteoporos Int, 2005, 16 (Suppl 2): S120-S128.).

Osteoporosis has been listed by the World Health Organization as the second most harmful disease to human health after cardiovascular diseases. Since the disease lacks warning signals, its first sign is fracture. Among people over 50 years old, an incidence proportion of osteoporotic fractures is as high as 19.2%. Within one year after a hip fracture occurs, 20% of people die from various complications, and about 50% of the survivors will be disabled. The osteoporotic fracture (also known as brittle fracture) is the most serious consequence of osteoporosis, which not only affects the life quality of patients, but also is one of the main causes of disability and death in elderly patients, causing a heavy burden on families and society.

Currently, the commonly used medicines for the treatment of osteoporosis in clinical practice include calcitonin, bisphosphonates, estrogen medicines and monoclonal antibodies against receptor activator of nuclear factor kappa-b ligand (RANKL). Although certain therapeutic effects have been achieved, these medicines can only inhibit bone resorption but cannot promote bone formation. Therefore, they can only delay the development of the disease but cannot cure the disease.

Most elderly patients with osteoporosis have very low bone metabolism and renewal rates. Many medicines for treating osteoporosis inhibit osteoclasts but actually have an inhibitory effect on bone metabolism or bone renewal. In terms of bone reconstruction and regeneration, female patients over 65 years old with osteoporotic vertebral fractures and BMD below -2.5SD, patients with multiple vertebral/hip fractures after menopause, patients who still have osteoporotic fractures after using medicines that inhibit bone absorption, and patients with multiple osteoporotic fractures should use more medicines that promote bone formation. There are two purposes: one is to effectively improve bone strength and prevent the next fracture; the other is to improve the quality of their bones, because some patients need surgery, and the quality of bones will affect the effect of surgical treatment.

In recent years, bone formation promoters have received extensive attention. Parathyroid hormone (PTH) is a peptide having 84 amino acids, which plays an important role in bone remodeling. The binding of PTH to parathyroid hormone receptor (PTHR) directly induces bone formation by activating cAMP and a classical wnt signaling pathway. Teriparatide (rhPTH (1-34)) is a commercially available therapeutic PTH peptide, which is a fragment of N-terminal 34 amino acids of mature human PTH. It has been shown to increase bone formation activity in patients with osteoporosis and is approved by the FDA for the treatment of primary osteoporosis, hypogonadal osteoporosis, and postmenopausal osteoporosis. Intermittent use of teriparatide at a low dose can achieve the effect of promoting bone formation and increasing bone mass, but daily subcutaneous injection of the medicine causes a lot of inconvenience to patients, so compliance of the patients is poor. In order to solve the problem of poor compliance with teriparatide, some research institutions have constructed PEG-PTH (1-34) to be expected to prolong the half-life of teriparatide. Although *in vitro* studies have shown that the activity of PEG-PTH (1-34) is equivalent to that of teriparatide, *in vivo* efficacy data show that the effect of PEG-PTH (1-34) in promoting bone formation significantly becomes worse. (Valderrama P, Jung R.E., Thoma D.S., et al. Evaluation of parathyroid hormone bound to a synthetic matrix for guided bone regeneration around dental implants: a histomorphometric study in dogs[J]. Journal of Periodontology, 2010, 81(5):737-747.DOI:10.1902/jop.2010. 090562.). In addition, if teriparatide is used continuously at a high dose for treatment, although it can promote bone formation, it will also cause the problem of bone loss. Therefore, it is not suitable for continuous blood drug exposure treatment. On the one hand, parathyroid hormone promotes the differentiation of bone marrow stem cells into osteoblasts, promotes bone formation, increases bone mass, and thus improves bone biomechanical properties (Friedl G, Turner RT, Evans GL, et al. Intermittent parathyroid hormone (PTH) treatment and age-dependent effects on rat cancellous bone and mineral metabolism[J]. J Orthop Res, 2007, 25:1454-1464), and on the other hand, it plays an important role in maintaining the metabolic balance of calcium and phosphorus in the body. Specifically, parathyroid hormone enhances the activity of osteoclasts to promote bone resorption and increase a serum calcium level; acts on the kidney to increase the serum calcium level and decrease a serum phosphorus level; and activates renal 1α-hydroxylase to indirectly promote the intestinal reabsorption of calcium and phosphorus, thereby increasing both serum calcium and serum phosphorus levels. Studies have found that the use of PTH (1-34) drugs (such as teriparatide) may induce side effects such as dizziness, nausea, back pain and lower limb cramps in human bodies (Effect of parathyroid hormone (1-34) on fractures and bone mineral density in postmenopausal women with osteoporosis. N Engl J Med 2001; 344:1434-41; The effect of teriparatide [human parathyroid hormone (1-34)] therapy on bone mineral density in men with osteoporosis. J Bone Miner Res 2003; 18:9-17.), and the occurrence of these side effects are associated with serum calcium and serum phosphorus levels.

Currently, the only approved drug on the market that can inhibit bone resorption while promoting bone formation is Romosozumab. Romosozumab is a humanized monoclonal antibody against sclerostin. However, this product has a Black Box Warning from the FDA, indicating that it is contraindicated for patients with a history of myocardial infarction or stroke within one year, and its maximum duration of use is limited to one year. Additionally, no combination therapy capable of simultaneously inhibiting bone resorption and promoting bone formation has yet been approved for market release. Although neutralizing antibodies targeting RANKL and therapeutic PTH peptides are both well known, and the co-administration of anti-RANKL monoclonal antibodies with therapeutic PTH peptides may be useful for patients with low bone mass conditions such as osteopenia, but the problem of poor patient compliance cannot be avoided; on the other hand, while co-formulations involving the simultaneous administration of multiple drugs may offer convenience, finding suitable formulation conditions is often highly challenging or even unattainable. Moreover, both co-administration and co-formulation involve additional costs related to additive development, manufacturing, and regulatory compliance for each individual drug. AMGEN Inc. previously attempted to develop a fusion compound (synPTH-α RANKL-1, which fuses rhPTH 1-34 to the N-terminus of the heavy chain of denosumab). When the inventors used conventional methods to prepare synPTH-α RANKL-1, they unexpectedly found that a large number of broken fragments of PTH functional region existed in the synPTH-α RANKL-1 product. This indicates low yield and poor stability during molecular preparation process, ultimately resulting in high preparation costs.

There are other bone disorders needing to be treated by regulating bone formation and/or bone resorption of the bones at lesion-associated sites, such as osteopenia, osteogenesis imperfecta, transplantation-associated bone loss, autoimmune-induced bone loss, disuse-induced bone loss, bone injuries, degenerative lumbar spondylolisthesis, or degenerative intervertebral disc diseases. For the bone injuries, for example bone fractures (especially osteoporotic fractures, fracture nonunion, and delayed fracture union), bone fissures, cartilage injury, osteonecrosis, and bone injuries caused by arthritis (such as rheumatoid arthritis, osteoarthritis, infectious arthritis, or other arthritis resulting from degeneration, trauma, overuse, infection, or autoimmune diseases) or tumors (such as bone tumors and tumor bone metastasis), the current commonly used treatment means often has the defects of poor patient compliance, suboptimal therapeutic efficacy, many side effects or the like, and there are no good treatment drugs in clinical practice.

Therefore, there is an urgent need to develop a novel product capable of inhibiting bone resorption and promoting bone formation.

### SUMMARY

The technical problems existing in the prior art include: the anti-RANKL-PTH fusion protein has poor structural stability, teriparatide has poor compliance, long-acting PTH (such as PEG-PTH1-34 or palopegteriparatide) may have the risk of hypercalcemia and/or catabolic effects, the anti-sclerostin antibody romosozumab inhibiting bone resorption and promoting bone formation has a maximum use period of 1 year and is contraindicated for patients with a history of myocardial infarction or stroke within one year, the existing marketed drugs are not effective in rapidly increasing bone mineral density in the short term, and anti-RANKL monoclonal antibodies have side effects such as mandibular osteonecrosis. Regarding the above-mentioned technical problems, the fusion protein (long-acting PTHR agonist fusion protein, LAPAF for short) developed in the present disclosure can inhibit not only bone resorption but also promote bone formation through a new design, and can quickly increase bone mineral density to a higher level in a short period of time (such as 2 months); the fusion protein has a long-acting effect but high safety, no risk of hypercalcemia, no catabolic effects, and a low risk of mandibular osteonecrosis, and is expected to be used for a long time; meanwhile, the fusion protein has a stable structure, a high preparation yield, and a low preparation cost.

The present disclosure attempts a variety of fusion structures. Compared with the reported fusion structures of anti-RANKL antibodies and PTH (such as US 8,992,925 B2 and US11,492,386 B2), the present disclosure selects a completely different fusion protein structure *(i.e.,* structure 1 of the present disclosure). A single molecule of this structure contains only one RANKL binding site and one PTHR binding site, and its molecular weight is lower than those of traditional monoclonal antibodies, so it is more conducive to the distribution of drugs in the body. At the same time, when LAPAF is prepared by using the conventional means, the breakage ratio of the PTH functional region is less than 25%, and the preferred molecular breakage ratio can even be lower than 20%, which is much lower than breakage ratio of the control sample synPTH-α RANKL-1 (breakage ratio 50%). At the same time, by adjusting anti-RANKL functional region of LAPAF, or adjusting linker, or adjusting length (from the shortest 11 amino acids to the longest 84 amino acids) or type (PTH or PTHrP) of the PTH functional region, or adjusting Fc region, LAPAF can maintain high structural stability and have a high expression level.

*In vitro* activity data shows that the proliferation inhibitory activity of LAPAF on Raw264.7 cells subjected to differentiation induced by RANKL is comparable to those of anti-RANKL monoclonal antibody and control sample synPTH-α RANKL-1, indicating that LAPAF has a good activity in inhibiting bone resorption. Meanwhile, LAPAF has a stronger *in vitro* activity than teriparatide on PTHR-induced cAMP release in cells expressing RANKL and PTHR (RANKL⁺/PTHR⁺ cells) while simulating osteoblasts, and the *in vitro* activity of the control sample synPTH-α RANKL-1 on PTHR-induced cAMP release in RANKL⁺/PTHR⁺ cells is only comparable to that of teriparatide. At the same time, by adjusting anti-RANKL functional region of LAPAF, or the linker, or the length or type (PTH or PTHrP) of the PTH functional region, or the Fc region, LAPAF can maintain superior activity on RANKL⁺/PTHR⁺ cells to teriparatide and synPTH-α RANKL-1. Therefore, LAPAF can enhance the effect of drugs on osteoblasts.

The results of *in vivo* efficacy studies show that in a RANKL-humanized transgenic mouse osteoporosis model induced by ovariectomy, LAPAF significantly increases bone mineral density on D56 after a single administration compared with control sample synPTH-α RANKL-1. On D56 after the single administration, LAPAF can still significantly down-regulate the content of TRAP5b, indicating that it can continuously inhibit the activity of osteoclasts, while the control sample synPTH-α RANKL-1 is basically ineffective; at the same time, on D56 after the single administration, LAPAF can also significantly up-regulate osteocalcin, indicating that it can continuously activate the activity of osteoblasts, while the control sample synPTH-α RANKL-1 is basically ineffective.

Literature reports that continuous infusion of human PTH (1-34) or Abaloparatide 80 µg/kg BW/day for 2 weeks can maintain strong bone formation activity in mice, but at the same time, the activity of osteoclasts is also significantly enhanced, which ultimately leads to a significant decrease in bone mineral density in mice, that is, the phenomenon of catabolic effects occurs (Le Henaff C, Finnie B, Pacheco M, He Z, Johnson J, Partridge NC. Abaloparatide Has the Same Catabolic Effects on Bones of Mice When Infused as PTH (1-34). JBMR Plus. 2023 Jan 5;7(2):e10710. doi: 10.1002/jbm4.10710.). Similarly, preclinical studies on TransCon PTH (palopegteriparatide) have also reported the phenomenon of catabolic effects. In a TPTx (parathyroidectomy) rat model, TransCon PTH was administered daily at a dose of 1.2 nmol/kg for a low dose group and at a dose of 2.4 nmol/kg for a high dose group. After 4 weeks, the bone mineral density was significantly decreased compared with that of model group (Holten-Andersen L, Pihl S, Rasmussen CE, et al. Design and Preclinical Development of TransCon PTH, an investigational Sustained -Release PTH Replacement Therapy for Hypoparathyroidism J Bone Miner Res. 2019 Nov;34(11):2075-2086. DOI:10.1002/jbmr.3824). Although sustained and highly active blood drug exposure of LAPAF was observed in a single administration experiment in the RANKL-humanized transgenic mouse osteoporosis model caused by ovariectomy (on D56, the blood sample of the 10 mpk dose group after being diluted 10 times still had an *in vitro* activity level equivalent to that of LYN101-2 at a concentration of 1 nM on PTHR-induced cAMP release in RANKL⁺/PTHR⁺ cells), the bone mineral density still showed a better improvement effect, bone resorption markers were still in a down-regulated state, and there were no phenomena of bone mineral density decrease or catabolic effects, the conditions were completely different from the above report.

Furthermore, in the same RANKL-humanized transgenic mouse osteoporosis model induced by ovariectomy, the present disclosure increases the administration frequency, adopts a weekly administration interval, and performs continuous administration for four weeks to further increase the blood drug exposure of LAPAF. At the same time, a combined medication control group of anti-RANKL monoclonal antibody and teriparatide is set up. The results show that LAPAF can still significantly down-regulate CTX-1, indicating that it can continue to significantly inhibit the activity of osteoclasts, and can significantly up-regulate osteocalcin, indicating that it can continue to significantly activate the activity of osteoblasts, and ultimately achieve a significant increase in bone mineral density without occurrence of catabolic effects. The bone mineral density improvement effect is even better than that of the combination group of the anti-RANKL monoclonal antibody (weekly administration) and teriparatide (daily administration).

The above two experimental studies show that the present disclosure effectively overcomes the catabolic effects and bone loss problems caused by continuous exposure of PTH activity in an innovative way, solves the compliance problem of PTH treatment, and unexpectedly shows sustained effectiveness for up to 56 days or even longer.

In order to avoid species differences, the present disclosure further investigates the *in vivo* efficacy on non-human primates that are closer to humans. The results of the *in vivo* efficacy experiment of a single administration in a spontaneous elderly osteoporosis rhesus monkey model show that LAPAF can still overcome the problem of bone loss caused by continuous exposure to PTH activity. It is not obvious to find that LAPAF has a particularly fast onset of efficacy and a particularly strong bone mineral density increase. It can quickly increase bone mineral density in a short period of time (such as 2 months), and the bone mineral density increase ratio (>30%) is significantly better than that of other osteoporosis treatment drugs on the market reported in the literature. For example, after 6 months of continuous administration of teriparatide (once a day), the bone mineral density of L2-L4 (L stands for lumbar vertebra, hereinafter referred to as L) only increased by about 5% (accessdata.fda.gov/drugsatfda_docs/nda/2002/21-318_FORTEO_Pharmr_P1.pdf), the anti-sclerostin monoclonal antibody romosozumab only increased the bone mineral density of L1-L4 by about 8% (accessdata.fda.gov/drugsatfda_docs/nda/2019/7610620rig1s000MultidisciplineR.pdf)after 4 months of continuous administration (once a month), and the anti-RANKL monoclonal antibody denosumab only increased the bone mineral density of L1-L4 by about 2% 3 months after the single administration. And there is no literature report that the above drugs can quickly increase bone mineral density in a short period of time (such as 2 months). This further verifies the long-term, rapid and potent effects of LAPAF.

Due to the excellent characteristics demonstrated in the experiments, the present disclosure is feasible for unmet clinical needs that require rapid or/and continuous increase in bone mass including the promotion of osteogenesis, such as fracture union, bone nonunion (such as nonunion or delayed fracture union), bone injuries caused by osteoarthritis, etc. Osteoporotic femoral fracture models of RANKL-humanized transgenic mice (osteoporotic fracture model mice) are induced by ovarian removal and surgical modeling of femoral fracture, and the *in vivo* efficacy results also show that after continuous administration for four weeks (once a week), LAPAF can significantly accelerate fracture union in osteoporotic fracture model mice (For example, LAPAF can increase the number of animals with closed fracture lines in osteoporotic fracture model mice, and significantly increase the bone mineral apposition rate and bone formation rate at the fracture sites), and the bone densities at the fracture site (femur) and non-fracture site (tibia) are significantly increased. In addition, the data of bone mineral apposition rate (MAR) and bone formation rate (BFR) obtained using undecalcified bone tissue sectioning technology show that LAPAF can significantly increase the MAR and BFR of osteoporotic fracture model mice, and the data of some mice can even be increased to near normal levels. The efficacy of fracture union is significantly better than that of the teriparatide (daily administration) group and the combination group of anti-RANKL monoclonal antibody (weekly administration) and teriparatide (daily administration). In addition, significant efficacy is also observed in the *in vivo* efficacy experiment of the mouse osteoarthritis model induced by medial meniscal transection (MMT). For example, after eight weeks of continuous administration (once a week), LAPAF can significantly improve the weight-bearing capacity of the modeling side foot of the mouse and significantly relieve the joint pain caused by MMT.

Furthermore, the present disclosure conducts a toxicity and toxicokinetic study in rhesus monkeys by subcutaneously injecting LAPAF once every three weeks for a total of five times over twelve consecutive weeks. The results show that no obvious PTH-related toxicity such as hypercalcemia is observed in LAPAF at a dose of 0.1 mpk and 0.4 mpk once every three weeks for five consecutive subcutaneous injections. The hypercalcemia caused by PTH is mainly attributed to the action of PTH on the renal PTHR, which increases calcium reabsorption. The biggest difference between kidney tissues and bone tissues is the difference in the expression of RANKL, specifically, osteoblasts are double-positive cells (PTHR⁺/RANKL⁺) simultaneously expressing both RANKL and PTHR, while kidney tissues are single-positive cells (PTHR⁺/RANKL⁻) that express PTHR rather than RANK. To this end, the present disclosure further investigates the *in vitro* activity of LAPAF on PTHR single positive cells (PTHR⁺/RANKL⁻) simulating kidney cells. The results show that the *in vitro* activity of LAPAF on cAMP release induced by PTHR in PTHR⁺/RANKL⁻ cells is 100 times weaker than that of teriparatide, while the control sample synPTH-α RANKL-1 has similar activity to teriparatide. It can be seen that LAPAF shows unexpected and extremely strong RANKL selectivity compared with control sample and teriparatide; and adjusting the anti-RANKL functional region of LAPAF, or adjusting the linker, or adjusting the length or type of the PTH functional region (PTH or PTHrP), or adjusting the Fc region, still maintains significant RANKL selectivity.

In summary, the long-acting parathyroid hormone receptor agonist fusion protein (LAPAF) of the present disclosure simultaneously has multiple advantages of better drug efficacy, longer lasting effect, better safety, higher structural stability, higher expression level and the like, and the overall performance of LAPAF is superior to that of the existing or potential treatment means, *e.g*., teriparatide, PEG-PTH 1-34, an anti-RANKL monoclonal antibody, or control sample synPTH-α RANKL-1.

The present disclosure solves the above-mentioned technical problems through the following technical solutions.

The first aspect of the present disclosures provides a long-acting parathyroid hormone receptor agonist fusion protein, which comprises a first arm and a second arm, wherein the first arm comprises a functional region targeting RANKL and an Fc region, the functional region targeting RANKL is Fv, ScFv, SDA, Fab or ScFab (preferably, Fab or ScFab), and the functional region targeting RANKL is located at the N-terminus of the Fc region; and the second arm comprises a functional region capable of activating PTHR (parathyroid hormone receptor 1) and an Fc region, and the C-terminus of the functional region capable of activating PTHR in the second arm is connected to the N-terminus of the Fc region directly or through a linker, or the functional region capable of activating PTHR in the second arm is connected to the amino acid residues before the CPPCP sequence of the Fc region through a site-directed coupling technology.

In some embodiments of the present disclosure, the long-acting parathyroid hormone receptor agonist fusion protein is a dual-targeting fusion protein of the present disclosure.

The second aspect of the present disclosure provides a long-acting parathyroid hormone receptor agonist fusion protein, which comprises a first arm and a second arm, wherein the first arm consists of a functional region targeting RANKL and an Fc region, the functional region targeting RANKL is in the form of Fv, ScFv, SDA, Fab or ScFab (preferably, Fab or ScFab), and the functional region targeting RANKL is located at the N-terminus of the Fc region; and the second arm consists of a functional region capable of activating PTHR (parathyroid hormone receptor 1) and an Fc region, and the C-terminus of the functional region capable of activating PTHR in the second arm is connected to the N-terminus of the Fc region directly or through a linker, or the functional region capable of activating PTHR in the second arm is connected to the amino acid residues before the CPPCP sequence of the Fc region through a site-directed coupling technology.

In some embodiments of the present disclosure, the Fc region consists of FcA and FcB, and the FcA and the FcB represent tandem heavy chain constant region 2 (CH2) and heavy chain constant region 3 (CH3) of human IgG containing a hinge region, respectively. The FcA and the FcB are the same, or are Knob (s) mutation and Hole (s) mutation, respectively. Preferably, the FcA and the FcB are Knob (s) mutation and Hole (s) mutation, respectively.

In some preferred embodiments of the present disclosure, the human IgG is selected from wild types or mutant types of human IgG1, IgG2, IgG3 and IgG4.

In some more preferred embodiments of the present disclosure, the IgG1 is of a mutant type with a silent Fc immune effector function (such as a combination of one or more of LALA mutation, PGLALA mutation, N297A mutation and other Fc immune effector function-silent IgG 1 mutations), the IgG2 includes a mutant type with a normal Fc immune effector function or with a silent Fc immune effector function, the IgG3 is of the mutant type with the silent Fc immune effector function, and the IgG4 includes the mutant type with the silent Fc immune effector function (such as one or more of E233P/F234V/L235A/D265A/R409K mutations) and/or a mutant type with S228P mutation that improves molecular stability. The numbering of the Fc region residues adopts the numbering of the EU index in Kabat.

In some embodiments of the present disclosure, when the FcA comprises an amino acid sequence as shown in SEQ ID NO: 6, the FcB comprises the amino acid sequence as shown in SEQ ID NO: 6.

In some embodiments of the present disclosure, when the FcA comprises an amino acid sequence as shown in SEQ ID NO: 7, the FcB comprises an amino acid sequence as shown in SEQ ID NO: 8; when the FcA comprises the amino acid sequence as shown in SEQ ID NO: 8, the FcB comprises the amino acid sequence as shown in SEQ ID NO: 7; when the FcA comprises an amino acid sequence as shown in SEQ ID NO: 39, the FcB comprises an amino acid sequence as shown in SEQ ID NO: 40; and when the FcA comprises the amino acid sequence as shown in SEQ ID NO: 40, the FcB comprises the amino acid sequence as shown in SEQ ID NO: 39.

In some embodiments of the present disclosure, the functional region capable of activating PTHR comprises an agonistic antigen-binding variable region targeting PTHR or an agonistic antigen-binding fragment targeting PTHR, or a PTH functional region, wherein the agonistic antigen-binding variable region targeting PTHR is Fv, ScFv or SDA (preferably, ScFv or SDA); the agonistic antigen-binding fragment targeting PTHR is Fab or ScFab; the PTH functional region is selected from a mature human parathyroid hormone (PTH), truncated PTH, a PTH mutant, a mutant of truncated PTH, a truncated mature human parathyroid hormone-related protein (PTHrP), a mutant of truncated PTHrP and an analog of truncated PTHrP; the truncated PTH is preferably truncated mature human PTH obtained by truncating no less than 11 amino acid residues from the N-terminus; and more preferably, the truncated PTH is a truncated mature human parathyroid hormone obtained by truncating no less than 34 amino acid residues from the N-terminus.

In some preferred embodiments of the present disclosure, the PTH functional region is a PTH (1-11) mutant, a PTH (1-14) mutant, a PTH (1-20) mutant, a PTH (1-34) mutant, a PTH (1-37) mutant, a PTH (1-38) mutant, a PTH (1-41) mutant, a PTH (1-84) mutant, a PTHrP (1-34) mutant, a PTHrP (1-36) mutant, a PTHrP (7-34) mutant or Abaloparatide.

In some embodiments of the present disclosure, the Abaloparatide can only be connected to the Fc region by the site-specific coupling technology; preferably, the site-specific connection region is an amino acid residue before the CPPCP sequence of the Fc region.

In some more preferred embodiments of the present disclosure, the PTH functional region comprises an amino acid sequence as shown in any one of SEQ ID NOs: 5, 30, 31, 32, 33, 34, 35, 36, 37 and 38 or sequence 69 in CN1439021A, or an amino acid sequence obtained by addition, deletion, modification and/or conservative substitution of at least one amino acid residue such as 1, 2, 3, 4, 5 or 6 amino acid residues in the amino acid sequences as shown in the aforementioned sequences.

In some embodiments of the present disclosure, the sequence of the linker is selected from (GGGGS)n, (GGGS)n, (GGS)n, (G)n, (GS)n, (EAAAK)n or (XP)n, or a combination of the aforementioned sequences, n is a natural number such as 1-20, and preferably the length of the linker is no more than 20 amino acids.

In some preferred embodiments of the present disclosure, the sequence of the linker comprises an amino acid sequence selected from any one of SEQ ID NOs: 9-12 and SEQ ID NOs: 28-29.

In some specific embodiments of the present disclosure, the linker is selected from GGGGS (SEQ ID NO: 9), GGGGSGGGGS (SEQ ID NO: 10), GGGGSGGGGSGGGGS (SEQ ID NO: 11), GGGGSGGGGSGGGGSGGGGS (SEQ ID NO: 12), GGGS (SEQ ID NO: 28) and EAAAK (SEQ ID NO: 29).

In some specific embodiments of the present disclosure, the amino acid sequence of the linker is as shown in SEQ ID NO: 9.

In some embodiments of the present disclosure, the first arm comprises a first arm A chain and a first arm B chain;
the structure of the first arm A chain from the N-terminus to the C-terminus is VL-CL;
the structure of the first arm B chain from the N-terminus to the C-terminus is VH-CH1-FcA;
the VL and VH or CL and CH1 are optionally interchanged by a CrossMab technology.

In some embodiments of the present disclosure, when the functional region of the second arm capable of activating PTHP is an agonistic antigen-binding variable region targeting PTHR or an agonistic antigen-binding fragment targeting PTHR and is ScFab, ScFv or SDA, or when the functional region capable of activating PTHR in the second arm is a PTH functional region, the structure of the second arm from the N-terminus to the C-terminus is a functional region capable of activating PTHR-FcB or the functional region capable of activating PTHR-linker-FcB.

In some embodiments of the present disclosure, when the functional region capable of activating PTHR in the second arm is an agonistic antigen-binding variable region targeting PTHR or an agonistic antigen-binding fragment targeting PTHR and is Fab or Fv, the second arm comprises a second arm C chain and a second arm D chain; the structure of the second arm C chain from the N-terminus to the C-terminus is VL-CL or VL, and the structure of the second arm D chain from the N-terminus to the C-terminus is VH-CH1-FcB, VH-CH1-linker-FcB, VH-FcB or VH-linker-FcB; or, the structure of the second arm C chain from the N-terminus to the C-terminus is VH-CL or VH, and the structure of the second arm D chain from the N-terminus to the C-terminus is VL-CH1-FcB or VL-CH1-linker-FcB or VL-FcB or VL-linker-FcB, and the VL and VH or CL and CH1 are optionally interchanged by the CrossMab technology

In some embodiments of the present disclosure, the Fv or ScFv targeting RANKL comprises 1) a heavy chain variable region (VH) comprising heavy chain complementary determining regions HCDR1-3, and 2) a light chain variable region (VL) comprising light chain complementary determining regions LCDR 1-3, wherein the amino acid sequences of the HCDR1-3 are respectively as shown in SEQ ID NOs: 16-18 or SEQ ID NOs: 22-24, and the amino acid sequences of the LCDR1-3 are respectively as shown in SEQ ID NOs: 19-21 or SEQ ID NOs: 25-27.

In some specific embodiments of the present disclosure, the Fv or ScFv targeting RANKL comprises 1) a heavy chain variable region (VH) comprising heavy chain complementary determining regions HCDR1-3, and 2) a light chain variable region (VL) comprising light chain complementary determining regions LCDR1-3, wherein the amino acid sequences of the HCDR1-3 are respectively as shown in SEQ ID NOs: 16-18, and the amino acid sequences of the LCDR1-3 are respectively as shown in SEQ ID NOs: 19-21.

In some specific embodiments of the present disclosure, the Fv or ScFv targeting RANKL comprises 1) a heavy chain variable region (VH) comprising heavy chain complementary determining regions HCDR1-3, and 2) a light chain variable region (VL) comprising light chain complementary determining regions LCDR 1-3, wherein the amino acid sequences of the HCDR1-3 are respectively as shown in SEQ ID NOs: 16-18, and the amino acid sequences of the LCDR1-3 are respectively as shown in SEQ ID NOs: 25-27.

In some specific embodiments of the present disclosure, the Fv or ScFv targeting RANKL comprises 1) a heavy chain variable region (VH) comprising heavy chain complementary determining regions HCDR1-3, and 2) a light chain variable region (VL) comprising light chain complementary determining regions LCDR1-3, wherein the amino acid sequences of the HCDR1-3 are respectively as shown in SEQ ID NOs: 22-24, and the amino acid sequences of the LCDR1-3 are respectively as shown in SEQ ID NOs: 19-21.

In some specific embodiments of the present disclosure, the Fv or ScFv targeting RANKL comprises 1) a heavy chain variable region (VH) comprising heavy chain complementary determining regions HCDR1-3, and 2) a light chain variable region (VL) comprising light chain complementary determining regions LCDR1-3, wherein the amino acid sequences of the HCDR1-3 are respectively as shown in SEQ ID NOs: 22-24, and the amino acid sequences of the LCDR1-3 are respectively as shown in SEQ ID NOs: 25-27.

In some embodiments of the present disclosure, the Fab or ScFab targeting RANKL comprises 1) a heavy chain variable region (VH) comprising heavy chain complementary determining regions HCDR1-3, 2) a light chain variable region (VL) comprising light chain complementary determining regions LCDR1-3, 3) a light chain constant region (CL) and 4) a heavy chain constant region 1 (CH1), wherein the amino acid sequences of HCDR1-3 are respectively as shown in SEQ ID NOs: 16-18 or SEQ ID NOs: 22-24, and the amino acid sequences of LCDR1-3 are respectively as shown in SEQ ID NOs: 19-21 or SEQ ID NOs: 25-27; and the Fab or ScFab can exchange the heavy chain variable region and the light chain variable region, or exchange the heavy chain constant region 1 and the light chain constant region through the CrossMab technology.

In some embodiments of the present disclosure, the Fab or ScFab targeting RANKL comprises 1) a heavy chain variable region (VH) containing heavy chain complementary determining regions HCDR1-3, 2) a light chain variable region (VL) containing light chain complementary determining regions LCDR1-3, 3) a light chain constant region (CL), and 4) a heavy chain constant region 1 (CH1), wherein the amino acid sequences of the HCDR1-3 are respectively as shown in SEQ ID NOs: 16-18, and the amino acid sequences of the LCDR1-3 are respectively as shown in SEQ ID NOs: 19-21; and the Fab or ScFab can exchange the heavy chain variable region and the light chain variable region or exchange the heavy chain constant region 1 and the light chain constant region through the CrossMab technology.

In some embodiments of the present disclosure, the Fab or ScFab targeting RANKL comprises 1) a heavy chain variable region (VH) comprising heavy chain complementary determining regions HCDR1-3, 2) a light chain variable region (VL) comprising light chain complementary determining regions LCDR1-3, 3) a light chain constant region (CL) and 4) a heavy chain constant region 1 (CH1), wherein the amino acid sequences of the HCDR1-3 are respectively as shown in SEQ ID NOs: 16-18, and the amino acid sequences of the LCDR1-3 are respectively as shown in SEQ ID NOs: 25-27; and the Fab or ScFab can exchange the heavy chain variable region and the light chain variable region or exchange the heavy chain constant region 1 and the light chain constant region through the CrossMab technology.

In some embodiments of the present disclosure, the Fab or ScFab targeting RANKL comprises 1) a heavy chain variable region (VH) comprising heavy chain complementary determining regions HCDR1-3, 2) a light chain variable region (VL) comprising light chain complementary determining regions LCDR1-3, 3) a light chain constant region (CL) and 4) a heavy chain constant region 1 (CH1), wherein the amino acid sequences of the HCDR1-3 are respectively as shown in SEQ ID NOs: 22-24, and the amino acid sequences of the LCDR1-3 are respectively as shown in SEQ ID NOs: 19-21; and the Fab or ScFab can exchange the heavy chain variable region and the light chain variable region or exchange the heavy chain constant region 1 and the light chain constant region through the CrossMab technology.

In some embodiments of the present disclosure, the Fab or ScFab targeting RANKL comprises 1) a heavy chain variable region (VH) comprising heavy chain complementary determining regions HCDR1-3, 2) a light chain variable region (VL) comprising light chain complementary determining regions LCDR1-3, 3) a light chain constant region (CL) and 4) a heavy chain constant region 1 (CH1), wherein the amino acid sequences of HCDR1-3 are respectively as shown in SEQ ID NOs: 22-24, and the amino acid sequences of LCDR1-3 are respectively as shown in SEQ ID NOs: 25-27; the Fab or ScFab can exchange the heavy chain variable region and the light chain variable region, or exchange the heavy chain constant region 1 and the light chain constant region through the CrossMab technology.

In some embodiments of the present disclosure, the amino acid sequence of the heavy chain variable region (VH) comprises an amino acid sequence as shown in SEQ ID NO: 1 or 3, and the amino acid sequence of the light chain variable region (VL) comprises an amino acid sequence as shown in SEQ ID NO: 2 or 4.

In some embodiments of the present disclosure, the amino acid sequence of the heavy chain constant region 1 (CH1) comprises an amino acid sequence as shown in SEQ ID NO: 14, and the amino acid sequence of the light chain constant region (CL) comprises an amino acid sequence as shown in SEQ ID NO: 15.

In some specific embodiments of the present disclosure, the long-acting parathyroid hormone receptor agonist fusion protein comprises the long-acting parathyroid hormone receptor agonist fusion protein as described in the third aspect below.

The third aspect of the present disclosure provides a long-acting parathyroid hormone receptor agonist fusion protein, which consists of a first arm and a second arm, the first arm comprises a first arm A chain and a first arm B chain; wherein:
when the amino acid sequence of the first arm A chain comprises SEQ ID NO: 2 and SEQ ID NO: 15 connected sequentially, the amino acid sequence of the first arm B chain comprises SEQ ID NO: 1, SEQ ID NO: 14 and SEQ ID NO: 6 connected sequentially, or SEQ ID NO: 1, SEQ ID NO: 14 and SEQ ID NO: 7 connected sequentially, or SEQ ID NO: 1, SEQ ID NO: 14, and SEQ ID NO: 8 connected sequentially, or SEQ ID NO: 1, SEQ ID NO: 14 and SEQ ID NO: 39 connected sequentially, or SEQ ID NO: 1, SEQ ID NO: 14 and SEQ ID NO: 40 connected sequentially; or,
when the amino acid sequence of the first arm A chain comprises SEQ ID NO: 1 and SEQ ID NO: 15 connected sequentially, the amino acid sequence of the first arm B chain comprises SEQ ID NO: 2, SEQ ID NO: 14 and SEQ ID NO: 6 connected sequentially, or SEQ ID NO: 2, SEQ ID NO: 14, and SEQ ID NO: 7 connected sequentially, or SEQ ID NO: 2, SEQ ID NO: 14 and SEQ ID NO: 8 connected sequentially, or SEQ ID NO: 2, SEQ ID NO: 14 and SEQ ID NO: 39 connected sequentially, or SEQ ID NO: 2, SEQ ID NO: 14 and SEQ ID NO: 40 connected sequentially; or,
when the amino acid sequence of the first arm A chain comprises SEQ ID NO: 2 and SEQ ID NO: 14 connected sequentially, the amino acid sequence of the first arm B chain comprises SEQ ID NO: 1, SEQ ID NO: 15 and SEQ ID NO: 6 connected sequentially, or SEQ ID NO: 1, SEQ ID NO: 15 and SEQ ID NO: 7 connected sequentially, or SEQ ID NO: 1, SEQ ID NO: 15 and SEQ ID NO: 8 connected sequentially, or SEQ ID NO: 1, SEQ ID NO: 15, and SEQ ID NO: 39 connected sequentially, or SEQ ID NO: 1, SEQ ID NO: 15 and SEQ ID NO: 40 connected sequentially; or,
when the amino acid sequence of the first arm A chain comprises SEQ ID NO: 1 and SEQ ID NO: 14 connected sequentially, the amino acid sequence of the first arm B chain comprises SEQ ID NO: 2, SEQ ID NO: 15 and SEQ ID NO: 6 connected sequentially, or SEQ ID NO: 2, SEQ ID NO: 15 and SEQ ID NO: 7 connected sequentially, or SEQ ID NO: 2, SEQ ID NO: 15, and SEQ ID NO: 8 connected sequentially, or SEQ ID NO: 2, SEQ ID NO: 15 and SEQ ID NO: 39 connected sequentially, or SEQ ID NO: 2, SEQ ID NO: 15 and SEQ ID NO: 40 connected sequentially; or,
when the amino acid sequence of the first arm A chain comprises SEQ ID NO: 4 and SEQ ID NO: 15 connected sequentially, the amino acid sequence of the first arm B chain comprises SEQ ID NO: 3, SEQ ID NO: 14 and SEQ ID NO: 6 connected sequentially, or SEQ ID NO: 3, SEQ ID NO: 14 and SEQ ID NO: 7 connected sequentially, or SEQ ID NO: 3, SEQ ID NO: 14 and SEQ ID NO: 8 connected sequentially, or SEQ ID NO: 3, SEQ ID NO: 14 and SEQ ID NO: 39 connected sequentially, or SEQ ID NO: 3, SEQ ID NO: 14 and SEQ ID NO: 40 connected sequentially; or,
when the amino acid sequence of the first arm A chain comprises SEQ ID NO: 3 and SEQ ID NO: 15 connected sequentially, the amino acid sequence of the first arm B chain comprises SEQ ID NO: 4, SEQ ID NO: 14, and SEQ ID NO: 6 connected sequentially, or SEQ ID NO: 4, SEQ ID NO: 14, and SEQ ID NO: 7 connected sequentially, or SEQ ID NO: 4, SEQ ID NO: 14 and SEQ ID NO: 8 connected sequentially, or SEQ ID NO: 4, SEQ ID NO: 14 and SEQ ID NO: 39 connected sequentially, or SEQ ID NO: 4, SEQ ID NO: 14 and SEQ ID NO: 40 connected sequentially; or,
when the amino acid sequence of the first arm A chain comprises SEQ ID NO: 4 and SEQ ID NO: 14 connected sequentially, the amino acid sequence of the first arm B chain comprises SEQ ID NO: 3, SEQ ID NO: 15 and SEQ ID NO: 6 connected sequentially, or SEQ ID NO: 3, SEQ ID NO: 15 and SEQ ID NO: 7 connected sequentially, or SEQ ID NO: 3, SEQ ID NO: 15 and SEQ ID NO: 8 connected sequentially, or SEQ ID NO: 3, SEQ ID NO: 15 and SEQ ID NO: 39 connected sequentially, or SEQ ID NO: 3, SEQ ID NO: 15 and SEQ ID NO: 40 connected sequentially; or,
when the amino acid sequence of the first arm A chain comprises SEQ ID NO: 3 and SEQ ID NO: 14 connected sequentially, the amino acid sequence of the first arm B chain comprises SEQ ID NO: 4, SEQ ID NO: 15 and SEQ ID NO: 6 connected sequentially, or SEQ ID NO: 4, SEQ ID NO: 15 and SEQ ID NO: 7 connected sequentially, or SEQ ID NO: 4, SEQ ID NO: 15 and SEQ ID NO: 8 connected sequentially, or SEQ ID NO: 4, SEQ ID NO: 15 and SEQ ID NO: 39 connected sequentially, or SEQ ID NO: 4, SEQ ID NO: 15 and SEQ ID NO: 40 connected sequentially; or,

In some embodiments of the present disclosure:
when the amino acid sequence of the first arm B chain comprises SEQ ID NO: 6, the amino acid sequence of the second arm comprises a PTH functional region, a linker and an Fc region connected sequentially, the PTH functional region is selected from SEQ ID NOs: 5 and 31-38, the linker is selected from SEQ ID NOs: 9-12, and the Fc region sequence is SEQ ID NO: 6; or,
when the amino acid sequence of the first arm B chain comprises SEQ ID NO: 7, the amino acid sequence of the second arm comprises a PTH functional region, a linker and an Fc region connected sequentially, the PTH functional region is selected from SEQ ID NOs: 5 and 31-38, the linker is selected from SEQ ID NOs: 9-12, and the Fc region sequence is SEQ ID NO: 8; or,
when the amino acid sequence of the first arm B chain comprises SEQ ID NO: 8, the amino acid sequence of the second arm comprises a PTH functional region, a linker and an Fc region connected sequentially, the PTH functional region is selected from SEQ ID NOs: 5 and 31-38, the linker is selected from SEQ ID NOs: 9-12, and the Fc region sequence is SEQ ID NO: 7; or,
when the amino acid sequence of the first arm B chain comprises SEQ ID NO: 39, the amino acid sequence of the second arm comprises a PTH functional region, a linker and an Fc region connected sequentially, the PTH functional region is selected from SEQ ID NOs: 5 and 31-38, the linker is selected from SEQ ID NOs: 9-12, and the Fc region sequence is SEQ ID NO: 40; or,
when the amino acid sequence of the first arm B chain comprises SEQ ID NO: 40, the amino acid sequence of the second arm comprises a PTH functional region, a linker and an Fc region connected sequentially, the PTH functional region is selected from SEQ ID NOs: 5 and 31-38, the linker is selected from SEQ ID NOs: 9-12, and the Fc region sequence is SEQ ID NO: 39.

In some embodiments of the present disclosure:
when the amino acid sequence of the first arm A chain comprises SEQ ID NO: 2 and SEQ ID NO: 15 connected sequentially, the amino acid sequence of the first arm B chain comprises SEQ ID NO: 1, SEQ ID NO: 14 and SEQ ID NO: 7 connected sequentially; or, the amino acid sequence of the first arm A chain comprises SEQ ID NO: 2 and SEQ ID NO: 14 connected sequentially, and the amino acid sequence of the first arm B chain comprises SEQ ID NO: 1, SEQ ID NO: 15 and SEQ ID NO: 7 connected sequentially; or, the amino acid sequence of the first arm A chain comprises SEQ ID NO: 1 and SEQ ID NO: 14 connected sequentially, and the amino acid sequence of the first arm B chain comprises SEQ ID NO: 2, SEQ ID NO: 15 and SEQ ID NO: 7 connected sequentially; or, the amino acid sequence of the first arm A chain comprises SEQ ID NO: 1 and SEQ ID NO: 15 connected sequentially, and the amino acid sequence of the first arm B chain comprises SEQ ID NO: 2, SEQ ID NO: 14 and SEQ ID NO: 7 connected sequentially;
the amino acid sequence of the second arm comprises SEQ ID NO: 5, SEQ ID NO: 9 and SEQ ID NO: 8 connected sequentially, or SEQ ID NO: 5 and SEQ ID NO: 8 connected sequentially, or SEQ ID NO: 5, SEQ ID NO: 10 and SEQ ID NO: 8 connected sequentially, or SEQ ID NO: 5, SEQ ID NO: 11 and SEQ ID NO: 8 connected sequentially, or SEQ ID NO: 5, SEQ ID NO: 12 and SEQ ID NO: 8 connected sequentially, or SEQ ID NO: 31 and SEQ ID NO: 8 connected sequentially, or SEQ ID NO: 31, SEQ ID NO: 9 and SEQ ID NO: 8 connected sequentially, or SEQ ID NO: 31, SEQ ID NO: 10 and SEQ ID NO: 8 connected sequentially, or SEQ ID NO: 31, SEQ ID NO: 11, and SEQ ID NO: 8 connected sequentially, or SEQ ID NO: 31, SEQ ID NO: 12 and SEQ ID NO: 8 connected sequentially, or SEQ ID NO: 32 and SEQ ID NO: 8 connected sequentially, or SEQ ID NO: 32, SEQ ID NO: 9 and SEQ ID NO: 8 connected sequentially, or SEQ ID NO: 32, SEQ ID NO: 10, and SEQ ID NO: 8 connected sequentially, or SEQ ID NO: 32, SEQ ID NO: 11 and SEQ ID NO: 8 connected sequentially, or SEQ ID NO: 32, SEQ ID NO: 12 and SEQ ID NO: 8 connected sequentially, or SEQ ID NO: 33 and SEQ ID NO: 8 connected sequentially, or SEQ ID NO: 33, SEQ ID NO: 9 and SEQ ID NO: 8 connected sequentially, or SEQ ID NO: 33, SEQ ID NO: 10 and SEQ ID NO: 8 connected sequentially, or SEQ ID NO: 33, SEQ ID NO: 11 and SEQ ID NO: 8 connected sequentially, or SEQ ID NO: 33, SEQ ID NO: 12 and SEQ ID NO: 8 connected sequentially, or SEQ ID NO: 34 and SEQ ID NO: 8 connected sequentially, or SEQ ID NO: 34, SEQ ID NO: 9 and SEQ ID NO: 8 connected sequentially, or SEQ ID NO: 34, SEQ ID NO: 10 and SEQ ID NO: 8 connected sequentially, or SEQ ID NO: 34, SEQ ID NO: 11 and SEQ ID NO: 8 connected sequentially, or SEQ ID NO: 34, SEQ ID NO: 12 and SEQ ID NO: 8 connected sequentially, or SEQ ID NO: 35 and SEQ ID NO: 8 connected sequentially, or SEQ ID NO: 35, SEQ ID NO: 9 and SEQ ID NO: 8 connected sequentially, or SEQ ID NO: 35, SEQ ID NO: 10 and SEQ ID NO: 8 connected sequentially, or SEQ ID NO: 35, SEQ ID NO: 11 and SEQ ID NO: 8 connected sequentially, or SEQ ID NO: 35, SEQ ID NO: 12 and SEQ ID NO: 8 connected sequentially, or SEQ ID NO: 36 and SEQ ID NO: 8 connected sequentially, or SEQ ID NO: 36, SEQ ID NO: 9 and SEQ ID NO: 8 connected sequentially, or SEQ ID NO: 36, SEQ ID NO: 10 and SEQ ID NO: 8 connected sequentially, or SEQ ID NO: 36, SEQ ID NO: 11 and SEQ ID NO: 8 connected sequentially, or SEQ ID NO: 36, SEQ ID NO: 12 and SEQ ID NO: 8 connected sequentially, or SEQ ID NO: 37 and SEQ ID NO: 8 connected sequentially, or SEQ ID NO: 37, SEQ ID NO: 9 and SEQ ID NO: 8 connected sequentially, or SEQ ID NO: 37, SEQ ID NO: 10 and SEQ ID NO: 8 connected sequentially, or SEQ ID NO: 37, SEQ ID NO: 11 and SEQ ID NO: 8 connected sequentially, or SEQ ID NO: 37, SEQ ID NO: 12 and SEQ ID NO: 8 connected sequentially, or SEQ ID NO: 38 and SEQ ID NO: 8 connected sequentially, or SEQ ID NO: 38, SEQ ID NO: 9 and SEQ ID NO: 8 connected sequentially, or SEQ ID NO: 38, SEQ ID NO: 10 and SEQ ID NO: 8 connected sequentially, or SEQ ID NO: 38, SEQ ID NO: 11 and SEQ ID NO: 8 connected sequentially, or SEQ ID NO: 38, SEQ ID NO: 12 and SEQ ID NO: 8 connected sequentially.

When the amino acid sequence of the first arm A chain comprises SEQ ID NO: 2 and SEQ ID NO: 15 connected sequentially, the amino acid sequence of the first arm B chain comprises SEQ ID NO: 1, SEQ ID NO: 14 and SEQ ID NO: 8 connected sequentially; or, the amino acid sequence of the first arm A chain comprises SEQ ID NO: 2 and SEQ ID NO: 14 connected sequentially, and the amino acid sequence of the first arm B chain comprises SEQ ID NO: 1, SEQ ID NO: 15 and SEQ ID NO: 8 connected sequentially; or, the amino acid sequence of the first arm A chain comprises SEQ ID NO: 1 and SEQ ID NO: 14 connected sequentially, and the amino acid sequence of the first arm B chain comprises SEQ ID NO: 2, SEQ ID NO: 15 and SEQ ID NO: 8 connected sequentially; or, the amino acid sequence of the first arm A chain comprises SEQ ID NO: 1 and SEQ ID NO: 15 connected sequentially, and the amino acid sequence of the first arm B chain comprises SEQ ID NO: 2, SEQ ID NO: 14 and SEQ ID NO: 8 connected sequentially;
the amino acid sequence of the second arm comprises SEQ ID NO: 5, SEQ ID NO: 9 and SEQ ID NO: 7 connected sequentially, or SEQ ID NO: 5 and SEQ ID NO: 7 connected sequentially, or SEQ ID NO: 5, SEQ ID NO: 10 and SEQ ID NO: 7 connected sequentially, or SEQ ID NO: 5, SEQ ID NO: 11 and SEQ ID NO: 7 connected sequentially, or SEQ ID NO: 5, SEQ ID NO: 12 and SEQ ID NO: 7 connected sequentially, or SEQ ID NO: 31 and SEQ ID NO: 7 connected sequentially, or SEQ ID NO: 31, SEQ ID NO: 9 and SEQ ID NO: 7 connected sequentially, or SEQ ID NO: 31, SEQ ID NO: 10 and SEQ ID NO: 7 connected sequentially, or SEQ ID NO: 31, SEQ ID NO: 11 and SEQ ID NO: 7 connected sequentially, or SEQ ID NO: 31, SEQ ID NO: 12 and SEQ ID NO: 7 connected sequentially, or SEQ ID NO: 32 and SEQ ID NO: 7 connected sequentially, or SEQ ID NO: 32, SEQ ID NO: 9 and SEQ ID NO: 7 connected sequentially, or SEQ ID NO: 32, SEQ ID NO: 10, and SEQ ID NO: 7 connected sequentially, or SEQ ID NO: 32, SEQ ID NO: 11 and SEQ ID NO: 7 connected sequentially, or SEQ ID NO: 32, SEQ ID NO: 12 and SEQ ID NO: 7 connected sequentially, or SEQ ID NO: 33 and SEQ ID NO: 7 connected sequentially, or SEQ ID NO: 33, SEQ ID NO: 9 and SEQ ID NO: 7 connected sequentially, or SEQ ID NO: 33, SEQ ID NO: 10 and SEQ ID NO: 7 connected sequentially, or SEQ ID NO: 33, SEQ ID NO: 11 and SEQ ID NO: 7 connected sequentially, or SEQ ID NO: 33, SEQ ID NO: 12 and SEQ ID NO: 7 connected sequentially, or SEQ ID NO: 34 and SEQ ID NO: 7 connected sequentially, or SEQ ID NO: 34, SEQ ID NO: 9 and SEQ ID NO: 7 connected sequentially, or SEQ ID NO: 34, SEQ ID NO: 10, and SEQ ID NO: 7 connected sequentially, or SEQ ID NO: 34, SEQ ID NO: 11 and SEQ ID NO: 7 connected sequentially, or SEQ ID NO: 34, SEQ ID NO: 12 and SEQ ID NO: 7 connected sequentially, or SEQ ID NO: 35 and SEQ ID NO: 7 connected sequentially, or SEQ ID NO: 35, SEQ ID NO: 9 and SEQ ID NO: 7 connected sequentially, or SEQ ID NO: 35, SEQ ID NO: 10 and SEQ ID NO: 7 connected sequentially, or SEQ ID NO: 35, SEQ ID NO: 11 and SEQ ID NO: 7 connected sequentially, or SEQ ID NO: 35, SEQ ID NO: 12 and SEQ ID NO: 7 connected sequentially, or SEQ ID NO: 36 and SEQ ID NO: 7 connected sequentially, or SEQ ID NO: 36, SEQ ID NO: 9 and SEQ ID NO: 7 connected sequentially, or SEQ ID NO: 36, SEQ ID NO: 10 and SEQ ID NO: 7 connected sequentially, or SEQ ID NO: 36, SEQ ID NO: 11 and SEQ ID NO: 7 connected sequentially, or SEQ ID NO: 36, SEQ ID NO: 12 and SEQ ID NO: 7 connected sequentially, or SEQ ID NO: 37 and SEQ ID NO: 7 connected sequentially, or SEQ ID NO: 37, SEQ ID NO: 9 and SEQ ID NO: 7 connected sequentially, or SEQ ID NO: 37, SEQ ID NO: 10 and SEQ ID NO: 7 connected sequentially, or SEQ ID NO: 37, SEQ ID NO: 11 and SEQ ID NO: 7 connected sequentially, or SEQ ID NO: 37, SEQ ID NO: 12 and SEQ ID NO: 7 connected sequentially, or SEQ ID NO: 38 and SEQ ID NO: 7 connected sequentially, or SEQ ID NO: 38, SEQ ID NO: 9 and SEQ ID NO: 7 connected sequentially, or SEQ ID NO: 38, SEQ ID NO: 10 and SEQ ID NO: 7 connected sequentially, or SEQ ID NO: 38, SEQ ID NO: 11 and SEQ ID NO: 7 connected sequentially, or SEQ ID NO: 38, SEQ ID NO: 12 and SEQ ID NO: 7 connected sequentially.

When the amino acid sequence of the first arm A chain comprises SEQ ID NO: 2 and SEQ ID NO: 15 connected sequentially, the amino acid sequence of the first arm B chain comprises SEQ ID NO: 1, SEQ ID NO: 14 and SEQ ID NO: 39 connected sequentially; or, the amino acid sequence of the first arm A chain comprises SEQ ID NO: 2 and SEQ ID NO: 14 connected sequentially, and the amino acid sequence of the first arm B chain comprises SEQ ID NO: 1, SEQ ID NO: 15 and SEQ ID NO: 39 connected sequentially; or, the amino acid sequence of the first arm A chain comprises SEQ ID NO: 1 and SEQ ID NO: 14 connected sequentially, and the amino acid sequence of the first arm B chain comprises SEQ ID NO: 2, SEQ ID NO: 15 and SEQ ID NO: 39 connected sequentially; or, the amino acid sequence of the first arm A chain comprises SEQ ID NO: 1 and SEQ ID NO: 15 connected sequentially, and the amino acid sequence of the first arm B chain comprises SEQ ID NO: 2, SEQ ID NO: 14 and SEQ ID NO: 39 connected sequentially;
the amino acid sequence of the second arm comprises SEQ ID NO: 5, SEQ ID NO: 9 and SEQ ID NO: 40 connected sequentially, or SEQ ID NO: 5 and SEQ ID NO: 40 connected sequentially, or SEQ ID NO: 5, SEQ ID NO: 10 and SEQ ID NO: 40 connected sequentially, or SEQ ID NO: 5, SEQ ID NO: 11, and SEQ ID NO: 40 connected sequentially, or SEQ ID NO: 5, SEQ ID NO: 12, and SEQ ID NO: 40 connected sequentially, or SEQ ID NO: 31 and SEQ ID NO: 40 connected sequentially, or SEQ ID NO: 31, SEQ ID NO: 9 and SEQ ID NO: 40 connected sequentially, or SEQ ID NO: 31, SEQ ID NO: 10 and SEQ ID NO: 40 connected sequentially, or SEQ ID NO: 31, SEQ ID NO: 11 and SEQ ID NO: 40 connected sequentially, or SEQ ID NO: 31, SEQ ID NO: 12 and SEQ ID NO: 40 connected sequentially, or SEQ ID NO: 32 and SEQ ID NO: 40 connected sequentially, or SEQ ID NO: 32, SEQ ID NO: 9 and SEQ ID NO: 40 connected sequentially, or SEQ ID NO: 32, SEQ ID NO: 10 and SEQ ID NO: 40 connected sequentially, or SEQ ID NO: 32, SEQ ID NO: 11 and SEQ ID NO: 40 connected sequentially, or SEQ ID NO: 32, SEQ ID NO: 12 and SEQ ID NO: 40 connected sequentially, or SEQ ID NO: 33 and SEQ ID NO: 40 connected sequentially, or SEQ ID NO: 33, SEQ ID NO: 9 and SEQ ID NO: 40 connected sequentially, or SEQ ID NO: 33, SEQ ID NO: 10 and SEQ ID NO: 40 connected sequentially, or SEQ ID NO: 33, SEQ ID NO: 11 and SEQ ID NO: 40 connected sequentially, or SEQ ID NO: 33, SEQ ID NO: 12 and SEQ ID NO: 40 connected sequentially, or SEQ ID NO: 34 and SEQ ID NO: 40 connected sequentially, or SEQ ID NO: 34, SEQ ID NO: 9 and SEQ ID NO: 40 connected sequentially, or SEQ ID NO: 34, SEQ ID NO: 10 and SEQ ID NO: 40 connected sequentially, or SEQ ID NO: 34, SEQ ID NO: 11 and SEQ ID NO: 40 connected sequentially, or SEQ ID NO: 34, SEQ ID NO: 12 and SEQ ID NO: 40 connected sequentially, or SEQ ID NO: 35 and SEQ ID NO: 40 connected sequentially, or SEQ ID NO: 35, SEQ ID NO: 9 and SEQ ID NO: 40 connected sequentially, or SEQ ID NO: 35, SEQ ID NO: 10 and SEQ ID NO: 40 connected sequentially, or SEQ ID NO: 35, SEQ ID NO: 11 and SEQ ID NO: 40 connected sequentially, or SEQ ID NO: 35, SEQ ID NO: 12 and SEQ ID NO: 40 connected sequentially, or SEQ ID NO: 36 and SEQ ID NO: 40 connected sequentially, or SEQ ID NO: 36, SEQ ID NO: 9 and SEQ ID NO: 40 connected sequentially, or SEQ ID NO: 36, SEQ ID NO: 10 and SEQ ID NO: 40 connected sequentially, or SEQ ID NO: 36, SEQ ID NO: 11 and SEQ ID NO: 40 connected sequentially, or SEQ ID NO: 36, SEQ ID NO: 12 and SEQ ID NO: 40 connected sequentially, or SEQ ID NO: 37 and SEQ ID NO: 40 connected sequentially, or SEQ ID NO: 37, SEQ ID NO: 9 and SEQ ID NO: 40 connected sequentially, or SEQ ID NO: 37, SEQ ID NO: 10 and SEQ ID NO: 40 connected sequentially, or SEQ ID NO: 37, SEQ ID NO: 11 and SEQ ID NO: 40 connected sequentially, or SEQ ID NO: 37, SEQ ID NO: 12 and SEQ ID NO: 40 connected sequentially, or SEQ ID NO: 38 and SEQ ID NO: 40 connected sequentially, or SEQ ID NO: 38, SEQ ID NO: 9 and SEQ ID NO: 40 connected sequentially, or SEQ ID NO: 38, SEQ ID NO: 10 and SEQ ID NO: 40 connected sequentially, or SEQ ID NO: 38, SEQ ID NO: 11 and SEQ ID NO: 40 connected sequentially, or SEQ ID NO: 38, SEQ ID NO: 12 and SEQ ID NO: 40 connected sequentially;
when the amino acid sequence of the first arm A chain comprises SEQ ID NO: 2 and SEQ ID NO: 15 connected sequentially, the amino acid sequence of the first arm B chain comprises SEQ ID NO: 1, SEQ ID NO: 14 and SEQ ID NO: 40 connected sequentially; or, the amino acid sequence of the first arm A chain comprises SEQ ID NO: 2 and SEQ ID NO: 14 connected sequentially, and the amino acid sequence of the first arm B chain comprises SEQ ID NO: 1, SEQ ID NO: 15 and SEQ ID NO: 40 connected sequentially; or, the amino acid sequence of the first arm A chain comprises SEQ ID NO: 1 and SEQ ID NO: 14 connected sequentially, and the amino acid sequence of the first arm B chain comprises SEQ ID NO: 2, SEQ ID NO: 15 and SEQ ID NO: 40 connected sequentially; or, the amino acid sequence of the first arm A chain comprises SEQ ID NO: 1 and SEQ ID NO: 15 connected sequentially, and the amino acid sequence of the first arm B chain comprises SEQ ID NO: 2, SEQ ID NO: 14 and SEQ ID NO: 40 connected sequentially;
the amino acid sequence of the second arm comprises SEQ ID NO: 5, SEQ ID NO: 9, and SEQ ID NO: 39 connected sequentially, or SEQ ID NO: 5 and SEQ ID NO: 39 connected sequentially, or SEQ ID NO: 5, SEQ ID NO: 10 and SEQ ID NO: 39 connected sequentially, or SEQ ID NO: 5, SEQ ID NO: 11 and SEQ ID NO: 39 connected sequentially, or SEQ ID NO: 5, SEQ ID NO: 12 and SEQ ID NO: 39 connected sequentially, or SEQ ID NO: 31 and SEQ ID NO: 39 connected sequentially, or SEQ ID NO: 31, SEQ ID NO: 9 and SEQ ID NO: 39 connected sequentially, or SEQ ID NO: 31, SEQ ID NO: 10 and SEQ ID NO: 39 connected sequentially, or SEQ ID NO: 31, SEQ ID NO: 11 and SEQ ID NO: 39 connected sequentially, or SEQ ID NO: 31, SEQ ID NO: 12 and SEQ ID NO: 39 connected sequentially, or SEQ ID NO: 32 and SEQ ID NO: 39 connected sequentially, or SEQ ID NO: 32, SEQ ID NO: 9 and SEQ ID NO: 39 connected sequentially, or SEQ ID NO: 32, SEQ ID NO: 10 and SEQ ID NO: 39 connected sequentially, or SEQ ID NO: 32, SEQ ID NO: 11 and SEQ ID NO: 39 connected sequentially, or SEQ ID NO: 32, SEQ ID NO: 12 and SEQ ID NO: 39 connected sequentially, or SEQ ID NO: 33 and SEQ ID NO: 39 connected sequentially, or SEQ ID NO: 33, SEQ ID NO: 9 and SEQ ID NO: 39 connected sequentially, or SEQ ID NO: 33, SEQ ID NO: 10 and SEQ ID NO: 39 connected sequentially, or SEQ ID NO: 33, SEQ ID NO: 11 and SEQ ID NO: 39 connected sequentially, or SEQ ID NO: 33, SEQ ID NO: 12 and SEQ ID NO: 39 connected sequentially, or SEQ ID NO: 34 and SEQ ID NO: 39 connected sequentially, or SEQ ID NO: 34, SEQ ID NO: 9 and SEQ ID NO: 39 connected sequentially, or SEQ ID NO: 34, SEQ ID NO: 10 and SEQ ID NO: 39 connected sequentially, or SEQ ID NO: 34, SEQ ID NO: 11 and SEQ ID NO: 39 connected sequentially, or SEQ ID NO: 34, SEQ ID NO: 12 and SEQ ID NO: 39 connected sequentially, or SEQ ID NO: 35 and SEQ ID NO: 39 connected sequentially, or SEQ ID NO: 35, SEQ ID NO: 9 and SEQ ID NO: 39 connected sequentially, or SEQ ID NO: 35, SEQ ID NO: 10 and SEQ ID NO: 39 connected sequentially, or SEQ ID NO: 35, SEQ ID NO: 11 and SEQ ID NO: 39 connected sequentially, or SEQ ID NO: 35, SEQ ID NO: 12 and SEQ ID NO: 39 connected sequentially, or SEQ ID NO: 36 and SEQ ID NO: 39 connected sequentially, or SEQ ID NO: 36, SEQ ID NO: 9 and SEQ ID NO: 39 connected sequentially, or SEQ ID NO: 36, SEQ ID NO: 10 and SEQ ID NO: 39 connected sequentially, or SEQ ID NO: 36, SEQ ID NO: 11 and SEQ ID NO: 39 connected sequentially, or SEQ ID NO: 36, SEQ ID NO: 12 and SEQ ID NO: 39 connected sequentially, or SEQ ID NO: 37 and SEQ ID NO: 39 connected sequentially, or SEQ ID NO: 37, SEQ ID NO: 9 and SEQ ID NO: 39 connected sequentially, or SEQ ID NO: 37, SEQ ID NO: 10 and SEQ ID NO: 39 connected sequentially, or SEQ ID NO: 37, SEQ ID NO: 11 and SEQ ID NO: 39 connected sequentially, or SEQ ID NO: 37, SEQ ID NO: 12 and SEQ ID NO: 39 connected sequentially, or SEQ ID NO: 38 and SEQ ID NO: 39 connected sequentially, or SEQ ID NO: 38, SEQ ID NO: 9 and SEQ ID NO: 39 connected sequentially, or SEQ ID NO: 38, SEQ ID NO: 10 and SEQ ID NO: 39 connected sequentially, or SEQ ID NO: 38, SEQ ID NO: 11 and SEQ ID NO: 39 connected sequentially, or SEQ ID NO: 38, SEQ ID NO: 12 and SEQ ID NO: 39 connected sequentially.

The fourth aspect of the present disclosure provides an isolated nucleic acid molecule encoding the long-acting parathyroid hormone receptor agonist fusion protein as described in the first aspect, the second aspect or the third aspect.

The fifth aspect of the present disclosure provides a recombinant expression vector comprising the nucleic acid molecule as described in the fourth aspect.

The sixth aspect of the present disclosure provides an engineered cell comprising the recombinant expression vector as described in the fifth aspect.

In some embodiments of the present disclosure, a host cell of the engineered cell is a eukaryotic cell.

In some embodiments of the present disclosure, the eukaryotic cell is a yeast cell or a mammalian cell.

In some preferred embodiments of the present disclosure, the mammalian cell is a CHO cell or a HEK293 cell.

The seventh aspect of the present disclosure provides a pharmaceutical composition, which comprises the long-acting parathyroid hormone receptor agonist fusion protein as described in the first aspect, the second aspect or the third aspect, the nucleic acid molecule as described in the fourth aspect, the recombinant expression vector as described in the fifth aspect, and the engineered cell as described in the sixth aspect;
optionally, the pharmaceutical composition further comprises a pharmaceutically acceptable carrier and/or excipients.

The eighth aspect of the present disclosure provides a use of the long-acting parathyroid hormone receptor agonist fusion protein as described in the first aspect, the second aspect or the third aspect, the nucleic acid molecule as described in the fourth aspect, the recombinant expression vector as described in the fifth aspect, the engineered cell as described in the sixth aspect or the pharmaceutical composition as described in the seventh aspect in the manufacture of a medicament for treatment, prevention, adjuvant treatment or neoadjuvant treatment of a bone disorder, wherein the bone disorder is a disease requiring treatment, prevention, adjuvant treatment or neoadjuvant treatment by regulating bone formation and/or bone resorption at lesion-associated sites.

In some embodiments of the present disclosure, the bone disorder is osteoporosis, osteopenia, osteogenesis imperfecta, transplantation-associated bone loss, autoimmune-induced bone loss, disuse-induced bone loss, bone injury, degenerative lumbar spondylolisthesis, or a degenerative intervertebral disc disease; the bone injury is preferably bone fracture, bone fissure, cartilage injury, osteonecrosis, or bone injury caused by arthritis or tumors; the bone fracture is preferably osteoporotic fracture, fracture nonunion, or delayed fracture union; the arthritis comprises arthritis caused by degeneration, trauma, overuse, infection, autoimmune diseases or inflammations, such as rheumatoid arthritis, osteoarthritis and infectious arthritis; and the tumors comprise bone tumors and tumor bone metastasis.

The ninth aspect of the present disclosure provides a method for preparing a long-acting parathyroid hormone receptor agonist fusion protein, the method comprises a step of culturing the engineered cell as described in the sixth aspect.

The tenth aspect of the present disclosure provides a method for treatment, prevention, adjuvant treatment or neoadjuvant treatment of a bone disorder by regulating bone formation and/or bone resorption at lesion-associated sites, the method comprises administering to a subject in need thereof an effective amount of the long-acting parathyroid hormone receptor agonist fusion protein as described in the first aspect, the second aspect or the third aspect, the nucleic acid molecule as described in the fourth aspect, the recombinant expression vector as described in the fifth aspect, the engineered cell as described in the sixth aspect or the pharmaceutical composition as described in the seventh aspect.

In some embodiments of the present disclosure, the bone disorder is osteoporosis, osteopenia, osteogenesis imperfecta, transplantation-associated bone loss, autoimmune-induced bone loss, disuse-induced bone loss, bone injury, degenerative lumbar spondylolisthesis, or a degenerative intervertebral disc disease; the bone injury is preferably bone fracture, bone fissure, cartilage injury, osteonecrosis, or bone injury caused by arthritis or tumors; the bone fracture is preferably osteoporotic fracture, fracture nonunion, or delayed fracture union; the arthritis comprises arthritis caused by degeneration, trauma, overuse, infection, autoimmune diseases or inflammations, such as rheumatoid arthritis, osteoarthritis, and infectious arthritis; and the tumors comprise bone tumors and tumor bone metastasis.

In some embodiments of the present disclosure, the method of administration is selected from oral administration (*e.g.*, administration via an oral delivery device) or injection; and the injection is preferably intravenous injection, subcutaneous injection, intramuscular injection, or local injection.

The eleventh aspect of the present disclosure provides a use of the long-acting parathyroid hormone receptor agonist fusion protein as described in the first aspect, the second aspect or the third aspect, the nucleic acid molecule as described in the fourth aspect, the recombinant expression vector as described in the fifth aspect, the engineered cell as described in the sixth aspect or the pharmaceutical composition as described in the seventh aspect as a medicament.

The twelfth aspect of the present disclosure provides the long-acting parathyroid hormone receptor agonist fusion protein as described in the first aspect, the second aspect, or the third aspect, the nucleic acid molecule as described in the fourth aspect, the recombinant expression vector as described in the fifth aspect, the engineered cell as described in the sixth aspect, or the pharmaceutical composition as described in the seventh aspect for use in treatment, prevention, adjuvant treatment or neoadjuvant treatment of a bone disorder.

In some embodiments of the present disclosure, the bone disorder is osteoporosis, osteopenia, osteogenesis imperfecta, transplantation-associated bone loss, autoimmune-induced bone loss, disuse-induced bone loss, bone injury, degenerative lumbar spondylolisthesis or a degenerative intervertebral disc disease; the bone injury is preferably bone fracture, bone fissure, cartilage injury, osteonecrosis, or bone injury caused by arthritis or tumors; the bone fracture is preferably osteoporotic fracture, fracture nonunion, or delayed fracture union; the arthritis comprises arthritis caused by degeneration, trauma, overuse, infection, autoimmune diseases or inflammations, such as rheumatoid arthritis, osteoarthritis and infectious arthritis; and the tumors comprise bone tumors and tumor bone metastasis.

Based on common senses in the art, the above-mentioned preferred conditions can be arbitrarily combined to obtain the preferred embodiments of the present disclosure.

The reagents and raw materials used in the present disclosure are commercially available.

The present disclosure has the following positive and progressive effects:

The long-acting parathyroid hormone receptor agonist fusion protein (LAPAF) of the present disclosure has the effect of inhibiting bone resorption and promoting bone formation, has a long-lasting effect, no catabolic effects, strong bone tissue targeting performance; after multiple administrations, its possesses the efficacy better than that of a combined group of an anti-RANKL monoclonal antibody and teriparatide; it has a fast-acting property, in the experiment of non-human primates, its increase ratio of bone mineral density on D56 after a single administration is much higher than the therapeutic effect of multiple administrations of other osteoporosis treatment drugs on the market; while ensuring the efficacy, it has higher safety, no side effect of persistent hypercalcemia, extremely low risk of mandibular osteonecrosis, and no drug-related toxicity, and is expected to be used for a long time; it significantly reduces the problem of high proportion of PTH functional region breakages in the fusion structure of anti-RANKL antibody and PTH in the prior art, it has high expression level, extremely high preparation yield, low preparation cost and good serum stability, and achieves a better long-term therapeutic effect. At the same time, the long-acting parathyroid hormone receptor agonist fusion protein of the present disclosure can accelerate fracture union, and has better effects in the treatment of diseases such as osteoarthritis caused by bone injuries.

### BRIEF DESCRIPTION OF THE DRAWINGS

Fig.1 is a schematic diagram of an exemplary structure of a dual-targeting fusion protein.
Fig. 2 is a reduced capillary electrophoresis (CE) spectrum of LAPAF LYN101-2 of the present disclosure and the control sample synPTH-α RANKL-1.
Fig. 3 is a mass spectrometry analysis spectrum of control sample synPTH-α RANKL-1.
Fig. 4 is a mass spectrometry analysis spectrum of LAPAF LYN101-1 of the present disclosure.
Fig. 5 is a mass spectrometry analysis spectrum of LAPAF LYN101-2 of the present disclosure.
Fig. 6 is a mass spectrometry analysis spectrum of LAPAF LYN101-3 of the present disclosure.
Fig. 7 shows inhibitory activities of LAPAF LYN101-2 of the present disclosure, denosumab and control sample synPTH-α RANKL-1 on proliferation of Raw264.7 cells subjected to differentiation induced by RANKL.
Fig. 8A shows *in vitro* activities of LAPAF LYN101-2 of the present disclosure, teriparatide and control sample synPTH-α RANKL-1 on PTHR-induced cAMP release in UMR106-RANKL-pool cells.
Fig. 8B shows *in vitro* activities of LAPAF LYN101-2, LYN101-11, LYN101-12, LYN101-13, LYN101-14, LYN101-15, LYN101-17 and LYN101-18 of the present disclosure and teriparatide on PTHR-induced cAMP release in UMR106-RANKL-4 monoclonal cells.
Fig. 9 shows changes in bone mineral density (BMD) in a RANKL-humanized transgenic mouse osteoporosis model caused by ovariectomy (OVX) at different doses of LAPAF LYN101-2 of the present disclosure and control sample synPTH-αRANKL-1. Through one way ANOVA Dunnett's multiple comparison, compared with OVX group, each administration group showed ⁿp>0.05, * p<0.05 and **p<0.01.
Fig. 10 shows changes in bone resorption markers in a RANKL-humanized transgenic mouse osteoporosis model caused by ovariectomy (OVX) at different doses of LAPAF LYN101-2 of the present disclosure and control sample synPTH-αRANKL-1. Through two-way ANOVA Dunnett's multiple comparison, compared with OVX group, each administration group showed ⁿp>0.05 and ****p<0.0001.
Fig. 11 shows changes in bone formation markers in a RANKL-humanized transgenic mouse osteoporosis model caused by ovariectomy (OVX) at different doses of LAPAF LYN101-2 of the present disclosure and control sample synPTH-αRANKL-1. Through two-way ANOVA Dunnett's multiple comparison, compared with OVX group, each administration group showed ⁿp>0.05 and ****p<0.0001.
Fig. 12 shows *in vitro* activity of LAPAF LYN101-2 of the present disclosure and mouse serum at various time points after administration on PTHR-induced cAMP release in UMR106-RANKL-pool cells.
Fig. 13 shows changes in bone mineral density in a RANKL-humanized transgenic mouse osteoporosis model caused by ovariectomy (OVX) after administrating LAPAF LYN101-2 of the present disclosure and the combination of denosumab and teriparatide. Through one way ANOVA Dunnett's multiple comparison, compared with OVX group, Sham group showed ***p<0.001, LYN101-2 10mpk group showed **p<0.01, and combination group of denosumab and teriparatide showed *p<0.05; and compared with the combination group of denosumab and teriparatide, LYN101-2 10mpk showed ^{&&}p<0.01.
Fig. 14 shows CTX-1 levels in a RANKL-humanized transgenic mouse osteoporosis model caused by ovariectomy after administrating LAPAF LYN101-2 of the present disclosure and the combination of denosumab and teriparatide. Through two-way ANOVA Dunnett's multiple comparison, compared with OVX group, each administration group showed *p<0.05, **p<0.01 and **** p<0.0001.
Fig. 15A shows results of percentages of mice with fracture line union in a RANKL-humanized transgenic mouse model with femoral fracture caused by ovariectomy and surgical modeling after LAPAF LYN101-2 of the present disclosure is administrated.
Fig. 15B shows micro-CT results of a fracture site with fracture line union in a RANKL-humanized transgenic mouse model with femoral fractures caused by ovariectomy and surgical modeling after LAPAF LYN101-2 of the present disclosure is administrated.
Fig. 16 shows bone mineral density at a fracture site in a RANKL-humanized transgenic mouse model with femoral fracture induced by ovariectomy (OVX) and surgical modeling when LAPAF LYN101-2 of the present disclosure is continuously administrated for 6 weeks. Through one way ANOVA Dunnett's multiple comparison, compared with OVX group, each administration group showed *p<0.05.
Fig. 17 shows bone mineral density at tibia (non-fracture site) in a RANKL-humanized transgenic mouse model with femoral fracture caused by ovariectomy (OVX) and surgical modeling when LAPAF LYN101-2 of the present disclosure is continuously administrated for 6 weeks. Through one way ANOVA Dunnett's multiple comparison, compared with OVX group, each administration group showed ****p<0.0001.
Fig. 18 shows a mineral apposition rate (MAR) at a fracture site in a RANKL-humanized transgenic mouse model with femoral fracture caused by ovariectomy and surgical modeling when LAPAF LYN101-2 of the present disclosure is continuously administrated for 6 weeks. Through one way ANOVA Dunnett's multiple comparison, compared to teriparatide group, LYN101 0.4 mpk group showed **p<0.01; and LYN101 10mpk group showed ****p<0.0001; and when compared to the combination group of denosumab and teriparatide, LYN101 10mpk group showed **p<0.01.
Fig. 19A shows *in vitro* activity results of LAPAF LYN101-2 of the present disclosure, teriparatide, and control sample synPTH-α RANKL-1 on PTHR-induced cAMP release in UMR106 cells (PTHR⁺/RANKL⁻ cells).
Fig. 19B shows *in vitro* activity results of LAPAF (LYN101-2, LYN101-11, LYN101-12, LYN101-13, LYN101-14, LYN101-15, LYN101-17 and LYN101-18) of the present disclosure and teriparatide on PTHR-induced cAMP release in UMR106 cells (PTHR⁺/RANKL⁻ cells).

### DETAILED DESCRIPTION OF THE PREFERRED EMBODIMENT

### Definitions

As used herein, the term "PTH" or "parathyroid hormone" is a polypeptide secreted by the parathyroid gland, and mature human PTH contains 84 amino acids. The PTH described in the present disclosure refers to mature human PTH, and its amino acid sequence is as shown in amino acids 32-115 of the Uniprot number P01270, that is, PTH (1-84). According to reports in the prior art, mature full-length human PTH (1-84), or truncated PTH such as PTH (1-34) (a generic name of rhPTH (1-34): teriparatide), PTH (1-37), PTH (1-38), PTH (1-41) and PTH (1-20), or mutants of the truncated PTH such as PTH (1-11) mutants and PTH (1-14) mutants all have the function of activating PTHR (parathyroid hormone receptor 1). For example, PTH (1-34) mentioned above refers to a polypeptide formed by amino acids 1-34 of the mature human PTH sequence, *i.e.,* amino acids 32-65 of the Uniprot number P01270; PTH (1-20), PTH (1-37), PTH (1-38), PTH (1-41) and PTH (1-84) refer to a polypeptide formed by amino acids 1-20 of the mature human PTH sequence, amino acids 1-37 of the mature human PTH sequence, amino acids 1-38 of the mature human PTH sequence, amino acids 1-41 of the mature human PTH sequence and amino acids 1-84 of the mature human PTH sequence, respectively. PTH (1-11) mutants and PTH (1-14) mutants refer to mutants of truncated PTH obtained by addition, deletion, modification and/or conservative substitution of at least one amino acid residue such as 1-6 amino acid residues in the mature human PTH sequence, respectively, for example, SEQ ID NO: 31 or SEQ ID NO: 38 of the present disclosure.

As used herein, the terms "PTHR", "PTH1R", "PTHR1", "parathyroid hormone receptor 1" and "parathyroid hormone receptor" are used interchangeably in the present disclosure, and refer to human parathyroid hormone receptor 1 (PTHR1).

As used herein, the term "PTHrP" refers to a parathyroid hormone-related protein (PTHrP), which was originally found to exist in the plasma of patients with humoral hypercalcemia (HHM) caused by malignant tumors, and is a polypeptide containing 141 amino acids (sites 37-177 of Uniprot number P12272). Because 11 amino acids in the N-terminal fragment (sites 37-70 of Uniprot number P12272) composed of 34 residues at the N-terminus of mature human PTHrP (sites 37-177 of Uniprot number P12272) are the same as those in PTH (1-34), which helps to generate similar binding affinity and exert a similar biological effect, and therefore PTHrP also has similar biological activity to parathyroid hormone (PTH), that is, it has the function of activating PTHR. The truncated PTHrP of the present disclosure refers to truncated mature human PTHrP, for example, PTHrP (1-36) refers to a polypeptide formed by amino acids 1-36 of the mature human PTHrP sequence, *i.e.,* amino acids 37-72 of Uniprot number P12272, PTHrP (1-34), PTHrP (1-74) and PTHrP (1-84) refer to amino acids 1-34 of the mature human PTHrP sequence, amino acids 1-74 of the mature human PTHrP sequence, and amino acids 1-84 of the mature human PTHrP sequence, respectively. A mutant of truncated PTHrP refers to a mutant of truncated PTHrP obtained by addition, deletion, modification and/or conservative substitution of at least one amino acid residue such as 1-3 amino acid residues of the amino acids of the truncated mature human PTHrP, such as a PTHrP (7-34) mutant, *i.e.,* a sequence 69 in CN1439021A.

As used herein, the term "Abaloparatide" is an artificially synthetic polypeptide containing 34 amino acids, and is an analog of PTHrP. Its homologies with human PTH (1-34) and human PTHrP (1-34) are 41% and 76%, respectively, and it also has the function of activating PTHR.

As used herein, the term "functional region capable of activating PTHR" refers to a functional fragment that targets PTHR and can activate PTHR, which is selected from an agonistic antigen-binding variable region targeting PTHR (comprising a heavy chain variable region and a light chain variable region, or VHH), an agonistic antigen-binding fragment targeting PTHR, or a PTH functional region, or a combination of the foregoing. The agonistic antigen-binding variable region targeting PTHR is selected from a heavy chain variable region (VH) and a light chain variable region (VL) that target PTHR, or VHH targeting PTHR, and the agonistic antigen-binding fragment of PTHR is selected from Fab or ScFab targeting PTHR, including a heavy chain variable region, a light chain variable region, a heavy chain constant region 1 (CH1) and a light chain constant region (CL). The PTH functional region refers to mature full-length human PTH (such as PTH (1-84)), truncated mature human PTH (such as PTH (1-20), PTH (1-34), PTH (1-37), PTH (1-38), and PTH (1-41)), mutants of truncated mature human PTH (such as PTH (1-11) mutant and PTH (1-14) mutant), truncated mature human PTHrP (such as PTHrP (1-34) and PTHrP (1-36)), or mutants of truncated mature human PTHrP (such as PTHrP (7-34) mutants), or analogs of truncated mature human PTHrP (such as Abaloparatide).

As used herein, the term "anti-RANKL monoclonal antibody" refers to a monoclonal antibody targeting RANKL (Receptor Activator of Nuclear Factor-κ B Ligand). The anti-RANKL monoclonal antibody used in the embodiments of the present disclosure is Denosumab.

As used herein, the term "anti-RANKL antibody" refers to a functional region targeting RANKL (Receptor Activator of Nuclear Factor-κ B Ligand) and having an antibody or half-antibody structure, which is one of the components of a dual/multi-targeting antibody or a dual/multi-targeting fusion protein.

As used herein, the term "anti-RANKL functional region" or "functional region|targeting RANKL" refers to an antigen-binding fragment targeting RANKL, such as a heavy chain variable region and a light chain variable region (such as Fv or ScFv) that target RANKL, VHH (SDA) targeting RANKL, or a Fab region targeting RANKL (such as Fab or ScFab, including a heavy chain variable region, a light chain variable region, a heavy chain constant region 1 (CH1) and a light chain constant region (CL)).

As used herein, the term "dual-targeting fusion protein" refers to an Fc fusion protein targeting RANKL and PTHR, which comprises or consists of an anti-RANKL functional region, a functional region capable of activating PTHR and an Fc region. The functional region capable of activating PTHR is an agonistic antigen-binding variable region (including ScFv, SDA, or Fv) targeting PTHR, an agonistic antigen-binding fragment (including ScFab or Fab) targeting PTHR, or a PTH functional region; when the functional region capable of activating PTHR is a PTH functional region, the PTH functional region can be connected to the anti-RANKL functional region or the Fc region of human IgG through a Linker, either directly or through a site-specific coupling technology. When the agonistic antigen-binding fragment comprising the anti-RANKL functional region or targeting PTHR is Fab or ScFab, a person of ordinary skill in the art can also use a common dual antibody technology such as Crossmab to optimize the structure of the Fab region, for example interchanging VH and VL in Fab, or interchanging CH1 and CL in Fab. The CH2 and CH3 domains of the IgG constant region and the hinge region constitute the Fc region. As used herein, the term "dual-targeting fusion protein of the present disclosure" refers to a dual-targeting fusion protein with structure 1.

As used herein, the terms "long-acting parathyroid hormone receptor agonist fusion protein", "Long-Acting PTHR Agonist Fusion protein" and "LAPAF" can be used interchangeably in the present disclosure. They refer to the dual-targeting fusion protein of the present disclosure, or a fusion protein comprising the dual-targeting fusion protein of the present disclosure sequence and targeting bone tissue cells (such as osteoblasts) expressing RANKL, such as a triple-targeting/multi-targeting fusion protein, or a dual-targeting fusion protein of the present disclosure or triple-targeting/multi-targeting fusion protein coupled with a polypeptide, a small molecule compound, an oligonucleotide and other molecules.

As used herein, the term "silencing of Fc immune effector function" refers to weakening or eliminating the ADCP and/or ADCC and/or CDC effects of the Fc region, and common Fc mutants of IgG1 with silencing of a Fc immune effector function comprise LALA mutation (L234A/L235A), PGLALA mutation (L234A, L235A and P329G), N297A, etc., and Fc mutants of IgG4 comprises one or more of E233P/F234V/L235A/D265A/R409K mutations, etc. The numbering of Fc region residues herein adopts the numbering of the EU index in Kabat.

As used herein, the term "connection" refers to the connection of two peptide chains into a single peptide chain via a peptide bond or a peptide chain, or the connection of two peptide chains via a site-specific coupling technology.

As used herein, the term "Fab" consists of a light chain variable region (VL) domain, a light chain constant region (CL) domain, a heavy chain variable region (VH) domain and a heavy chain constant region 1 (CH1) domain. Fab can bind to an antigen, and comprises two peptide chains, chain a and chain b; when the chain a is VH-CH1, the chain b is VL-CL, and vice versa; when the chain a is VH-CL, the chain b is VL-CH1, and vice versa.

As used herein, the term "ScFab" refers to a single peptide chain which is formed by connecting the two peptide chains of the above-mentioned Fab via a Linker.

As used herein, the term "Fv" comprises two peptide chains which are a heavy chain variable region (VH) and a light chain variable region (VL), respectively.

As used herein, the term "ScFv" refers to a single peptide chain which is formed by connecting a heavy chain variable region (VH) and a light chain variable region (VL) via a Linker.

As used herein, the term "SDA" or "VHH" refers to an antigen-binding region of a single domain antibody (nanobody), that is, a nanobody heavy chain variable region.

As used herein, the term "complementary determining region" or "CDR" refers to an amino acid residue responsible for antigen binding in a variable region of an antibody. The heavy chain variable region CDRs (HCDR1, HCDR2 and HCDR3) and light chain variable region CDRs (LCDR1, LCDR2 and LCDR3) described in the present disclosure are defined by a Kabat numbering system. However, as is well known in the art, the CDR regions can also be determined based on heavy chain/light chain variable region sequences by other systems such as Chothia and IMGT, AbM and other numbering systems/methods. It should be noted that the boundaries of the CDRs of the same antibody variable region obtained by different numbering systems may be different. That is, the CDR sequences of the same antibody variable region defined by different numbering systems are different. Therefore, when it comes to defining an antibody having a specific CDR sequence defined in the present disclosure, the range of the antibody also covers such antibodies whose variable region sequences comprise specific CDR sequences. But due to the use of different schemes (*e.g.*, different numbering system rules or combinations thereof), the claimed CDR boundaries claimed by the schemes are different from the specific CDR boundaries defined in the present disclosure.

As used herein, the term "Knob(s)-into-Hole(s) technology", or "knob-into-hole" technology, or "protrusion-into-cavity" technology or "knob-hole" technology refers to a fact that a genetic engineering technology is used to introduce different mutations into two CH3 domains of heavy chains, so as to promote heterodimerization of the heavy chain, a knob is made on one heavy chain, a hole is made on the other heavy chain, and then the knob and the hole preferentially engage with each other to form an asymmetric antibody (Ridgway JB, et al. 'Knobs-into-holes' engineering of antibody CH3 domains for heavy chain heterodimerization. Protein Engineering, 1996, 9(7): 617-621). As known to those skilled in the art, multiple knobs and/or holes can be made on one heavy chain, and correspondingly, multiple holes and/or knobs can be made on the other heavy chain.

As used herein, the term "host cell" generally includes a single cell, a cell line or a cell culture, which comprises the polynucleotide or recombinant expression vector disclosed in the present application, or expresses the long-acting parathyroid hormone receptor agonist fusion protein of the present disclosure. The host cell may include the offspring of a single host cell. Due to natural, accidental or intentional mutations, the offspring may not necessarily be completely identical to the original parent cell (in morphology or in a genomic total DNA complement). The host cell may include a cell transfected *in vitro* with the vector disclosed in the present application. The host cell is a eukaryotic cell, such as a yeast cell, a HEK293 cell, a COS cell, a Chinese hamster ovary (CHO) cell, a HeLa cell or a myeloma cell. In some embodiments, the host cell is a mammalian cell. In some embodiments, the mammalian cell is a CHO cell or a HEK293 cell.

As used herein, the term "vector" generally refers to a nucleic acid molecule which can replicate itself in a suitable host. The vector can transfer an inserted nucleic acid molecule into a host cell and/or between host cells. This term can include vectors that are mainly used to insert DNA into cells, vectors that are mainly used to replicate DNA, and expression vectors that are used for transcription and/or translation of DNA or RNA. Nucleic acid molecules encoding different chains of the long-acting parathyroid hormone receptor agonist fusion protein of the present disclosure can be in the same DNA molecule or in different DNA molecules. Vectors that provide more than one of the above functions are also included. "Expression vector" is a polynucleotide that can be transcribed and translated into a polypeptide when introduced into a suitable host cell. The host cell herein generally comprises an expression vector with a desired expression yield.

The term "composition" refers to a composition comprising the long-acting parathyroid hormone receptor agonist fusion protein of the present disclosure and optional adjuvants, excipients or a pharmaceutically acceptable carrier. The composition may comprise the pharmaceutically acceptable carrier. The composition may exist in any form of pharmaceutical formulation, including but not limited to injections, powders, freeze-dried powders, etc., or in the form of formulations that can be placed in an oral delivery therapeutic device. The composition in the form of the pharmaceutical formulation is also referred to herein as a "pharmaceutical composition", which can be prepared according to conventional techniques of pharmaceutics, including mixing a pharmaceutically active ingredient, the long-acting parathyroid hormone receptor agonist fusion protein of the present disclosure, with a drug carrier to be prepared into a desired dosage form according to conventional techniques of pharmaceutics. The composition may also comprise an expression vector of a nucleic acid molecule encoding the long-acting parathyroid hormone receptor agonist fusion protein and an optional pharmaceutically acceptable carrier.

The term "therapeutant" refers to the long-acting parathyroid hormone receptor agonist fusion protein or composition of the present disclosure.

The term "effective amount" or "therapeutically effective amount" refers to an amount of a long-acting parathyroid hormone receptor agonist fusion protein or composition (*e.g*., a composition comprising the long-acting parathyroid hormone receptor agonist fusion protein as described herein or a long-acting parathyroid hormone receptor agonist fusion protein) sufficient to achieve intended applications (including but not limited to disease treatment, disease prevention, and disease alleviation). The therapeutically effective amount may vary according to the intended applications (*e.g*., *in vitro* or *in vivo*) or a subject and disease condition being treated, such as the weight and age of the subject, the severity of the disease condition and the method of administration, and can be easily determined by a person of ordinary skill in the art. This term can also be applied to doses that can induce a specific response (*e.g.*, target gene induction, proliferation and/or apoptosis) in target cells. The specific dose will vary according to a specific molecule selected, a dosing regimen followed, whether it is administered in combination with other molecules, the time arrangement of administration, a tissue to which it is administered, and a physical delivery system in which it is administered.

The terms "treatment" or "alleviation" or "improvement" are used interchangeably herein and refer to methods of obtaining beneficial or desired results (including but not limited to therapeutic benefits and/or preventive benefits). As used herein, the therapeutic benefits generally refer to eradication or reduction in the severity of the underlying condition being treated. In addition, the therapeutic benefits are achieved by eradication or reduction in severity, or reduction in the incidence of one or more physiological symptoms associated with the underlying condition, such that an improvement is observed in the subject (although the subject may still be afflicted with the underlying condition). For the preventive benefits, a composition may be administered to a subject at risk of developing a particular disease, or a subject reporting one or more physiological symptoms of a disease, to reduce the risk of the subject developing the particular disease, even though a diagnosis of the disease may not have yet been made. "Adjuvant treatment" refers to treatment given after surgery, and "neoadjuvant treatment" refers to treatment given before surgery.

As used herein, the term "administration" refers to delivering a therapeutically effective amount of therapeutants or control samples to a subject. Administration can be systemic or topical. Administration can be performed by an administration device, such as a syringe or a therapeutant oral delivery device. Methods of administration include, but are not limited to, oral administration, injection, etc. Routes of administration include intravenous, subcutaneous or intramuscular administration, local injection, etc.

As used herein, the terms "co-administration", "administration in combination with...", or "used in combination" and grammatical equivalents thereof generally include administration of two or more agents to a subject such that the agents and/or their metabolites are present in the subject at the same time. Co-administration includes simultaneous administration in a separate composition, administration in the separate composition at different time points, or administration in a composition in which both agents exist.

As used herein, the term "subject" or "individual" or "animal" or "patient" refers to a human or non-human animal, including mammals or primates, desired for diagnosis, prognosis, alleviation, prevention and/or treatment of a disease or condition. Mammalian subjects include humans, livestock animals, farm animals, and zoo, sports or pet animals, such as dogs, cats, guinea pigs, rabbits, rats, mice, horses, pigs, cows and bears.

As used herein, the term *"in vivo"* generally refers to events that occur within the body of a subject (*in vivo*)*.*

As used herein, the term "*in vitro*" generally refers to events that occur outside the body of a subject (*in vitro* or *ex vivo*)*.* For example, an *in vitro* assay includes any assay performed outside the body of the subject. The *in vitro* assay includes cell-based assays using dead or live cells. The *in vitro* assay also includes cell-free assays without using intact cells.

Next, the present disclosure will be further illustrated by means of examples, but the present disclosure is not limited to the scope of the examples. The experimental methods in the following examples without specifying specific conditions are carried out according to conventional methods and conditions, or are selected according to the product specifications.

### Example 1: Structural design of dual-targeting fusion protein and construction of expression vector

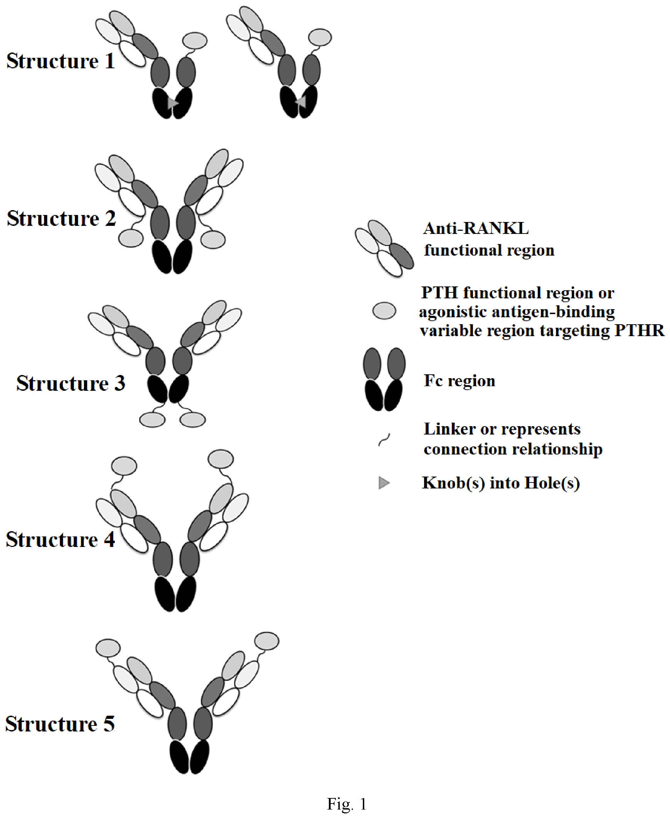

The present disclosure designed dual-targeting fusion proteins with various structures. The structural forms of these designed dual-targeting fusion proteins are shown in Fig. 1. In Fig. 1, Knob(s) and Hole(s) in a Knob(s)-into-Hole(s) structure can be interchanged in two Fc regions.

The dual-targeting fusion protein and control sample synPTH-α RANKL-1 were prepared by directly synthesis after codon optimization based on protein sequences by GENEWIZ and General Biol, ligating into a pCDNA3.4 plasmid, and confirming by sequencing. The above-mentioned different expression plasmids were mixed and paired to transfect expression cells to obtain the dual-targeting fusion proteins or control samples (see Table 1). Subsequent experimental materials were obtained by extraction after transfection of expression cells with this series of plasmids.

**Table 1 Exemplary molecular sequences of dual-targeting fusion proteins and control samples**

| **Name of dual-targeting fusion proteins or control sample** | **Amino acid composition or source of sample** |
|---|---|
| LYN101-1 (Structure 1) | Heavy chain of the first arm: consisting of SEQ ID NO: 1, 14 and 7 connected sequentially |
| | Light chain of the first arm: consisting of SEQ ID NO: 2 and 15 connected sequentially |
| | The second arm: consisting of SEQ ID NO: 5 and 8 connected sequentially |
| LYN101-2 (Structure 1) | Heavy chain of the first arm: consisting of SEQ ID NO: 1, 14 and 7 connected sequentially |
| | Light chain of the first arm: consisting of SEQ ID NO: 2 and 15 connected sequentially |
| | The second arm: consisting of SEQ ID NO: 5, 9 and 8 connected sequentially |
| | Heavy chain of the first arm: consisting of SEQ ID NO: 1, 14 and 7 connected sequentially |
| LYN101-3 (Structure 1) | Light chain of the first arm: consisting of SEQ ID NO: 2 and 15 connected sequentially |
| | The second arm: consisting of SEQ ID NO: 5, 12 and 8 connected sequentially |
| LYN101-4 (Structure 2) | Chain 1: consisting of SEQ ID NO: 1, 14 and 6 connected sequentially |
| | Chain 2: consisting of SEQ ID NO: 2, 15, 10 and 5 connected sequentially |
| LYN101-5 (Structure 3) | Chain 1: consisting of SEQ ID NO: 1, 14, 6, 11 and 5 connected sequentially |
| | Chain 2: consisting of SEQ ID NO: 2 and 15 connected sequentially |
| LYN101-6 (Structure 4) | Chain 1: consisting of SEQ ID NO: 5, 11, 1, 14 and 6 connected sequentially |
| | Chain 2: consisting of SEQ ID NO: 2 and 15 connected sequentially |
| LYN101-7 (Structure 5) | Chain 1: consisting of SEQ ID NO: 1, 14 and 6 connected sequentially |
| | Chain 2: consisting of SEQ ID NO: 5, 10, 2 and 15 connected sequentially |
| LYN101-8 (Structure 1) | Heavy chain of the first arm: consisting of SEQ ID NO: 3, 14 and 7 connected sequentially |
| | Light chain of the first arm: consisting of SEQ ID NO: 4 and 15 connected sequentially |
| | The second arm: consisting of SEQ ID NO: 5, 9 and 8 connected sequentially |
| | Heavy chain of the first arm: consisting of SEQ ID NO: 1, 14 and 7 connected sequentially |
| LYN101-9 (Structure 1) | Light chain of the first arm: consisting of SEQ ID NO: 2 and 15 connected sequentially |
| | The second arm: consisting of SEQ ID NO: 31, 9 and 8 connected sequentially |
| | Heavy chain of the first arm: consisting of SEQ ID NO: 1, 14 and 7 connected sequentially |
| LYN101-10 (Structure 1) | Light chain of the first arm: consisting of SEQ ID NO: 2 and 15 connected sequentially |
| | The second arm: consisting of SEQ ID NO: 32, 9 and 8 connected sequentially |
| | Heavy chain of the first arm: consisting of SEQ ID NO: 1, 14 and 7 connected sequentially |
| LYN101-11 (Structure 1) | Light chain of the first arm: consisting of SEQ ID NO: 2 and 15 connected sequentially |
| | The second arm: consisting of SEQ ID NO: 33, 9 and 8 connected sequentially |
| | Heavy chain of the first arm: consisting of SEQ ID NO: 1, 14 and 7 connected sequentially |
| LYN101-12 (Structure 1) | Light chain of the first arm: consisting of SEQ ID NO: 2 and 15 connected sequentially |
| | The second arm: consisting of SEQ ID NO: 34, 9 and 8 connected sequentially |
| | Heavy chain of the first arm: consisting of SEQ ID NO: 1, 14 and 7 connected sequentially |
| LYN101-13 (Structure 1) | Light chain of the first arm: consisting of SEQ ID NO: 2 and 15 connected sequentially |
| | The second arm: consisting of SEQ ID NO: 35, 9 and 8 connected sequentially |
| | Heavy chain of the first arm: consisting of SEQ ID NO: 1, 14 and 7 connected sequentially |
| LYN101-14 (Structure 1) | Light chain of the first arm: consisting of SEQ ID NO: 2 and 15 connected sequentially |
| | The second arm: consisting of SEQ ID NO: 36, 9 and 8 connected sequentially |
| | Heavy chain of the first arm: consisting of SEQ ID NO: 1, 14 and 7 connected sequentially |
| LYN101-15 (Structure 1) | Light chain of the first arm: consisting of SEQ ID NO: 2 and 15 connected sequentially |
| | The second arm: consisting of SEQ ID NO: 37, 9 and 8 connected sequentially |
| LYN101-16 (Structure 1) | Heavy chain of the first arm: consisting of SEQ ID NO: 1, 14 and 7 connected sequentially |
| | Light chain of the first arm: consisting of SEQ ID NO: 2 and 15 connected |
| | sequentially |
| | The second arm: consisting of SEQ ID NO: 38, 9 and 8 connected sequentially |
| LYN101-17 (Structure 1) | Heavy chain of the first arm: consisting of SEQ ID NO: 1, 14 and 8 connected sequentially |
| | Light chain of the first arm: consisting of SEQ ID NO: 2 and 15 connected sequentially |
| | The second arm: consisting of SEQ ID NO: 5, 9 and 7 connected sequentially |
| | Heavy chain of the first arm: consisting of SEQ ID NO: 1, 14 and 39 connected sequentially |
| LYN101-18 (Structure 1) | Light chain of the first arm: consisting of SEQ ID NO: 2 and 15 connected sequentially |
| | The second arm: consisting of SEQ ID NO: 5, 9 and 40 connected sequentially |
| synPTH-α RANKL-1 (control sample) | Light chain: SEQ ID NO: 8 in US8,992,925B2 |
| | Heavy chain: SEQ ID NO: 10 in US8,992,925B2 |
| Signal peptide | SEQ ID NO: 13 |
| Teriparatide | Purchased Recombinant teriparatide for injection produced by Shanghai United Cell Biotechnology, Co., Ltd. (Trade name: Ostiogen) |
| Denosumab | Purchased Denosumab Injection produced by Amgen Manufacturing Limited (Trade name: Prolia) |

### Example 2: Preparation of expression plasmids, cell transfection and expression and purification of target proteins

### 1. Preparation of expression plasmids

Glycerol bacteria containing expression plasmids (obtained by adding 0.5 mL of 60% sterile glycerol solution to 1 mL of Escherichia coli bacterial liquid containing expression plasmids to be mixed thoroughly) were inoculated into a liquid LB medium in a ratio of 1:1000 to undergo shake cultivation for 16 h at 37°C and 220 rpm, and then the cultivated glycerol bacteria were centrifuged to collect thalli. The collected thalli were extracted by using an EndoFree Plasmid Maxi Kit (DP117, purchased from Tiangen Biotech (Beijing) Co., Ltd.) according to a standard process provided in a kit instruction to obtain the expression plasmids.

### 2. Cell transfection and protein expression

After the obtained expression plasmids were filtered with a 0.22 µm filter membrane, 50 µg of plasmids (wherein a mass ratio of expression plasmids of a first arm heavy chain, a first arm light chain and a second arm chain of a dual-targeting fusion protein of the present disclosure (a dual-targeting fusion protein with structure 1) was 1:2:2; and a mass ratio of expression plasmids of chains 1 and chains 2 of dual-targeting fusion proteins with structure 2, structure 3, structure 4 and structure 5 was 2:3) were aspirated and added into 2 mL of Opti PRO SFM Medium (GIBCO) to be mixed evenly. 160 µL of transfection reagent Expi Fectamine CHO Reagent was aspirated and added to 2 mL of Opti PRO SFM Medium to be mixed evenly. The obtained transfection reagent mixed solution was added into the mixed solution containing the plasmids to be mixed evenly. The mixture of the plasmids and the transfection reagent was slowly and evenly added to 50 mL of a host cell ExpiCHO-S (Thermo Fisher) suspension with a cell density of 6× 10⁶ viable cells/mL, and cultured in an incubator at 37°C and 8% CO₂. On day 1 (18-22 h later), 300 µL of ExpiCHO Enhancer and 8 mL of ExpiCHO Feed were added, and the culture temperature was decreased to 32°C; on day 5, 8 mL of ExpiCHO Feed was added for the second feeding, and the cells were harvested after 12 days of culture. The cell suspension was centrifuged at 8000 rpm for 15 min, and the supernatant obtained after centrifugation, *i.e.,* a cell culture harvest liquid, was used for purification of a target protein.

### 3. Protein purification

### 1) Sample capture (Protein A affinity capture)

The cell culture harvest liquid was centrifuged at 10,000 rpm for 30 min to remove cells and their debris, then loaded onto a Protein A affinity column (GE Healthcare), and eluted to obtain a target protein. Protein purity was detected by SDS-PAGE.

The purification method of Protein A affinity capture was a conventional protein purification method well known to those skilled in the art. The specific experimental method can refer to GE Healthcare Protein A product instructions and GE Antibody Purification Manual.

### 2) Sample refined purification

a) The following method was applicable to a dual-targeting fusion protein with structure 1 (the dual-targeting fusion protein of the present disclosure).

A chromatography column (anti-kappa chromatography column) was equilibrated with an equilibration buffer (50 mM Tris-HC1, 150 mm NaCl, pH7.2) until an UV detection line was stable; the sample was loaded with a sample pump, the retention time was 5 min, and the loading capacity was ≤30 mg/mL; the target protein was eluted by using an eluent (0.1 M Gly-HCl, pH2.7), the pH value of the eluent was adjusted to 5.5 with 1 M Tris, and the protein purity was detected by SDS-PAGE.

The chromatography column (Sperdex 200 pg chromatography column (Cytiva 16/600)) was equilibrated with an equilibration buffer (50 mM NaAC-HAC, pH 5.5) until the UV detection line was stable; the sample was loaded with a sample pump, the retention time was 120 min, the sample volume was ≤2 mL, and the loading capacity was ≤30 mg; the chromatography column was rinsed with the equilibration buffer (50 mM NaAC-HAC, pH 5.5); proteins of different sizes were subjected to fractional collection based on UV280, and the protein purity was detected by SDS-PAGE and SEC-HPLC. The dual-targeting fusion proteins of the present disclosure after refined purification as described above were used for studies in Examples 3-12.

b) The following method was applicable to the dual-targeting fusion proteins with structure 2, structure 3, structure 4 and structure 5.

A chromatography column (Sperdex 200 pg chromatography column (Cytiva 16/600)) was equilibrated with an equilibration buffer (50 mM NaAC-HAC, pH 5.5) until the UV detection line was stable; the sample was loaded with a sample pump, the retention time was 120 min, the sample volume was ≤2 mL, and the loading capacity was ≤30 mg; the chromatography column was rinsed with the equilibration buffer (50 mM NaAC-HAC, pH 5.5); proteins of different sizes were subjected to fractional collection based on UV280, and the protein purity was detected by SDS-PAGE and SEC-HPLC. The dual-targeting fusion proteins after refined purification as described above were used for study in Example 3.

c) The following method is applicable to control sample synPTH-α RANKL-1.

First, a chromatography column was treated with a disinfectant liquid (0.5 M NaOH), then a chromatography column (AKTAAVANT/Pure (cytiva) cation chromatography column) was rinsed with an equilibration buffer (20 mM HAC-NaAC, pH5.0), and sample loading was started after UV absorption reading was stable. After the sample loading was completed, the chromatography column was continued to be rinsed with the equilibration buffer. Then the chromatography column was rinsed with a leacheate (50 mM HAc-NaAc+0-0.5 M NaCl, pH5.0), and then rinsed with an elution buffer (20 mM HAc-NaAc+1.5 M NaCl, pH5.0). After the UV absorption reading was raised, the eluted sample was collected until the UV reading decreased and tended to be stable. Then the chromatography column was rinsed respectively with a disinfectant liquid and an equilibration solution, and finally was preserved with a preservation solution (20% ethanol). The sample subjected to refined purification was subsequently used for studies in Examples 3, 4, 5 and 6.

### Example 3: Analysis of protein structural stability of long-acting parathyroid hormone receptor agonist fusion protein

### 1) Expression titer analysis

The expression titer (expression amount) of the dual-targeting fusion protein in the supernatant of the cell culture harvest liquid in Example 2 was detected by a high performance liquid chromatography (HPLC) method. The cell culture harvest liquids of the dual-targeting fusion protein and a control sample were centrifuged at 13,000 rpm for 10 min and then the supernatant was separately taken and detected. The specific detection method was as follows: the expression titer in cell supernatant was evaluated by using HPLC (Agilent 1260) and Protein A affinity chromatography column (Thermo Fisher/POROS 20A), wherein mobile phase A: 50 mM PBS+150 mM NaCl, mobile phase B: 12 mM HCl+150 mM NaCl, then gradient elution was carried out, the detection time was 3 min, the flow rate was 2.0 mL/min, the column temperature was 25°C, the sample loading amount is 10 µL, and the detection wavelength was 280 nm. The detection results are shown in Table 2.

**Table 2 Expression titer of dual-targeting fusion proteins**

| Type of structure of the dual-targeting fusion protein | Name of the dual-targeting fusion protein | SEQ ID NO of Anti-RANKL functional region | SEQ ID NO of Linker | SEQ ID NO of PTH functional region | Expression titer (g/L) |
|---|---|---|---|---|---|
| | LYN101-1 | 1+2 | Connected directly | 5 | 0.66 |
| | LYN101-2 | 1+2 | 9 | 5 | 0.83 |
| | LYN101-8 | 3+4 | 9 | 5 | 0.88 |
| | LYN101-3 | 1+2 | 12 | 5 | 0.71 |
| Structure 1 | LYN101-9 | 1+2 | 9 | 31 | 0.9 |
| | LYN101-10 | 1+2 | 9 | 32 | 1.0 |
| | LYN101-11 | 1+2 | 9 | 33 | 0.85 |
| | LYN101-12 | 1+2 | 9 | 34 | 0.8 |
| | LYN101-13 | 1+2 | 9 | 35 | 0.84 |
| | LYN101-14 | 1+2 | 9 | 36 | 0.82 |
| | LYN101-15 | 1+2 | 9 | 37 | 0.72 |
| | LYN101-16 | 1+2 | 9 | 38 | 0.92 |
| | LYN101-17 | 1+2 | 9 | 5 | 0.86 |
| | LYN101-18 | 1+2 | 9 | 5 | 0.85 |
| Structure 2 | LYN101-4 | 1+2 | 10 | 5 | 0.50 |
| Structure 3 | LYN101-5 | 1+2 | 11 | 5 | 0.34 |
| Structure 4 | LYN101-6 | 1+2 | 11 | 5 | 0.54 |
| | synPTH-α RANKL-1 | / | / | / | 0.51 |
| Structure 5 | LYN101-7 | 1+2 | 10 | 5 | 0.07 |

As shown in Table 2, the detection results were:

When the structure of the dual-targeting fusion protein was structure 1, its expression titer (expression titer ≥ 0.66 g/L) was significantly higher than that of other structural types (expression titer 0.07 g/L-0.54 g/L).

When the sequences of the anti-RANKL functional regions were the same, among the dual-targeting fusion proteins of different structural types or Linkers or PTH functional regions, LYN101-10 had the highest titer (expression titer 1 g/L), which was significantly higher than the titers of other dual-targeting fusion proteins, such as the expression titer of LYN101-7 with structure 5 (expression titer 0.07 g/L).

When the structures, Linkers, PTH functional regions and Fc regions of the dual-targeting fusion proteins were the same, the replacement of the anti-RANKL functional regions had almost no impact on the protein yield, *i.e.,* the expression titers were at the same level. As shown in Table 2 data, the expression titers of LYN101-2 and LYN101-8 were both greater than 0.8 g/L, which were at a relatively high expression level.

When the structures, anti-RANKL functional regions, Linkers and Fc regions of the dual-targeting fusion proteins were the same, changing the length (11 amino acids to 84 amino acids) or type (PTH or PTHrP) of the PTH functional region had almost no impact on protein yield, *i.e*., the expression titers were at the same level (expression titer ≥0.72g/L).

When the structures, anti-RANKL functional regions, Linkers and PTH functional regions of the dual-targeting fusion proteins were the same, changing Fc region sequences (human IgG2, IgG4, Fc immune effector-silenced IgG1) or interchanging the Fc sequences of the first and second arms did not affect the expression titers of the proteins, *i.e*., the expression titer was at the same level. As shown by data in Table 2, the expression titers of LYN101-17, LYN101-18 and LYN101-2 were all greater than 0.8 g/L, which were at a relatively high expression level.

In summary, among the dual-targeting fusion proteins with different structures, the dual-targeting fusion protein with structure 1 (*i.e*., the dual-targeting fusion protein of the present disclosure) had the highest protein expression amount (expression titer), indicating that the dual-targeting fusion protein of the present disclosure (LAPAF) could achieve a relatively high expression level, a relatively high yield and relatively low production cost; when the dual-targeting fusion protein had structure 1 (*i.e*., the dual-targeting fusion protein of the present disclosure), and when the Linker selected Linker1 (SEQ ID NO: 9), its protein expression amount (yield) was relatively higher; and the changes in the anti-RANKL functional region, the PTH functional region (length or type) and the Fc region of the dual-targeting fusion protein of the present disclosure did not affect the expression amount (yield) of the dual-targeting fusion protein of the present disclosure (LAPAF).

### 2) Structural stability analysis of PTH functional region

### 2.1) CE-SDS analysis

The sample after purifying with protein A was further analyzed by reduced CE-SDS to determine an impurity ratio. The steps were as follows: the sample was diluted with water to 2.5 mg/mL, 40 µL of sample as described above was taken to prepare a reduced sample, and then the reduced sample prepared was heated at 70°C for 10 min, and the sample was loaded for CE-SDS separation. The equipment was SCIEX PA800Plus, a detector was a UV detector, and the detection wavelength was 220 nm. The detection results are shown in Table 3.

**Table 3 Analysis of breakage ratio of PTH functional region of dual-targeting fusion protein**

| Structural type of the dual-targeting fusion protein | Name of the dual-targeting fusion protein | PTH impurity proportion % |
|---|---|---|
| Structure 1 | LYN101-1 | 23 |
| | LYN101-2 | 17 |
| | LYN101-3 | 25 |
| Structure 2 | LYN101-4 | 33 |
| Structure 3 | LYN101-5 | 36 |
| Structure 4 | LYN101-6 | 50 |
| | synPTH-α RANKL-1 | 50 |

| | | |
|---|---|---|
| Note: a PTH impurity ratio was calculated based on the percentage of a peak area, wherein the PTH impurity referred to a peak whose separation time was slightly earlier than that of a PTH chain, and the PTH impurity ratio referred to a ratio of an area of a peak whose separation time was slightly earlier than that of a PTH chain (a chain containing the PTH functional region) to an area of a PTH-related total peak. To make the dual-targeting fusion proteins with different structures comparable, the PTH functional regions of the dual-targeting fusion proteins listed in Table 3 were all PTH(1-34), *i.e*., the sequence was as shown in SEQ ID NO: 5. | | |

As shown in Table 3, the results were:

When the PTH functional region sequences were the same, when the structure type of the dual-targeting fusion protein was structure 1, the PTH impurity ratio was the lowest (≤25%), which was significantly lower than that of other structure types of dual-targeting fusion proteins, such as structure 4 (the PTH impurity ratios of control sample synPTH-α RANKL-1 and LYN101-6 were up to 50%). It could be seen that the PTH functional region of the dual-targeting fusion protein of the present disclosure (LAPAF) had the lowest breakage ratio during the preparation, and the PTH functional region had the highest structural stability. At the same time, the change in the anti-RANKL functional region in the dual-targeting fusion protein of the present disclosure (LAPAF) did not affect the structural stability of the PTH functional region in the dual-targeting fusion protein of the present disclosure (LAPAF). For example, the dual-targeting fusion protein of the present disclosure (LAPAF) LYN101-8, like LYN101-2, also had the characteristics of low breakage ratio and high structural stability of the PTH functional region.

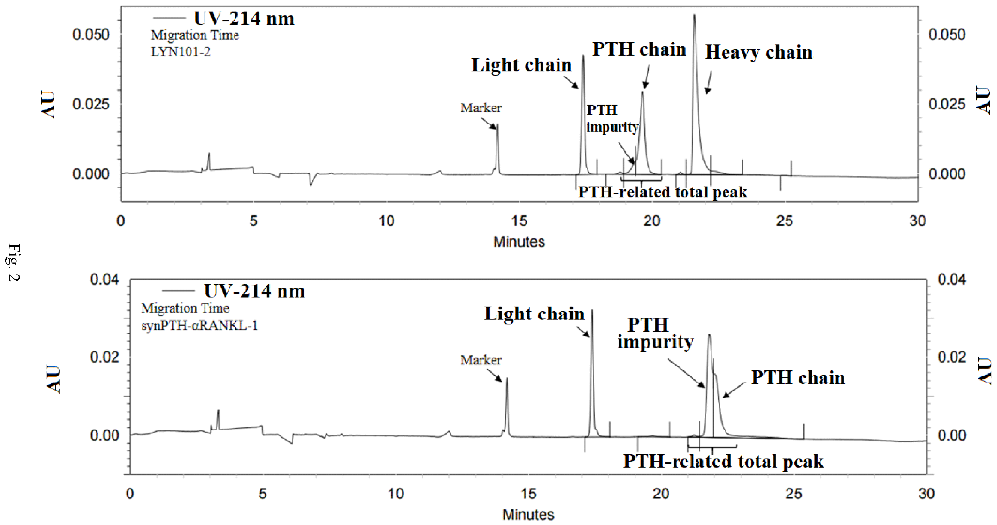

In addition, when the PTH functional region was PTH(1-34), among the dual-targeting fusion proteins of the present disclosure (LAPAFs), LYN101-2 (when the Linker sequence selected Linker1 (SEQ ID NO: 9)) had the best PTH functional region structural stability, and the PTH impurity ratio was only 17% which was much lower than that of control sample synPTH-α RANKL-1 (50%). The reduced CE spectra of LYN101-2 and control sample synPTH-α RANKL-1 were shown in Fig. 2. As shown in Fig. 2, a peak area of a main peak of control sample synPTH-α RANKL-1 heavy chain (PTH chain) was basically equivalent to the peak area of the PTH impurity. While an impurity peak (PTH impurity) of a second arm (PTH chain) of the dual-targeting fusion protein of the present disclosure LYN101-2 had a peak area significantly lower than the peak area of the main peak (PTH chain). It could be seen that the PTH functional region in the dual-targeting fusion protein of the present disclosure (LAPAF) LYN101-2 had high structural stability and a low breakage ratio.

At the same time, the present disclosure also analyzed the structural stability of PTH functional regions with other lengths or types in the dual-targeting fusion proteins of the present disclosure (LAPAFs), such as LYN101-9 with a PTH impurity ratio of only 1%.

In addition, the present disclosure also analyzed the influence of changes in the Fc region of the dual-targeting fusion protein of the present disclosure on the structural stability of the PTH functional region in the dual-targeting fusion protein of the present disclosure (LAPAF). The results showed that, compared to LYN101-2, the breakage ratios of PTH functional regions of the dual-targeting fusion proteins of the present disclosure (LAPAFs) LYN101-17 and LYN101-18 had no significant difference.

In summary, among the dual-targeting fusion proteins with different structures, the dual-targeting fusion protein with structure 1 (*i.e*., the dual-targeting fusion protein of the present disclosure) had the lowest PTH functional region breakage ratio and the most stable structure; when the dual-targeting fusion protein had structure 1 (*i.e*., the dual-targeting fusion protein of the present disclosure) and the PTH functional region was PTH(1-34), and when a Linker sequence selected Linker1 (SEQ ID NO: 9), it had the lowest breakage ratio and the most stable structure; and the changes in the anti-RANKL functional region, the PTH functional region or the Fc region in the dual-targeting fusion protein of the present disclosure did not affect or hardly affect the structural stability of the PTH functional region in the dual-targeting fusion protein of the present disclosure (LAPAF).

### 2.2) Mass spectrometry analysis

In order to further confirm the breakage form of a molecule, the inventors conducted mass spectrometry molecular weight analysis on the dual-targeting fusion protein samples of the present disclosure and control samples. The N-glycosylation modification of a test sample was removed with a PNGaseF enzyme and then the sample was injected for analysis. The mass spectrometer utilized Thermo QE, the scanning mode was a positive ion mode, the scanning range was: 2000-4000 m/z, mobile phase A was a 0.1% FA aqueous solution, mobile phase B was a 0.1% FA acetonitrile solution, gradient elution was carried out, the flow rate was 0.3 mL/min, the chromatography column was a desalting column (Waters Mass PREPTM Micro Desalting), the column temperature was 80°C, and the data analysis software was Thermo's Biopharma Finder4.0.

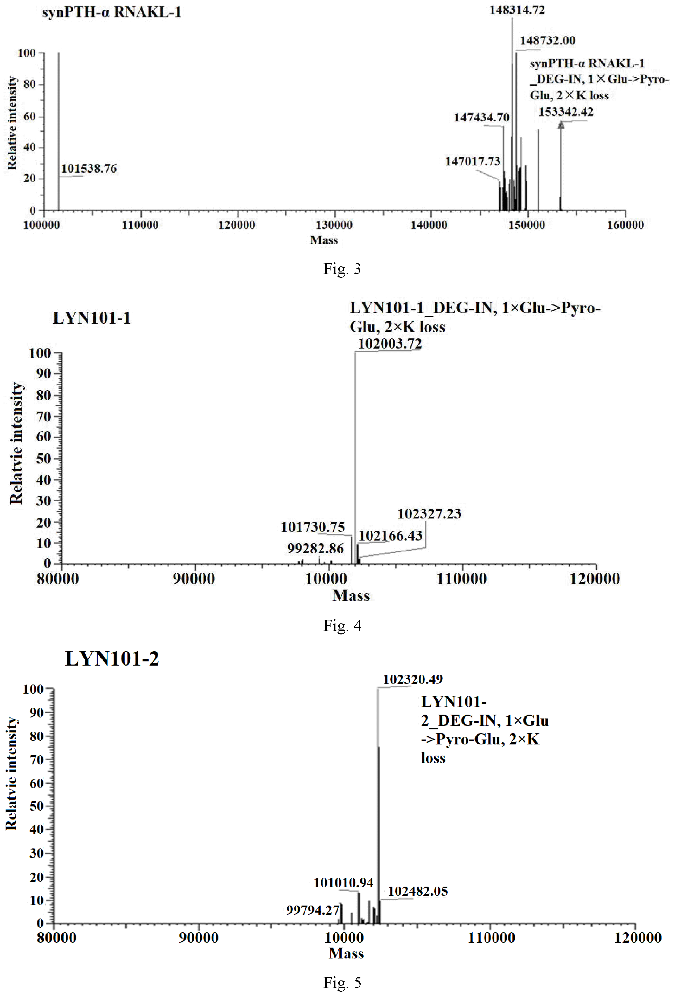

As shown in Fig. 3, control sample synPTH-α RANKL-1 had a relatively low target peak (153342.42Da) intensity of the complete molecule after deglycosylation, and had a large number of non-target molecular peaks with relatively high intensity. By matching the sequence with the measured value, it was speculated that control sample synPTH-α RANKL-1 had different severe degrees of breakage at different sites in the PTH functional region. Correspondingly, as shown in Fig. 4, Fig. 5 and Fig. 6, the mass spectrometry detection results of the dual-targeting fusion proteins of the present disclosure (LAPAFs) LYN101-1, LYN101-2 and LYN101-3 after deglycosylation showed that the target peaks (102003.72, 102320.49, and 103264.58, respectively) of the complete molecules were all the peaks with the highest intensity, and the peak intensities of low molecular weight impurities were extremely low.

Through the mass spectrometry analysis of the dual-targeting fusion protein of the present disclosure and the control sample, it was verified again that the dual-targeting fusion protein of the present disclosure (LAPAF) had a stable protein structure, an extremely low PTH functional region breakage ratio and an extremely high target protein yield.

According to the above-mentioned expression titer analysis and protein structural stability analysis such as PTH functional region structural stability analysis, it could be found that the dual-targeting fusion protein of the present disclosure (LAPAF) had the highest expression titer and the lowest PTH functional region breakage ratio. Therefore, it could be expected that the dual-targeting fusion protein of the present disclosure would ultimately obtain the most target proteins, the highest yield, the highest output, and the lowest production cost under the same preparation process. Changing the anti-RANKL functional region, or the Linker or the PTH functional region or the Fc region had little or no impact on the expression titer and the PTH functional region structural stability of the dual-targeting fusion protein of the present disclosure (LAPAF). Among them, when the PTH functional region was PTH(1-34), LYN101-2 was a preferred molecule with the highest expression titer and the lowest PTH functional region breakage ratio in the dual-targeting fusion protein of the present disclosure (LAPAF).

### Example 4: Inhibitory activity of LAPAF on RANKL-induced osteoclast proliferation

In this example, RANKL was used to induce differentiation of Raw264.7 cells (mouse mononuclear macrophage leukemia cells) into osteoclasts for testing the abilities of the dual-targeting fusion proteins of the present disclosure LYN101-1, LYN101-2, LYN101-3, LYN101-8, LYN101-9, LYN101-10, LYN101-11, LYN101-12, LYN101-13, LYN101-14, LYN101-15, LYN101-16, LYN101-17 and LYN101-18 to inhibit the binding of RANKL to cell surface RANK. By taking LYN101-2 as an example, the following method was applicable to all long-acting parathyroid hormone receptor agonist fusion proteins of the present disclosure (LAPAFs), control sample synPTH-α RANKL-1 and an anti-RANKL monoclonal antibody.

Raw264.7 cells in the logarithmic growth phase (purchased from ATCC, Catalog No. TIB-71) were taken and washed once with 6 mL of Dulbecco's phosphate-buffered saline (DPBS), and then 3 mL of preheated trypsin was added. The cells were digested in an incubator at 37°C and 5% CO₂ for 6-10 min. After the cells were completely digested, adherent cells were scraped off with a cell scraper, and then 10 mL of DMEM complete medium was added into the remaining cells for gentle purging. Raw264.7 cells were prepared into a cell suspension, and the cell density was adjusted to 5×10⁴ cells/mL, then the cell suspension was added into a 96-well cell culture plate at 100 µL/well and incubated in a constant temperature incubator (NUNRE/NU-8700E) at 37°C and 5% CO₂ for 1 h. The samples to be tested (the dual-targeting fusion protein of the present disclosure LYN101-2, denosumab and synPTH-α RANKL-1) were pre-diluted to 15 nM as a starting concentration, and then diluted downward by 1.5 times, with a total of 11 concentration gradients. Then an equal volume of 200 ng/mL RANKL diluent (purchased from Sino Biological, Catalog No.: 11682-HNCH) to sample dilution wells, mixed well, and incubated in a constant temperature incubator at 37°C and 5% CO₂ for 2 h. The above-mentioned incubated mixed sample was put into each well of a 96-well culture plate paved with Raw264.7 cells at 100 µL/well, and then incubated in a constant temperature incubator at 37°C and 5% CO₂ for (96±3) h. After the incubation was ended, the supernatant was discarded, then a 100 µL/well Citrate buffer (purchased from Sigma, Catalog No.: C3434-250G) was added, and the samples were lysized on a shaker at 450 rpm for 40 min. A 80 µL/well supernatant was added into an 80 µL/well prepared pNPP solution (purchased from Santa, Catalog No.: sc-3720) to be mixed well, the obtained mixture was incubated in a constant temperature incubator at 37°C and 5% CO₂ for 30 min, and then 40 µL/well 2.5 M NaOH (purchased from Sinopharm, Catalog No.: 10019718) was added to terminate the reaction. The 96-well cell culture plate was placed in a microplate reader (Molecular Devices\Spectra Max M2) and vibrated for 30 s, and the absorbance of the well plate at 405 nm were read and recorded. The data was analyzed by using Graphpad Prism software to obtain an IC50 value.

The results showed that the inhibitory activity of the dual-targeting fusion proteins of the present disclosure LYN101-1, LYN101-2, LYN101-3, LYN101-8, LYN101-9, LYN101-10, LYN101-11, LYN101-12, LYN101-13, LYN101-14, LYN101-15, LYN101-16, LYN101-17 and LYN101-18 on the proliferation of Raw264.7 cells induced to differentiate into osteoclasts by RANKL was comparable to the activity of the anti-RANKL monoclonal antibody (such as denosumab) and control sample synPTH-α RANKL-1, *i.e.,* the dual-targeting fusion protein of the present disclosure (LAPAF) could block the binding of RANKL and RANK, thereby inhibiting osteoclast activation and inhibiting bone resorption.

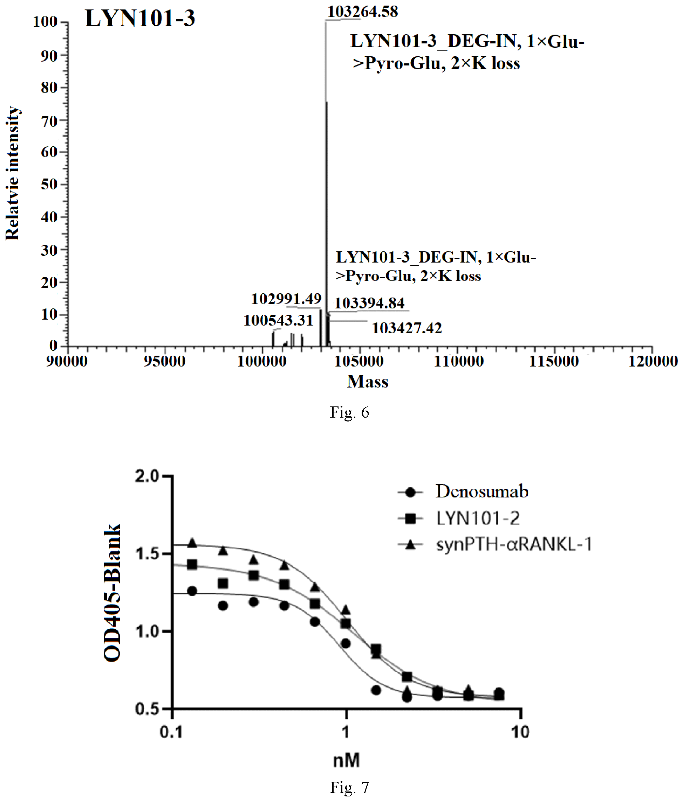

As shown in Fig. 7, the inhibitory activity of the dual-targeting fusion protein of the present disclosure LYN101-2 on the proliferation of Raw264.7 cells induced to differentiate by RANKL was comparable to the activities of Denosumab (trade name: Prolia, an anti-RANKL monoclonal antibody) and control sample synPTH-α RANKL-1, *i.e.,* the long-acting parathyroid hormone receptor agonist fusion protein LYN101-2 could block the binding of RANKL and RANK, thereby inhibiting osteoclast activation and inhibiting bone resorption.

In addition, the present disclosure also simultaneously detected the dual-targeting fusion proteins of the present disclosure LYN101-1, LYN101-3, LYN101-8, LYN101-9, LYN101-10, LYN101-11, LYN101-12, LYN101-13, LYN101-14, LYN101-15, LYN101-16, LYN101-17 and LYN101-18, which had different anti-RANKL functional regions, Linkers, PTH functional regions or Fc regions from LYN101-2. The results were similar to those of LYN101-2, with EC50 of about 1 nM. It could be seen that replacing the anti-RANKL functional region, Linker, the length or type (PTH or PTHrP) of the PTH functional region and the Fc region in the dual-targeting fusion protein of the present disclosure (LAPAF) did not affect the inhibitory activity of the dual-targeting fusion protein of the present disclosure (LAPAF) on RANKL-induced osteoclast proliferation, and the activity of the dual-targeting fusion protein (LAPAF) after replacement was equivalent to the activities of the anti-RANKL monoclonal antibody and control sample synPTH-α RANKL-1. The dual-targeting fusion protein (LAPAF) after replacement could well block the binding of RANKL and RANK, thereby inhibit osteoclast activation and inhibit bone resorption. In summary, the dual-targeting fusion protein of the present disclosure (LAPAF) had obvious inhibitory activity on the proliferation of Raw264.7 cells induced to differentiate by RANKL and its activity was equivalent to that of Denosumab. It could block the binding of RANKL and RANK, thereby inhibit osteoclast activation and inhibit bone resorption. Replacing the anti-RANKL functional region in the dual-targeting fusion protein of the present disclosure (LAPAF), or adjusting the length or type of the PTH functional region, or the Linker, or the Fc region had no impact on the inhibitory activity of the dual-targeting fusion protein of the present disclosure (LAPAF) on RANKL-induced osteoclast proliferation.

### Example 5: In vitro activity of LAPAF on PTHR-induced cAMP release in PTHR⁺/RANKL⁺ cells

### 1) Construction of UMR106-RANKL cell line for LAPAF in vitro activity

In this example, an UMR106-RANKL stable cell line was constructed by using the following method.
a) After UMR106 (purchased from ATCC, Catalog No.: CRL-1661) cells were washed once with 6 mL of DPBS, 3 mL of preheated trypsin was added therein to digest the cells in an incubator at 37°C and 5% CO₂ for 5 min. After the cells were completely digested, 10 mL of DMEM complete medium was added into the digested cells for gentle purging. The above cells were prepared into a cell suspension, and the cell density was adjusted to 1×10⁶ cells/mL. The adjusted cell suspension was added into each well of a 6-well cell culture plate at 1.5 mL/well and cultured overnight in a constant temperature incubator (NUNRE/NU-8700E) at 37°C and 5% CO₂.
b) The next day, UMR106 cells paved in advance were infected with RANKL lentivirus at a MOI of 50. After 48 h, 2 µg/mL puromycin was added for pressure screening. The cells were subjected to medium refreshing treatment (DMEM+10% FBS+2 µg/mL puromycin) every 2-3 days. After 2 weeks, a stably transfected cell pool expressing RANKL (UMR106-RANKL-pool) was obtained.
c) After the above RANKL stable cell pool was washed once with 6 mL of DPBS, 3 mL of preheated trypsin was added to digest the cells for 5 min in an incubator at 37°C and 5% CO₂. After the cells were completely digested, 10 mL of medium (DMEM+10% FBS+2 µg/mL puromycin) was added into the digested cells for gentle purging. The above cells were prepared into a cell suspension, then the cell suspension was diluted to 1 cell/well and paved in a 96-well plate at 100 µL/well, and cultured in a constant temperature incubator (NUNRE/NU-8700E) at 37°C and 5% CO₂ for 2 weeks. During the culture, the supernatant in the 96-well plate was aspirated every 2-3 days, and the medium (DMEM+10% FBS+2 µg/mL puromycin) was added at 100 µL/well. The wells with monoclonal cells were picked and marked during the culture. After the monoclonal cells in the 96-well plate were full of wells, 30 clones were picked and transferred to the 6-well plate for culture. After the cells in the 6-well plate were full of wells, they were expanded to T25. At the same time, some cells were taken to detect the expression level of RANKL by flow cytometry to obtain UMR106-RANKL monoclonal cell UMR106-RANKL-4.

### 2) Functional verification of UMR106-RANKL cell line for LAPAF in vitro activity

a) After the above-mentioned UMR106-RANKL-pool or UMR106-RANKL-4 monoclonal cells were washed once with 6 mL of DPBS, 3 mL of preheated trypsin was added therein to digest the cells in an incubator at 37°C and 5% CO₂ for 5 min. After the cells were completely digested, 10 mL of DMEM complete medium was added into the digested cells for gentle purging. The above-mentioned cells were prepared into a cell suspension, the cell density was adjusted to 1×10⁶ cells/mL, and the cell suspension was added into each well of a 96-well U-shaped plate at 100 µL/well for standby use.
b) Denosumab was diluted to 20 nM as a starting concentration, and then diluted downward 5 times, for a total of 6 concentration gradients. Then the cells treated in step a) were centrifuged at 1000 rpm for 5 min, and the supernatant was discarded. The above-mentioned antibody was added into the plate at 100 µL/well, and the cells were incubated at 4°C for 1 h and then centrifuged at 1000 rpm for 5 min, and the supernatant was discarded.
c) After the hIgG(H+L)-AlexaFluor^{®}488 fluorescent secondary antibody was diluted 500 times, the above-mentioned cells were added at 100 µL/well. The cells were incubated at 4°C for 1 h and centrifuged at 1000 rpm for 5 min, and then the supernatant was discarded. The cells were resuspended with a buffer at 100 µL/well.
d) The above 96-well plate was placed on a BD C6 flow cytometer, and the MFI of FL1-H was read and recorded. The data was analyzed by using Graphpad Prism software.

Denosumab (anti-RANKL monoclonal antibody) and the RANKL stably transfected cell pool (UMR106-RANKL-pool) bound in a gradient, proving that the stably transfected RANKL pool was successfully constructed. The binding curve of denosumab to RANKL stably transfected monoclonal cell UMR106-RANKL-4 was similar to that of UMR106-RANKL-pool cells, proving that the RANKL stably transfected monoclonal cell was successfully constructed.

To simplify the description, UMR106-RANKL-pool and UMR106-RANKL-4 were collectively referred as UMR106-RANKL, which referred to UMR106-RANKL-pool or UMR106-RANKL-4.

### 3) In vitro activity detection of LAPAF on PTHR-induced cAMP release in UMR106-RANKL (PTHR⁺/RANKL⁺) cells

In this example, the *in vitro* activity of the dual-targeting fusion proteins of the present disclosure LYN101-1, LYN101-2, LYN101-3, LYN101-8, LYN101-9, LYN101-10, LYN101-11, LYN101-12, LYN101-13, LYN101-14, LYN101-15, LYN101-16, LYN101-17 and LYN101-18 on PTHR-induced cAMP release in UMR106-RANKL cells was determined by using the following method. By taking LYN101-2 as an example, the following method was applicable to all the long-acting parathyroid hormone receptor agonist fusion proteins of the present disclosure (LAPAFs), control sample synPTH-α RANKL-1, teriparatide and agonistic antibodies targeting PTHR.

One day in advance, UMR106-RANKL-pool or UMR106-RANKL-4 cells were washed once with 6 mL of DPBS, and then 3 mL of preheated trypsin was added therein to digest the cells in an incubator 37°C and 5% CO₂ for 5 min. After the cells were completely digested, 10 mL of DMEM complete medium was added into the digested cells for gentle purging. The above two cells were separately prepared into cell suspensions, and the cell density was adjusted to 3×10⁵ cells/mL. The cell suspension was added into a 96-well cell culture plate at 100µL/well and cultured overnight in a constant temperature incubator (NUNRE/NU-8700E) at 37°C and 5% CO₂. The next morning, the supernatant in the 96-well culture plate was discarded, and then DMEM/F12 medium was added. The plate was placed in a constant temperature incubator at 37°C and 5% CO₂ to incubate the cells for 1 h, and then the supernatant was discarded. The samples to be tested (LYN101-2, teriparatide and control sample synPTH-α RANKL-1) were diluted to a starting concentration (the starting concentration of the sample was 100 nM), and then diluted downward 5 times, with a total of 8 concentration gradients (components of the diluent: DMEM/F12+1 mM IBMX+0.1% BSA). The above samples were added to each well of the 96-well cell culture plate paved with UMR106-RANKL-pool or UMR106-RANKL-4 cells at 100 µL/well. The plate was placed in a constant temperature incubator at 37°C and 5% CO₂ for 40 min of incubation. Then, 1×lysis buffer was added at 130 µL/well and the cells were incubated at room temperature for 30 min to obtain the sample to be tested (cell lysate supernatant). The subsequent operation was performed according to the cAMP Parameter Assay Kit (purchased from R&D, Catalog No.: SKGE002b). Briefly described as follows, a primary antibody in the kit was at 50 µL/well into a 96-well plate pre-coated with Goat Anti-mouse at 50 µL/well to be incubated at room temperature for 1 h at 500 rpm/min, and the plate was washed. Then 100 µL/well cell lysate supernatant and 50 µL/well cAMP Conjugate were added to incubate the cells at room temperature for 2 h at 500 rpm/min, and the plate was washed. After the substrate was added to react at room temperature for 30 min, a stop solution was added, and the absorbance at 450/570 nm was read and recorded using a microplate reader. The data was analyzed using Graphpad Prism software to obtain an EC50 value.

The results showed that the *in vitro* activity of control sample synPTH-α RANKL-1 on PTHR-induced cAMP release in UMR106-RANKL cells (PTHR⁺/RANKL⁺ cells) was comparable to that of teriparatide, but at the same molar concentration, the dual-targeting fusion proteins of the present disclosure (LAPAFs) LYN101-1, LYN101-2, LYN101-3, LYN101-8, LYN101-11, LYN101-12, LYN101-13, LYN101-14, LYN101-15, LYN101-17 and LYN101-18 were obviously superior to teriparatide in *in vitro* activities on PTHR-induced cAMP release in UMR106-RANKL cells (PTHR⁺/RANKL⁺ cells). The experimental results showed that for double positive cells (PTHR⁺/RANKL⁺ cells) expressing both PTHR and RANKL, at the same molar concentration, the dual-targeting fusion proteins of the present disclosure (LAPAFs) LYN101-1, LYN101-2, LYN101-3, LYN101-8, LYN101-11, LYN101-12, LYN101-13, LYN101-14, LYN101-15, LYN101-17 and LYN101-18 had stronger *in vitro* activity on PTHR-induced cAMP release than teriparatide or control sample synPTH-α RANKL-1. It was indicated that the dual-targeting fusion protein of the present disclosure (LAPAF) had a stronger effect on PTHR⁺/RANKL⁺ cells simulating osteoblasts, and could improve the effect of the drug on osteoblasts.

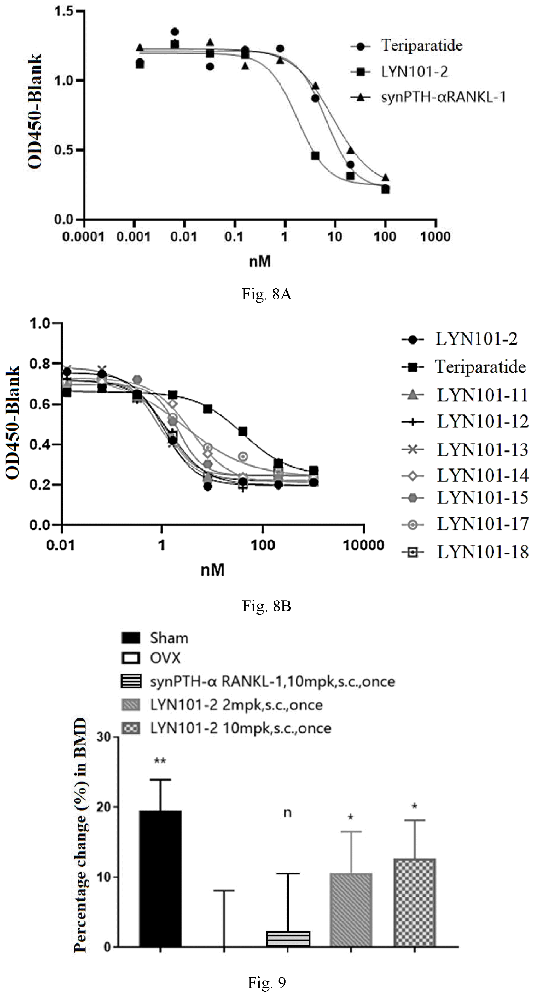

As shown in Fig. 8A, in the *in vitro* activity of PTHR-induced cAMP release in UMR106-RANKL-pool cells (PTHR⁺/RANKL⁺ cells), the activity of the dual-targeting fusion protein of the present disclosure (LAPAF) LYN101-2 is superior to that of teriparatide (EC50_{LYN101-2}=1.771nM, EC50_{teriparatide}=6.532nM, and EC50_{LYN101-2}<EC50_{teriparatide}), while the activity of control sample synPTH-α RANKL-1 is comparable to that of teriparatide (EC50_{synPTH-α RANKL-1}=8.969nM, EC50_{teriparatide}=6.532nM, EC50_{synPTH-α RANKL-1}≈EC50_{teriparatide}).

At the same time, in order to evaluate whether different selections of the anti-RANKL functional region, Linker, PTH functional region and Fc region in the dual-targeting fusion protein of the present disclosure (LAPAF) have an effect on the *in vitro* activity experimental results of PTHR-induced cAMP release in UMR106-RANKL cells, the inventors also simultaneously detected LYN101-8 having a different anti-RANKL functional region from LYN101-2, LYN101-1 and LYN101-3 having different linkers from LYN101-2, LYN101-11, LYN101-12, LYN101-13, LYN101-14, and LYN101-15 having different PTH functional regions from LYN101-2, and LYN101-17 and LYN101-18 having different Fc regions from LYN101-2. The results showed that the *in vitro* activity results of LYN101-8, LYN101-1, LYN101-3, LYN101-11, LYN101-12, LYN101-13, LYN101-14, LYN101-15, LYN101-17 and LYN101-18 on PTHR-induced cAMP release in UMR106-RANKL cells were similar to that of LYN101-2. It could be seen that changes in the anti-RANKL functional region, or linker, or PTH functional region or Fc region did not affect the *in vitro* activity of the dual-targeting fusion protein of the present disclosure (LAPAF) on PTHR-induced cAMP release in UMR106-RANKL cells (PTHR⁺/RANKL⁺ cells). And the dual-targeting fusion proteins exhibited superior efficacy compared with teriparatide.

The specific experimental results were as follows: the *in vitro* activity of the dual-targeting fusion protein of the present disclosure (LAPAF) LYN101-8 (having anti-RANKL functional region different from that in LYN101-2) on PTHR-induced cAMP release in UMR106-RANKL-pool cells (PTHR⁺/RANKL⁺ cells) was similar to that of LYN101-2, *i.e.,* the *in vitro* activity of LYN101-8 on PTHR-induced cAMP release in UMR106-RANKL-pool cells was stronger than that of teriparatide and control sample synPTH-α RANKL-1. The *in vitro* activity of LYN101-8 on PTHR-induced cAMP release in UMR106-RANKL-pool cells was comparable to that of LYN101-2. For example, the activation activity EC50s of LYN101-8 and LYN101-2 on PTHR-induced cAMP *in vitro* activation in UMR106-RANKL-pool cells were 1.7 nM (LYN101-2) and 1.4 nM (LYN101-8), respectively. It could be seen that changing the anti-RANKL functional region did not affect the superiority of the dual-targeting fusion protein of the present disclosure (LAPAF) on the *in vitro* activity of PTHR-induced cAMP release in PTHR⁺/RANKL⁺ cells compared with teriparatide.

The *in vitro* activities of the dual-targeting fusion proteins of the present disclosure (LAPAFs) LYN101-1 and LYN101-3 (selecting a Linker different from LYN101-2) on PTHR-induced cAMP release in UMR106-RANKL-pool cells (PTHR⁺/RANKL⁺ cells) were similar to that of LYN101-2, *i.e.,* the *in vitro* activities of LYN101-1 and LYN101-3 on PTHR-induced cAMP release in UMR106-RANKL-pool cells was stronger than those of teriparatide and control sample synPTH-α RANKL-1. It could be seen that changing the Linker did not affect the superiority of the *in vitro* activity of the targeted fusion protein of the present disclosure (LAPAF) on PTHR-induced cAMP release in PTHR⁺/RANKL⁺ cells compared with teriparatide.

In addition, in order to evaluate whether the length or type of the PTH functional region and the Fc region in the dual-targeting fusion protein of the present disclosure (LAPAF) had an effect on the *in vitro* activity of PTHR-induced cAMP release in UMR106-RANKL cells (PTHR⁺/RANKL⁺ cells), the inventors also simultaneously detected the *in vitro* activity of the dual-targeting fusion proteins of the present disclosure (LAPAFs) LYN101-11, LYN101-12, LYN101-13, LYN101-14, LYN101-15, LYN101-17 and LYN101-18 having the length or type (PTH or PTHrP) of the PTH functional region or the Fc region different from LYN101-2 on PTHR-induced cAMP release in UMR106-RANKL-4 cells (PTHR⁺/RANKL⁺ cells). The results showed that, as shown in Fig. 8B, the *in vitro* activities of the dual-targeting fusion proteins of the present disclosure (LAPAFs) LYN101-2, LYN101-11, LYN101-12, LYN101-13, LYN101-14, LYN101-15, LYN101-17 and LYN101-18 -were all superior to that of teriparatide regarding PTHR-induced cAMP release in UMR106-RANKL-4 cells (PTHR⁺/RANKL⁺ cells). For example, EC50_{teriparatide}=39.7 nM, EC50_{LYN101-12}=1.4 nM, and EC50_{LYN101-14}=3.6 nM, which were similar to the results of LYN101-2. It could be seen that changing the length or type of the PTH functional region and changing the Fc region also did not affect the superiority of the dual-targeting fusion protein of the present disclosure (LAPAF) on the *in vitro* activity of PTHR-induced cAMP release in PTHR⁺/RANKL⁺ cells compared with teriparatide.

### Example 6: Pharmacodynamic and pharmacokinetic experiments for evaluating single administration of test substance in RANKL-humanized transgenic mouse osteoporosis model caused by ovariectomy

In this example, the efficacy of a single administration of the dual-targeting fusion proteins of the present disclosure LYN101-1, LYN101-2, LYN101-3, LYN101-8, LYN101-9, LYN101-10, LYN101-11, LYN101-12, LYN101-13, LYN101-14, LYN101-15, LYN101-16, LYN101-17 and LYN101-18 in a RANKL-humanized transgenic mouse osteoporosis model caused by ovariectomy was evaluated by using the following method. By taking LYN101-2 as an example, the following method was applicable to all the long-acting parathyroid hormone receptor agonist fusion proteins of the present disclosure (LAPAFs).

30 RANKL-humanized transgenic mice (purchased from Biocytogen Jiangsu Gene Biotechnology Co., Ltd., B-hRANKL mice, Catalog No.: 111072) were randomly divided into 6 groups, including G1 Sham group (n=3), G2 OVX group (model group) (n=6), G3 control sample synPTH-α RANKL-1 10 mpk group (n=6), G4 LYN101-2 2 mpk group (n=6), G5 LYN101-2 10 mpk group (n=6), and G6 LYN101-2 10 mpk group (n=3). The Sham group underwent sham surgery, and other groups underwent ovariectomy to obtain osteoporosis mouse models. Drug administration began 4 weeks after surgery, and the day of drug administration was counted as Day 0; G1 and G2 groups were subcutaneously administered with an equal amount of Buffer (a normal saline solution containing 0.005% Tween 80), and G3-G6 groups were subcutaneously administered with a drug once. Serum was collected from G1 and G2 groups on D28 (*i.e.,* Day, called D for short) (before modeling), D21, D14 and D7; serum was collected from G1-G5 groups on D0 (before administration), 6 h, 24 h, D2, D3, D5, D7, D14, D21, D28, D42, and D56 after administration; serum was collected from G6 group at 312 h, 360 h, 1200 h and 1368 h after administration. Serum from G1-G5 groups was used for detecting PD markers (osteocalcin, TRAP-5b), and serum from G6 group was used for PTH functional activity detection; at the endpoint of the experiment, tibia, femur and vertebra samples from G1-G5 groups were collected for standby use. Tibial bone mineral density (BMD) was detected. The algorithm for the change percentage of bone mineral density was calculated as follows: change percentage of BMD in Sham group=(Sham group BMD-OVX group BMD)/Sham group BMD*100, the change percentage of BMD in synPTH-α RANKL-1 group=(BMD of synPTH-α RANKL-1 group-OVX group BMD)/OVX group BMD, the change percentage of BMD in LYN101-2, 2 mpk group=(BMD of LYN101-2, 2 mpk group-OVX group BMD)/OVX group BMD, and the change percentage of BMD in LYN101-2, 10 mpk group=(BMD of LYN101-2, 10 mpk group-OVX group BMD)/OVX group BMD.

By taking LYN101-2 as a representative of the dual-targeting fusion protein of the present disclosure (LAPAF) and synPTH-α RANKL-1 as a representative comparator of a sample. this example showed the pharmacodynamic experimental results of a single administration of LAPAF and control sample in the RANKL-humanized transgenic mouse osteoporosis model caused by ovariectomy.

As shown in Fig. 9, the bone mineral density of the proximal tibia of the mice decreased significantly after modeling (see OVX group, model group). On D56 (week 8), the control sample synPTH-α RANKL-1 had an extremely weak effect on improving the bone mineral density of the proximal tibia of mice (p>0.05), while the 2 mpk and 10 mpk groups of the dual-targeting fusion protein of the present disclosure (LAPAF) LYN101-2 had a very significant effect on improving the bone mineral density of the proximal tibia of mice (p<0.05, p<0.05), and there was a dose-effect trend. It could be seen that 8 weeks (D56) after a single administration, the dual-targeting fusion protein of the present disclosure (LAPAF) was significantly better than the control sample synPTH-α RANKL-1 in improving the bone mineral density of mice in osteoporosis model caused by ovariectomy. This suggested that a single administration of the dual-targeting fusion protein of the present disclosure (LAPAF) had a better and long-lasting effect on improving bone mineral density, and no catabolic effects were observed after continuous infusion of teriparatide, or abaloparatide, or TransCon PTH.

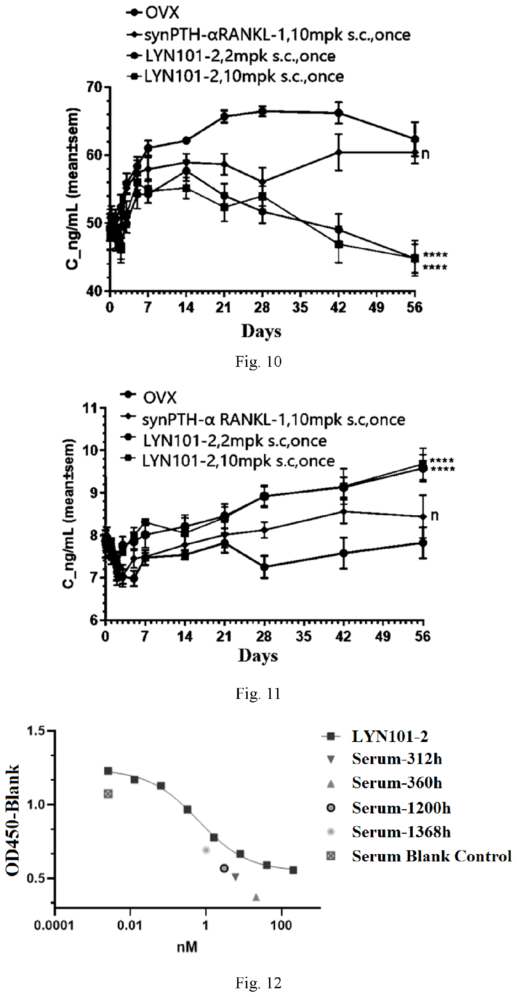

As shown in Fig. 10, the level of tartrate-resistant acid phosphatase 5b (TRAP5b), a bone resorption marker, in the serum of mouse was significantly increased after modeling (data not shown). On D56 (week 8), the levels of TRAP5b in the serum of control sample synPTH-α RANKL-1 and OVX group were basically equivalent, while the levels of TRAP5b in the 2 mpk and 10 mpk groups of the dual-targeting fusion protein of the present disclosure (LAPAF) LYN101-2 were significantly down-regulated compared with that in OVX group (p<0.0001, p<0.0001). It could be seen that 8 weeks after a single administration, the dual-targeting fusion protein of the present disclosure (LAPAF) LYN101-2 could effectively inhibit the activity of osteoclasts, and the efficacy was significantly better than that of the control sample synPTH-α RANKL-1. This indicated that a single administration of the dual-targeting fusion protein of the present disclosure (LAPAF) had superior and long-lasting effects on the levels of bone resorption markers. And it was further verified that the dual-targeting fusion protein of the present disclosure (LAPAF) did not generate catabolic effects after continuous infusion of teriparatide or abaloparatide, or TransCon PTH in the model of this example.

As shown in Fig. 11, the level of osteocalcin, a bone formation marker, decreased significantly after modeling (data not shown). On D56 (week 8), the levels of osteocalcin in the serum of the control sample synPTH-α RANKL-1 and the OVX group were basically equivalent, while the levels of osteocalcin in serum of the 2mpk and 10 mpk groups of the dual-targeting fusion protein of the present disclosure (LAPAF) LYN101-2 were significantly up-regulated compared with that in OVX group (p<0.0001, p<0.0001). It could be seen that 8 weeks after a single administration, the dual-targeting fusion protein of the present disclosure (LAPAF) LYN101-2 could still maintain a high efficacy in promoting bone formation, and the efficacy was significantly better than that of the control sample synPTH-α RANKL-1. This indicated that the single administration of the dual-targeting fusion protein of the present disclosure (LAPAF) has a superior and long-lasting effect on the level of bone formation markers.

Meanwhile, the inventors also investigated the blood drug exposure condition of the dual-targeting fusion protein of the present disclosure (LAPAF) LYN101-2 in mice and the activity of the corresponding PTH functional region. The inventors mixed the serum of 3 mice at each serum collection time point in LYN101-2 10 mpk group (G6 group) and diluted the mixed serum by 10 times to detect its *in vitro* activity on the PTHR-induced cAMP release in UMR106-RANKL-pool cells (a PTH functional activity experiment, the specific detection method was shown in Example 5). The results were shown in Fig. 12. The serum at each time point still maintained a strong *in vitro* activity on the PTHR-induced cAMP release in UMR106-RANKL-pool cells. On D56 (week 8, 1368 h) after single administration, the serum that was diluted by 10 times still had a strong PTH functional activity, and the activity was equivalent to the LYN101-2 1 nM level.

In summary, the present disclosure unexpectedly found that LAPAF (the dual-targeting fusion protein of the present disclosure) had a long-term high-concentration blood drug exposure in mice, but the phenomenon of catabolic effects reported in the literature did not occur. It could be seen that LAPAF (the dual-targeting fusion protein of the present disclosure) solved the catabolic effects caused by long-acting PTH (such as TransCon PTH) or continuous infusion of teriparatide or Abaloparatide.

### Example 7: In vivo efficacy experiment for evaluating multiple administrations of test substance in RANKL-humanized transgenic mouse osteoporosis model caused by ovariectomy

In this example, the *in vivo* efficacy of the dual-targeting fusion proteins of the present disclosure LYN101-1, LYN101-2, LYN101-3, LYN101-8, LYN101-9, LYN101-10, LYN101-11, LYN101-12, LYN101-13, LYN101-14, LYN101-15, LYN101-16, LYN101-17 and LYN101-18 in the RANKL-humanized transgenic mouse osteoporosis model caused by ovariectomy was evaluated by using the following method. By taking LYN101-2 as an example, the following method was applicable to all the long-acting parathyroid hormone receptor agonist fusion proteins of the present disclosure (LAPAFs).

24 RANKL-humanized transgenic mice (purchased from Biocytogen Jiangsu Gene Biotechnology Co., Ltd., B-hRANKL mice, Catalog No.: 111072) were randomly divided into 4 groups, with 6 mice in each group: G1 Sham group (subcutaneous administration of an equal amount of Buffer (a saline solution containing 0.005% Tween 80)), G2 OVX group (model group, subcutaneous administration of an equal amount of Buffer (a saline solution containing 0.005% Tween 80)), G3 denosumab (10 mpk, subcutaneous administration, once a week)+teriparatide (trade name: OstioGen) (0.1 mpk, subcutaneous administration, once a day) group (combination group), G4 LYN101-2 (10 mpk group, subcutaneous administration, once a week) (LYN101-2 group). Sham group underwent sham surgery, and other groups underwent ovariectomy to obtain osteoporosis mouse models (ovariectomy of mice was performed by operation means known in this field). Drug administration began 4 weeks after surgery and continued for 4 weeks. Blood was collected before administration, 3 weeks after administration and 4 weeks after administration to detect the levels of CTX-1, calcium and phosphorus in serum. Mice were sacrificed 4 weeks after administration, and the tibia, femur, and fourth lumbar vertebra were collected for standby use. Micro-CT analysis was performed on the left tibia. The algorithm for the change percentage of bone mineral density was as follows: change percentage of BMD in Sham group=(Sham group BMD-OVX group BMD)/sham group BMD, change percentage of BMD of Denosumab+teriparatide combination group=(BMD of denosumab+teriparatide combined group-OVX group BMD)/OVX group BMD, and change percentage of BMD of LYN101-2 group=(LYN101-2 group BMD-OVX group BMD)/OVX group BMD.

By taking LYN101-2 as a representative of the dual-targeting fusion protein of the present disclosure (LAPAF), and the combination group of denosumab (anti-RANKL monoclonal antibody) and teriparatide (rhPTH (1-34)) as a representative of control sample. this example showed the efficacy results of LAPAF in the RANKL-humanized transgenic mouse osteoporosis model caused by ovariectomy.

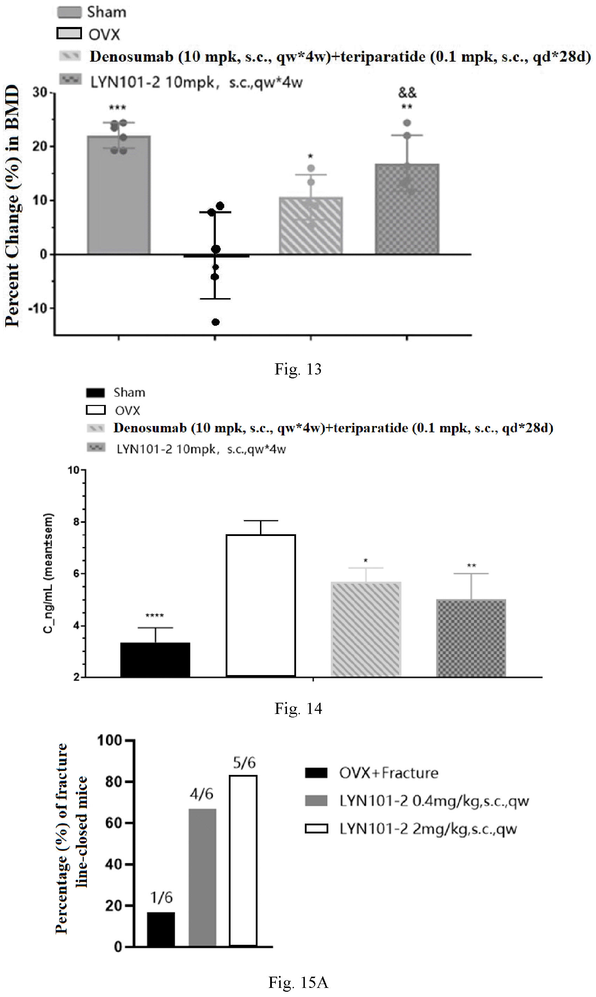

As shown in Fig. 13, after ovariectomy, the bone mineral density of the proximal tibia in OVX group (G2 group) decreased significantly (p<0.001). After 4 weeks of treatment, the bone mineral density in combination group (G3 group) and LYN101-2 group (G4 group) increased, but the change percentage of the improvement of the proximal tibia bone mineral density in the dual-targeting fusion protein of the present disclosure (LAPAF) LYN101-2 group (G4 group) was significantly better than that in the combination group of denosumab (anti-RANKL monoclonal antibody) and teriparatide (G3 group).

As shown in the results of Fig. 14, after ovariectomy, the level of CTX-1 in the serum of the OVX group (G2 group) increased significantly. After 4 weeks of treatment, compared with OVX group (G2 group), the levels of CTX-1 in the serum of the combination group of denosumab (anti-RANKL monoclonal antibody) and teriparatide (G3 group) and the dual-targeting fusion protein of the present disclosure (LAPAF) LYN101-2 group (G4 group) were significantly decreased (G3, p<0.05; G4, p<0.01), and the LYN101-2 group (G4) could reduce the level of CTX-1 in serum to a greater extent compared with the combination group (G3), thereby slowing down the rate of bone loss.

### Example 8: Preliminary experiments for evaluating in vivo efficacy of LAPAF in aged spontaneously osteoporotic rhesus monkey model after single administration

In this example, the *in vivo* efficacy of the dual-targeting fusion proteins of the present disclosure LYN101-1, LYN101-2, LYN101-3, LYN101-8, LYN101-9, LYN101-10, LYN101-11, LYN101-12, LYN101-13, LYN101-14, LYN101-15, LYN101-16, LYN101-17 and LYN101-18 in an aged spontaneously osteoporotic rhesus monkey model was evaluated by using the following method. By taking LYN101-2 as an example, the following method was applicable to all the long-acting parathyroid hormone receptor agonist fusion proteins of the present disclosure (LAPAFs).

2 aged spontaneously female osteoporotic rhesus monkeys were divided into 2 groups: LYN101-2 0.1 mpk and LYN101-2 0.5 mpk. The drug was administered starting from D0, and the levels of calcium and phosphorus in serum were detected on D1, D3, D7, D10, D14, D18, D21, D28, D35, D42, D49 and D56. The levels of bone formation markers PINP and TRAP5b were detected before the first administration and on D56. Bone mineral density scanning was performed on the lumbar vertebrae L1-L4 of the rhesus monkeys using GE Prodigy dual-energy X-ray absorptiometry before the first administration and on D56.

By taking LYN101-2 as a representative of LAPAF, this example demonstrated the results of the preliminary experiment of *in vivo* efficacy study in the aged spontaneously osteoporotic rhesus monkey model.

The results showed that, on D56 after a single administration of LYN101-2 0.1 mpk, the bone formation marker PINP of spontaneously aged osteoporotic rhesus monkeys decreased by 8.5% compared with the baseline before administration, and the bone resorption marker TRAP5b increased by 8.8% compared with the baseline before administration, indicating that the activity of osteoblasts on D56 was slightly decreased and the osteoclast activity was slightly increased compared with those before administration. This result suggested that the bone mineral density of the model animals on D56 should theoretically decrease, but the inventors unexpectedly observed the opposite result. Compared with the baseline before administration, the bone mineral density of L1-L4 (lumbar vertebrae) of aged spontaneously osteoporotic rhesus monkeys on D56 was increased by 33.1%. The effect of increasing the bone mineral density was significantly better than that of other osteoporosis treatment drugs on the market. For example, after continuous administration of teriparatide (once a day) for 6 months, the bone mineral density of L2-L4 was only increased by about 5% (accessdata.fda.gov/drugsatfda_docs/nda/2002/21-318_FORTEO_Pharmr_P1.pdf), after continuous administration (once a month) of the anti-sclerostin monoclonal antibody romosozumab for 4 months, the bone mineral density of L1-L4 only was increased by about 8% (accessdata.fda.gov/drugsatfda_docs/nda/2019/761062Orig1s000MultidisciplineR.pdf), and the anti-RANKL monoclonal antibody denosumab only increased the bone mineral density of L1-L4 by about 2% 3 months after a single administration. And there was no literature report that the above drugs could quickly increase bone mineral density in a short period of time (such as within 2 months). It could be seen that the dual-targeting fusion protein of the present disclosure (LAPAF) LYN101-2 had rapid and long-lasting effects. On D56 after a low-dose (0.1mpk) single administration, the bone mineral density of the aged spontaneously osteoporotic rhesus monkey was significantly increased, and the bone mineral density increase ratio in a short period of time (such as 2 months) was as high as 33.1%, which was significantly better than the bone mineral density increasing effect (2%-8%) of the existing osteoporosis treatment drugs for single or continuous administration for 3-6 months.

In addition, after single administration, the levels of calcium and phosphorus in the serum of the spontaneously aged female osteoporotic rhesus monkeys were observed. The results showed that regardless of whether 0.1 mpk or 0.5 mpk of LYN1101-2 was administered, the level of calcium in the serum at each observation time point was always within a normal range (9.2-11.6 mg/dL) (the normal range was referenced from: Cui Shufang, Experimental Animal Science, Shanghai, Second Military Medical University Press, 2013), and no hypercalcemia caused by other long-acting PTH drugs (such as TransCon PTH (generic name: palopegteriparatide, trade name: Yorvipath)) was observed.

### Example 9: In vivo efficacy experiments for evaluating multiple administrations of test substance in RANKL-humanized transgenic mouse models with osteoporosis caused by ovariectomy and surgically prepared femoral fractures

In this example, the *in vivo* efficacy of the dual-targeting fusion proteins of the present disclosure LYN101-1, LYN101-2, LYN101-3, LYN101-8, LYN101-9, LYN101-10, LYN101-11, LYN101-12, LYN101-13, LYN101-14, LYN101-15, LYN101-16, LYN101-17 and LYN101-18 on a RANKL-humanized transgenic mouse models with osteoporosis **caused by** ovariectomy and surgically prepared femoral fractures after multiple administrations was evaluated by using the following method. By taking LYN101-2 as an example, the following method was applicable to all the long-acting parathyroid hormone receptor agonist fusion proteins of the present disclosure (LAPAFs).

39 RANKL-humanized transgenic mice (purchased from Biocytogen Jiangsu Gene Biotechnology Co., Ltd., B-hRANKL mice, Catalog No.: 111072) were divided into 7 groups: Sham group, Sham+fracture group (non-OVX fracture model group), OVX (ovariectomy)+fracture group (OVX fracture model group), teriparatide group (4 µg/kg), teriparatide (4 µg/kg)+denosumab (10 mpk) (combination group), LYN101-2 0.4mpk group, and LYN101-2 2mpk group, with 3 mice for Sham group and 6 mice for other groups. OVX surgery (mouse ovariectomy) was performed on day -31 to prepare an osteoporosis model, and a left femoral fracture model (open fracture) was prepared on day -3. LYN101-2 0.4mpk group and LYN101-2 2mpk group were subjected to subcutaneous injection 3 days later (Day 0) and continued to be administered for 6 weeks. Except for teriparatide which was administered every Monday to Friday, other drugs were administered once a week. The Sham group and model groups were injected with an equal amount of Buffer (a normal saline solution containing 0.005% Tween 80) every week. The levels of calcium and phosphorus in serum were detected on day -31 (before OVX), day -3 (before fracture), day 7, day 28, and day 42; levels of ALP, CTX-1, osteocalcin, PINP, and TRAP5b in serum were detected, and the left femur was collected for fracture union analysis; the right tibia was collected for bone mineral density analysis; the right tibia and L4 lumbar vertebra were collected for standby use; the right femur was collected for standby use, and Micro-CT analysis was performed on the right proximal tibia and left femur fracture site. Calcein was injected intraperitoneally on D10 and D3 before the fracture samples of mice were collected, and alizarin red was injected intraperitoneally on D7 before the fracture samples of mice were collected, and the areas with vigorous bone metabolism in mice were fluorescently labeled. At the end of the animal experiment, the mice were humanely sacrificed and their femoral fracture specimens were obtained. The mineralization process of the femoral fracture specimens of mice was visually studied by using a fluorescent double labeling method. Ultrathin sections were obtained by using an undecalcified bone tissue sectioning technique, and then photographed under a laser confocal microscope to observe the spacing of fluorescent labeled substances, so as to calculate a bone mineral apposition rate (MAR, in µm/day) and a bone tissue formation rate (BFR, in µm³/µm²/day). The specific calculation method was as follows: the bone mineral apposition rate MAR refers to the thickness of mineralized bone daily newly deposited on the active bone formation surface. It was calculated as average width of double labeling band/interval days between double labeling. The bone tissue formation rate BFR=(MS/TP)*(1/MAR), wherein MS represented a bone tissue formation area and TP represented a bone trabecular thickness. The results were expressed as mean ± standard deviation.

By taking LYN101-2 as a representative of LAPAF, this example demonstrated the efficacy of multiple administrations in RANKL-humanized transgenic mouse models with osteoporosis caused by ovariectomy and surgically prepared femoral fractures

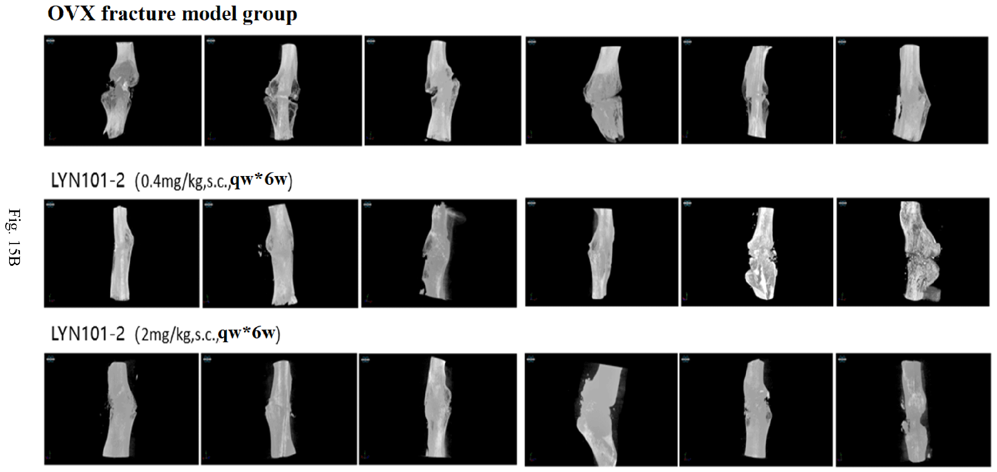

As shown in Fig. 15A and Fig. 15B: after the RANKL-humanized transgenic mice were subjected to ovariectomy and surgically preparation of femoral fracture, a very obvious fracture line appeared at the fracture site. After 6 weeks of treatment with administration once a week, only one mouse in OVX fracture model group showed fracture line closure, and 5 mice still had obvious fracture lines; correspondingly, in the dual-targeting fusion protein of the present disclosure (LAPAF) LYN101-2 0.4mpk group, 4 mice were observed to have fracture line closure, 1 mouse had a partial fracture line closure, and only 1 mouse had a relatively obvious fracture line; in the dual-targeting fusion protein of the present disclosure (LAPAF) LYN101-2 2mpk group, 5 mice had fracture lines closure, and only 1 mouse had a relatively obvious fracture line.

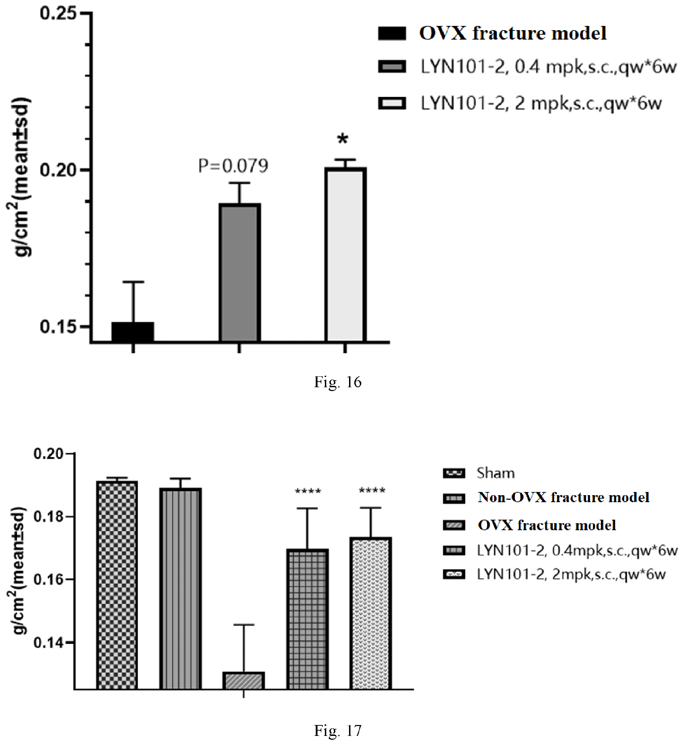

As shown in Fig. 16, after the RANKL-humanized transgenic mice were subjected to ovariectomy and femoral fracture, the bone mineral density of the femur (fracture site) decreased significantly. After 6 weeks of treatment (once a week), compared with OVX fracture model group, the bone mineral density at the fracture site of the mice in the dual-targeting fusion protein of the present disclosure (LAPAF) LYN101-2 0.4mpk group increased, and the bone mineral density at fracture site of the mice in the LYN101-2 2mpk group increased significantly.

As shown in Fig. 17, after the RANKL-humanized transgenic mice were subjected to ovariectomy and femoral fracture, the bone mineral density of the tibia (non-fracture site) decreased significantly. After 6 weeks of treatment (once a week), the bone mineral density of the proximal tibia of the mice in the dual-targeting fusion protein of the present disclosure (LAPAF) LYN101-2 0.4 mpk and 2 mpk groups increased significantly, and the bone mineral density of some mice was even close to the normal level.

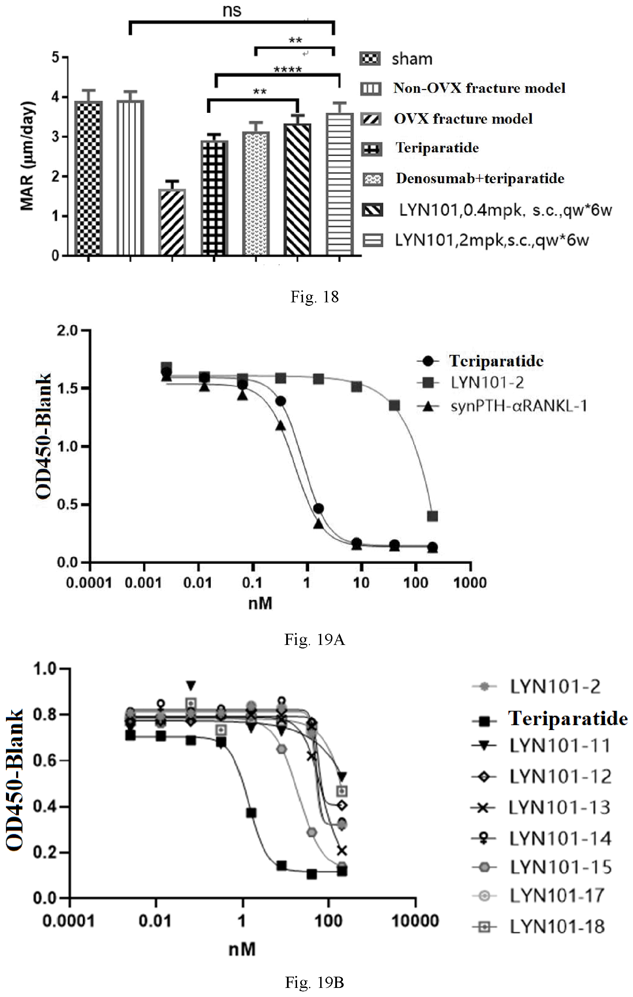

As shown in Fig. 18, after the RANKL-humanized transgenic mice were subjected to ovariectomy and femoral fracture, MAR was significantly reduced, indicating that the model was successfully constructed. After 6 weeks of administration, the MAR of the dual-targeting fusion protein of the present disclosure (LAPAF) LYN101-2 0.4 mpk group and 2 mpk group of mice increased significantly, and showed a dose-dependent increase trend. Compared with the teriparatide group, the MAR of the dual-targeting fusion protein of the present disclosure (LAPAF) LYN101-2 0.4 mpk group increased significantly, and compared with the teriparatide group and the combination group of teriparatide and denosumab, the MAR of the LYN101-2 2 mpk group also increased significantly, indicating that the fracture union treatment effect of 0.4 mpk LYN101-2 on osteoporotic fracture mice was significantly better than that of teriparatide, and the fracture union treatment effect of 2 mpk LYN101-2 on osteoporotic fracture mice is significantly better than that of teriparatide and the combination group of teriparatide and denosumab. At the same time, there was no statistical difference in the MAR of mice between the dual-targeting fusion protein of the present disclosure (LAPAF) LYN101-2 2 mpk group and the Sham group or non-OVX fracture model group, indicating that the dual-targeting fusion protein of the present disclosure (LAPAF) LYN101-2 can restore the MAR of osteoporotic fracture mice to a near normal level. The change trend of BFR was consistent with MAR.

### Example 10: Evaluation of efficacy of LAPAF on mouse osteoarthritis model induced by medial meniscus transection (MMT)

In this example, the efficacy of the dual-targeting fusion proteins of the present disclosure LYN101-1, LYN101-2, LYN101-3, LYN101-8, LYN101-9, LYN101-10, LYN101-11, LYN101-12, LYN101-13, LYN101-14, LYN101-15, LYN101-16, LYN101-17 and LYN101-18 on the mouse osteoarthritis model induced by medial meniscus transection (MMT) was evaluated by using the following method. By taking LYN101-2 as an example, the following method was applicable to all the long-acting parathyroid hormone receptor agonist fusion proteins of the present disclosure (LAPAFs).

This example mainly studied 15 11-week-old mice using Bio-Book, and randomly divided them into 3 groups according to the weight of the animals: Sham group, MMT model group, MMT model group+LYN101-2 0.4mpk group (LYN101-2 0.4mpk group). The surgical modeling process was as follows: the medial side of the femoral-tibial joint would be incised, the medial meniscus was exposed by blunt separation, and a full-thickness cut was made at the narrowest part of the meniscus. After completion, the joint was reset, the knee joint muscles, ligaments and skin were sutured, and the surgical site was disinfected. Subcutaneous injection began two weeks after surgery. In this experiment, the mice were administrated once a week, subcutaneously for the first four weeks, and intra-articularly for the last four weeks, for a total of 8 times. The Sham group and the MMT model group were injected with an equal amount of Buffer (a physiological saline solution containing 0.005% Tween 80) subcutaneously or intra-articularly. During the administration, the body weight, clinical signs, foot balance test and other indicators were mainly observed; after the end of the experiment, the right knee joints of all animals were fixed in 10% NBF for 48 h and then transferred to 70% ethanol. The samples were decalcified with formic acid, embedded in paraffin, sectioned on the coronal plane, and stained with H&E and Safranin-O. The sections were scored by professional pathologists for degenerative changes (main tissue properties, surface regularity, structural integrity, chondrocyte clusters, cartilage degenerative changes, inflammatory response in the subchondral bone area, neovascularization, and bone spurs). The scoring criteria were based on the Osteoarthritis Research Society International (OARSI) scoring system put forth in 2010 (20).

By taking LYN101-2 as a representative of LAPAF, this example demonstrated the efficacy results of LAPAF on the mouse osteoarthritis model induced by medial meniscus transection (MMT).

The experimental results showed that: the foot balance test results showed that significant efficacy was observed in the dual-targeting fusion protein of the present disclosure (LAPAF) LYN101-2 group. For example, the load-bearing capacity of the modeling side foot of the mice in the LYN101-2 0.4mpk group was significantly improved compared with the MMT model group (p<0.01), indicating that the dual-targeting fusion protein of the present disclosure (LAPAF) LYN101-2 could significantly relieve the joint pain caused by MMT.

### Example 11: Evaluation of repeated administration toxicity test for six weeks of recovery after five consecutive subcutaneous injections of LAPAF in rhesus monkeys every three weeks

In this example, the repeated administration toxicology study of the dual-targeting fusion proteins of the present disclosure LYN101-1, LYN101-2, LYN101-3, LYN101-8, LYN101-9, LYN101-10, LYN101-11, LYN101-12, LYN101-13, LYN101-14, LYN101-15, LYN101-16, LYN101-17 and LYN101-18 in rhesus monkeys was evaluated by using the following method to study the toxic responses and toxicokinetic characteristics of the dual-targeting fusion proteins of the present disclosure administrated to rhesus monkeys by different subcutaneous injection regimens, as well as to observe the recovery from toxicity (if any) or possible delayed toxic responses during the 6-week recovery. By taking LYN101-2 as an example, the following method was applicable to all the long-acting parathyroid hormone receptor agonist fusion proteins of the present disclosure (LAPAFs).

30 rhesus monkeys, half male and half female, were randomly divided into 3 groups according to the body weight and gender: control group (solvent group), LYN101-2 0.1 mpk group and LYN101-2 0.4 mpk group. In this test, the drug was administered once every 3 weeks for a total of 5 times. The low dose of 0.1 mpk was more than 2 times the effective dose, and the high dose of 0.4 mpk was about 10 times the effective dose. And a solvent control group was also set up. On D1, D22, D43, D64 and D85, each animal was administered once by subcutaneous injection. During the administration, the following indicators were mainly tested: cage observation, detailed clinical observation, irritation evaluation of the administration site, body weight, food consumption, ophthalmic examination, clinical pathological examination (including hematology, blood coagulation, serum biochemistry, immunoglobulin, complement, and urinalysis), and lymphocyte phenotype. At the same time, safety pharmacology (electrocardiogram, blood pressure, body temperature, respiratory system, central nervous system) research, biomarker detection, immunogenicity and toxicokinetic research were also carried out.

By taking LYN101-2 as a representative of LAPAF, this example demonstrated the toxic response and toxicokinetic results of five consecutive subcutaneous injections of rhesus monkeys for once every three weeks.

The specific results were as follows: during the test, in the 0.1mpk and 0.4mpk administration groups of the dual-targeting fusion protein of the present disclosure (LAPAF) LYN101-2, female and male monkeys were generally in good condition, had normal autonomous activities and clean fur, and all animals survived until the planned dissection time. No erythema or edema was found at the injection site. At each time point during the test period, no obvious abnormal changes were found in body weight, food intake, and ophthalmic examination of female and male monkeys in the LYN101-2 0.1mpk and 0.4mpk administration groups. Compared with the control group during the same period and before the first administration, in the LYN101-2 0.1mpk and 0.4mpk administration groups, there were no obvious test sample-related changes at each time point after administration in the following indicators: hematology (WBC, and count and percentage of each type of cell, RBC, HGB, HCT, MCV, MCH, MCHC, PLT, %RET and RET), blood biochemistry (ALP, TBIL, DBIL, AST, ALT, GGT, CK, UREA, CREA, TP, ALB, GLO, A/G, GLU, TCHO, TG, Ca, P, K⁺, Cl⁻ and Na⁺), blood coagulation function (APTT, PT, Fbg, and TT), complements (C3 and C4), immunoglobulins (IgM, IgG, and IgA), urine tests (pH, proteins, glucose, bilirubin, ketones, occult blood, leukocytes, etc.), and lymphocyte phenotype analysis. The evaluation results of the safety pharmacology on the cardiovascular system, respiratory system and central nervous system showed no abnormal changes related to the test samples. The changes in biomarkers such as PINP and TRAP-5b were consistent with expectations. The toxicokinetic results showed that there was no difference in drug exposure between male and female animals; the increase in exposure was linearly related to the increase in dose; and there was no significant accumulation in the last dose compared with the first dose.

Under the conditions of this test, rhesus monkeys were administered with LYN101-2 0.1mpk and LYN101-2 0.4mpk of the dual-targeting fusion protein of the present disclosure (LAPAF) once every three weeks for 5 consecutive subcutaneous injections, and no toxicity related to the dual-targeting fusion protein of the present disclosure (LAPAF) LYN101-2 was observed.

### Example 12: In vitro activity of LAPAF on PTHR-induced cAMP release in PTHR⁺/RANKL⁻cells

In this example, the *in vitro* activity of the dual-targeting fusion proteins of the present disclosure LYN101-1, LYN101-2, LYN101-3, LYN101-8, LYN101-9, LYN101-10, LYN101-11, LYN101-12, LYN101-13, LYN101-14, LYN101-15, LYN101-16, LYN101-17 and LYN101-18 on PTHR-induced cAMP release in PTHR⁺/RANKL⁻ cells was determined by using the following method. By taking LYN101-2 as an example, the following method was applicable to all the long-acting parathyroid hormone receptor agonist fusion proteins of the present disclosure (LAPAFs), and control samples synPTH-α RANKL-1, teriparatide, and agonistic antibodies targeting PTHR.

One day in advance, UMR106 cells were washed once with 6 mL of DPBS, and then 3 mL of preheated trypsin was added therein. The cells were digested in an incubator at 37°C and 5% CO₂ for 5 min. After the cells were completely digested, 10 mL of DMEM complete medium was added into the digested cells for gentle purging. The above-mentioned two cells were separately prepared into cell suspensions, and the cell density was adjusted to 3×10⁵ cells/mL. The cell suspension was added into a 96-well cell culture plate at 100 µL/well, and then cultured overnight in a constant temperature incubator (NUNRE/NU-8700E) at 37°C and 5% CO₂. The next morning, the supernatant in the 96-well culture plate was discarded, then the DMEM medium was added into the remaining cells and then incubated in a constant temperature incubator at 37°C and 5% CO₂ for 1 h. After that, the supernatant was discarded. The samples to be tested (LYN101-2, teriparatide, and control sample synPTH-α RANKL-1) were diluted to a starting concentration (the starting concentration of the sample was 200 nM), and then were diluted downward 5 times, with a total of 8 concentration gradients. The above samples were added into each well of the 96-well cell culture plate with UMR106 at 100 µL/well, and then cultured in a constant temperature incubator at 37°C and 5%CO₂ for 40 min. Then 1×lysis buffer was added at 130 µL/well and the cells were incubated at room temperature for 30 min to obtain the sample to be tested (cell lysate supernatant). The subsequent operation was performed according to cAMP Parameter Assay Kit (purchased from R&D, Catalog No.: SKGE002b). Briefly described as follows, the primary antibody in the kit was added into the 96-well plate pre-coated with Goat Anti-mouse at 50 µL/well to incubate the cells at room temperature for 1 h at 500 rpm/min, and the plate was washed. Then a 100 µL/well cell lysate supernatant and 50 µL/well cAMP Conjugate were added to incubate the cells at room temperature for 2 h at 500 rpm/min, and the plate was washed. After the substrate was added to react at room temperature for 30 min, the stop solution was added, and the absorbance at 450/570 nm was read and recorded using a microplate reader. The data was analyzed using Graphpad Prism software to obtain an EC50 value.

The results showed that the control samples synPTH-α RANKL-1 and teriparatide both had *in vitro* activity on PTHR-induced cAMP release in UMR106 cells (PTHR single positive cells, *i.e.,* cells expressing PTHR but not RANKL, PTHR⁺/RANKL⁻ cells), and the activities were comparable. However, the *in vitro* activity of the dual-targeting fusion proteins of the present disclosure (LAPAF) LYN101-1, LYN101-2, LYN101-3, LYN101-8, LYN101-9, LYN101-10, LYN101-11, LYN101-12, LYN101-13, LYN101-14, LYN101-15, LYN101-16, LYN101-17 and LYN101-18 on PTHR-induced cAMP release in UMR106 cells (PTHR⁺/RANKL⁻ cells) was significantly weaker than that of teriparatide. The experimental results showed that for PTHR single positive cells expressing PTHR but not RANKL (PTHR⁺/RANKL⁻ cells), at the same molar concentration, the dual-targeting fusion protein of the present disclosure (LAPAF) LYN101-1, LYN101-2, LYN101-3, LYN101-8, LYN101-9, LYN101-10, LYN101-11, LYN101-12, LYN101-13, LYN101-14, LYN101-15, LYN101-16, LYN101-17 and LYN101-18 had significantly weaker *in vitro* activity on PTHR-induced cAMP release in PTHR⁺/RANKL⁻ cells than teriparatide or the control sample synPTH-α RANKL-1. It was indicated that the *in vitro* activity of the dual-targeting fusion protein of the present disclosure (LAPAF) on PTHR single positive cells (PTHR⁺/RANKL⁻) simulating kidney cells was significantly weaker than that of teriparatide and the control sample synPTH-α RANKL-1, reducing the toxic side effects caused by binding to cells of non-target tissues only expressing PTHR, such as kidney cells.

As shown in Fig. 19A, on UMR106 cells (PTHR⁺/RANKL⁻ cells), the *in vitro* activity of the dual-targeting fusion protein of the present disclosure (LAPAF) LYN101-2 on PTHR-induced cAMP release was significantly weaker than that of teriparatide (EC50_{LYN101-2}=about 515279nM, EC50_{teriparatide}=0.8267nM, EC50_{LYN101-2}>>EC50_{teriparatide}), while the activity of the control sample synPTH-α RANKL-1 was comparable to that of teriparatide (EC50_{synPTH-α RANKL-1}=0.5863nM, EC50_{teriparatide}=0.8267nM, EC50_{synPTH-α RANKL-1}≈EC50_{teriparatide}). It could be seen that the activity of the dual-targeting fusion protein of the present disclosure (LAPAF) LYN101-2 on PTHR⁺/RANKL⁻ cells was significantly weaker than that of teriparatide and the control sample synPTH-α RANKL-1 at the same molar concentration. At a concentration of 100 nM or less, the dual-targeting fusion protein of the present disclosure (LAPAF) LYN101-2 hardly activated the *in vitro* activity of PTHR-induced cAMP release in UMR106 cells (PTHR⁺/RANKL⁻ cells), *i.e.,* at a concentration of 100 nM or less, the dual-targeting fusion protein of the present disclosure (LAPAF) LYN101-2 hardly bound to PTHR⁺/RANKL⁻ cells (such as kidney cells).

Meanwhile, in order to evaluate whether different selections of the anti-RANKL functional region, linker, PTH functional region and Fc region in the dual-targeting fusion protein of the present disclosure (LAPAF) had an effect on the *in vitro* activity experimental results of PTHR-induced cAMP release in UMR106 cells (PTHR⁺/RANKL⁻ cells), the inventors also simultaneously detected LYN101-8, which had a different anti-RANKL functional region from LYN101-2, LYN101-1 and LYN101-3, which had different linkers from LYN101-2, and LYN101-9, LYN101-10, LYN101-11, LYN101-12, LYN101-13, LYN101-14, LYN101-15, and LYN101-16, which had different PTH functional regions from LYN101-2, as well as LYN101-17 and LYN101-18 that had different Fc regions from LYN101-2. The results showed that the *in vitro* activity experimental results of LYN101-8, LYN101-1, LYN101-3, LYN101-9, LYN101-10, LYN101-11, LYN101-12, LYN101-13, LYN101-14, LYN101-15, LYN101-16, LYN101-17 and LYN101-18 on PTHR-induced cAMP release in UMR106 cells (PTHR⁺/RANKL⁻ cells) were similar to those of LYN101-2, indicating that changes in the anti-RANKL functional region, or linker, or PTH functional region, or Fc region of the dual-targeting fusion protein of the present disclosure (LAPAF) basically had a little effect on the *in vitro* activity on PTHR-induced cAMP release in PTHR⁺/RANKL⁻ cells.

The specific experimental results were as follows:
The *in vitro* activity of the dual-targeting fusion protein of the present disclosure (LAPAF) LYN101-8 (with an anti-RANKL functional region different from that of the LYN101-2) on PTHR-induced cAMP release in UMR106 (PTHR⁺/RANKL⁻ cells) was similar to that of LYN101-2, *i.e.,* the *in vitro* activity of LYN101-8 on PTHR-induced cAMP release in UMR106 cells was significantly weaker than that of teriparatide and the control sample synPTH-α RANKL-1.

The *in vitro* activity of the dual-targeting fusion proteins of the present disclosure (LAPAFs) LYN101-1 and LYN101-3 (with a Linker selected different from that of LYN101-2) on PTHR-induced cAMP release in UMR106 cells (PTHR⁺/RANKL⁻ cells) was similar to that of LYN101-2, that was, the *in vitro* activity of LYN101-1 and LYN101-3 on PTHR-induced cAMP release in UMR106 cells (PTHR⁺/RANKL⁻ cells) was weaker than that of teriparatide (more than 40 times) and the control sample synPTH-α RANKL-1.

In addition, in order to evaluate whether the length or type of the PTH functional region in the dual-targeting fusion protein of the present disclosure, and Fc region in the dual-targeting fusion protein of the present disclosure (LAPAF) had an effect on the *in vitro* activity of PTHR-induced cAMP release in PTHR⁺/RANKL⁻ cells, the inventors also simultaneously detected the *in vitro* activity of the dual-targeting fusion proteins of the present disclosure (LAPAFs) (LYN101-2, LYN101-11, LYN101-12, LYN101-13, LYN101-14, LYN101-15, LYN101-17 and LYN101-18) containing different lengths or types of (PTH or PTHrP) PTH functional region or containing different Fc regions on PTHR-induced cAMP release in UMR106 cells (PTHR⁺/RANKL⁻ cells).

As shown in Fig. 19B, in the *in vitro* activity of PTHR-induced cAMP release in UMR106 cells (PTHR⁺/RANKL⁻ cells), the *in vitro* activity of the dual-targeting fusion proteins of the present disclosure (LAPAFs) LYN101-2, LYN101-11, LYN101-12, LYN101-13, LYN101-14, LYN101-15, LYN101-17 and LYN101-18 on PTHR-induced cAMP release in UMR106 cells was significantly weaker than that of teriparatide, and the results were similar to those of LYN101-2. For example, EC50_{teriparatide}=1.4 nM, EC50_{LYN101-12}=162.3 nM, and EC50_{LYN101-14}=115 nM. It could be seen that changing the length or type of the PTH functional region and changing the Fc region also did not affect the *in vitro* activity of the targeting fusion protein of the present disclosure (LAPAF) in PTHR-induced cAMP release in UMR106 cells (PTHR⁺/RANKL⁻ cells).

The use of any and all embodiments or exemplary language (*e.g.,* "such as") provided herein is intended only to better illustrate the present disclosure and the present disclosure is not intended to limit the scope of the disclosure unless otherwise required. No language in the specification should be interpreted as indicating that any non-claimed element is necessary to implement the present disclosure.

All publications and patent applications cited in this specification are incorporated herein by reference as if each individual publication or patent application was specifically and individually indicated to be incorporated by reference. In addition, any theory, mechanism, proof or discovery described herein is intended to further enhance the understanding of the present disclosure and is not intended to limit the present disclosure to such theory, mechanism, proof or discovery in any way. Although the present disclosure has been shown and described in detail in the drawings and the foregoing description, the present disclosure should be considered illustrative and not restrictive.
Some sequences used in the present disclosure:

| SEQ ID NO | Name of Sequence | Sequence |
|---|---|---|
| 1 | Heavy chain variable region of anti-RANKL monoclonal antibody 1 | EVQLLESGGGLVQPGGSLRLSCAASGFTFSSYAMSWVRQAPGKG LEWVSGITGSGGSTYYADSVKGRFTISRDNSKNTLYLQMNSLRAE DTAVYYCAKDPGTTVIMSWFDPWGQGTLVTVSS |
| 2 | Light chain variable region of anti-RANKL monoclonal antibody 1 | EIVLTQSPGTLSLSPGERATLSCRASQSVRGRYLAWYQQKPGQAP RLLIYGASSRATGIPDRFSGSGSGTDFTLTISRLEPEDFAVFYCQQY GSSPRTFGQGTKVEIK |
| 3 | Heavy chain variable region of anti-RANKL monoclonal antibody 2 | QVQLVQSGAEVKKPGSSVKVSCKASGYAFTNYYIEWVRQAPGQ GLEWMGVINPGWGDTNYNEKFKGRVTITADKSTSTAYMELSSLR SEDTAVYYCARRDTAHGYYALDPWGQGTTVTVSS |
| 4 | Light chain variable region of anti-RANKL monoclonal antibody 2 | DIQMTQSPSSLSASVGDRVTITCKASQNVGTNVAWYQQKPGKAP KLLIYSASYRYSGVPSRFSGSGSGTDFTLTISSLQPEDFATYYCQQY WDYPLTFGGGTKVEIK |
| 5 | PTH(1-34) | SVSEIQLMHNLGKHLNSMERVEWLRKKLQDVHNF |
| 6 | Fc | ESKYGPPCPPCPAPEFLGGPSVFLFPPKPKDTLMISRTPEVTCVVV DVSQEDPEVQFNWYVDGVEVHNAKTKPREEQFNSTYRVVSVLT VLHQDWLNGKEYKCKVSNKGLPSSIEKTISKAKGQPREPQVYTL PPSQEEMTKNQVSLTCLVKGFYPSDIAVEWESNGQPENNYKTTPP VLDSDGSFFLYSRLTVDKSRWQEGNVFSCSVMHEALHNHYTQKS LSLSLGK |
| 7 | Fc1-IgG4 | ESKYGPPCPPCPAPEFLGGPSVFLFPPKPKDTLMISRTPEVTCVVV DVSQEDPEVQFNWYVDGVEVHNAKTKPREEQFNSTYRVVSVLT VLHQDWLNGKEYKCKVSNKGLPSSIEKTISKAKGQPREPQVYTL PPSQEEMTKNQVSLsCaVKGFYPSDIAVEWESNGQPENNYKTTPP |
| | | VLDSDGSFFLvSRLTVDKSRWQEGNVFSCSVMHEALHNHYTQKS LSLSLGK |
| 8 | Fc2-IgG4 | ESKYGPPCPPCPAPEFLGGPSVFLFPPKPKDTLMISRTPEVTCVVV DVSQEDPEVQFNWYVDGVEVHNAKTKPREEQFNSTYRVVSVLT VLHQDWLNGKEYKCKVSNKGLPSSIEKTISKAKGQPREPQVYTL PPSQEEMTKNQVSLwCLVKGFYPSDIAVEWESNGQPENNYKTTPP VLDSDGSFFLYSRLTVDKSRWQEGNVFSCSVMHEALHNHYTQKS LSLSLGK |
| 9 | Linker1 | GGGGS |
| 10 | Linker2 | GGGGSGGGGS |
| 11 | Linker3 | GGGGSGGGGSGGGGS |
| 12 | Linker4 | GGGGSGGGGSGGGGSGGGGS |
| 13 | Signal peptide | MRAWIFFLLCLAGRALA |
| 14 | CH1 | ASTKGPSVFPLAPCSRSTSESTAALGCLVKDYFPEPVTVSWNSGA LTSGVHTFPAVLQSSGLYSLSSVVTVPSSSLGTKTYTCNVDHKPSN TKVDKRV |
| 15 | CL | |
| 16 | HCDR1 of anti-RANKL monoclonal antibody 1 | SYAMS |
| 17 | HCDR2 of anti-RANKL monoclonal antibody 1 | GITGSGGSTYYADSVKG |
| 18 | HCDR3 of anti-RANKL monoclonal antibody 1 | DPGTTVIMSWFDP |
| 19 | LCDR1 of anti-RANKL monoclonal antibody 1 | RASQSVRGRYLA |
| 20 | LCDR2 of anti-RANKL monoclonal antibody 1 | GASSRAT |
| 21 | LCDR3 of anti-RANKL monoclonal antibody 1 | QQYGSSPRT |
| 22 | HCDR1 of anti-RANKL monoclonal antibody 2 | NYYIE |
| 23 | HCDR2 of anti-RANKL monoclonal antibody 2 | VINPGWGDTNYNEKFKG |
| 24 | HCDR3 of anti-RANKL monoclonal antibody 2 | RDTAHGYYALDP |
| 25 | LCDR1 of anti-RANKL monoclonal antibody 2 | KASQNVGTNVA |
| 26 | LCDR2 of anti-RANKL monoclonal antibody 2 | SASYRYS |
| 27 | LCDR3 of anti-RANKL monoclonal antibody 2 | QQYWDYPLT |
| 28 | Linker 5 | GGGS |
| 29 | Linker 6 | EAAAK |
| 30 | Abaloparatide | AVSEHQLLHDKGKSIQDLRRRELLEKLLXKLHTA |
| 31 | PTH(1-11) mutant | SVAEIALMHQR |
| 32 | PTH(1-20) | SVSEIQLMHNLGKHLNSMER |
| 33 | PTH(1-37) | SVSEIQLMHNLGKHLNSMERVEWLRKKLQDVHNFVAL |
| 34 | PTH(1-38) | SVSEIQLMHNLGKHLNSMERVEWLRKKLQDVHNFVALG |
| 35 | PTH(1-41) | SVSEIQLMHNLGKHLNSMERVEWLRKKLQDVHNFVALGAPL |
| 36 | PTHrP(1-36) | AVSEHQLLHDKGKSIQDLRRRFFLHHLIAEIHTAEI |
| 37 | PTH(1-84) | SVSEIQLMHNLGKHLNSMERVEWLRKKLQDVHNFVALGAPLAP RDAGSQRPRKKEDNVLVESHEKSLGEADKADVNVLTKAKSQ |
| 38 | PTH(1-14) mutant | AVAEIQLMHQRAKW |
| 39 | Fc1-IgG1 | EPKSCDKTHTCPPCPAPEAAGGPSVFLFPPKPKDTLMISRTPEVTC VVVDVSHEDPEVKFNWYVDGVEVHNAKTKPREEQYNSTYRVVS VLTVLHQDWLNGKEYKCKVSNKALPAPIEKTISKAKGQPREPQV YTLPPSREEMTKNQVSLSCAVKGFYPSDIAVEWESNGQPENNYK |
| | | TTPPVLDSDGSFFLVSKLTVDKSRWQQGNVFSCSVMHEALHNHY TQKSLSLSPGK |
| 40 | Fc2-IgG1 | EPKSCDKTHTCPPCPAPEAAGGPSVFLFPPKPKDTLMISRTPEVTC VVVDVSHEDPEVKFNWYVDGVEVHNAKTKPREEQYNSTYRVVS VLTVLHQDWLNGKEYKCKVSNKALPAPIEKTISKAKGQPREPQV YTLPPSREEMTKNQVSLWCLVKGFYPSDIAVEWESNGQPENNYK TTPPVLDSDGSFFLYSKLTVDKSRWQQGNVFSCSVMHEALHNHY TQKSLSLSPGK |

Although the specific embodiments of the present disclosure are described above, it should be understood by those skilled in the art that these are only examples, and various changes or modifications may be made to these embodiments without departing from the principles and essence of the present disclosure. Therefore, the protective scope of the present disclosure is limited by the appended claims.

## Claims

1. A long-acting parathyroid hormone receptor agonist fusion protein comprising a first arm and a second arm; wherein the first arm comprises a functional region targeting RANKL and an Fc region, the functional region targeting RANKL is Fab or ScFab, and the functional region targeting RANKL is located at the N-terminus of the Fc region; the second arm comprises a functional region capable of activating PTHR (parathyroid hormone receptor 1) and an Fc region, and the C-terminus of the functional region capable of activating PTHR in the second arm is connected to the N-terminus of the Fc region directly or through a linker.

2. The long-acting parathyroid hormone receptor agonist fusion protein according to claim 1, wherein the Fc region consists of FcA and FcB, wherein both the FcA and the FcB are tandem CH2 and CH3 of human IgG containing a hinge region, respectively; wherein the FcA and the FcB are Knob (s) mutated and Hole (s) mutated, respectively;
preferably, the human IgG is selected from IgG1, IgG2, IgG3 and IgG4; wherein the IgG1 is of a mutant type with a silent Fc immune effector function, and the IgG4 comprises the mutant type with the silent Fc immune effector function and/or a mutant type with S228P mutation that improves molecular stability.

3. The long-acting parathyroid hormone receptor agonist fusion protein according to claim 2, wherein when the FcA comprises the amino acid sequence of SEQ ID NO: 7, the FcB comprises the amino acid sequence of SEQ ID NO: 8; when the FcA comprises the amino acid sequence of SEQ ID NO: 8, the FcB comprises the amino acid sequence of SEQ ID NO: 7; when the FcA comprises the amino acid sequence of SEQ ID NO: 39, the FcB comprises the amino acid sequence of SEQ ID NO: 40; and when the FcA comprises the amino acid sequence of SEQ ID NO: 40, the FcB comprises the amino acid sequence of SEQ ID NO: 39.

4. The long-acting parathyroid hormone receptor agonist fusion protein according to any one of claims 1 to 3, wherein the functional region capable of activating PTHR comprises an agonistic antigen-binding variable region targeting PTHR or a PTH functional region, wherein the agonistic antigen-binding variable region targeting PTHR is ScFv or SDA; the PTH functional region is selected from a mature human parathyroid hormone (PTH), truncated PTH, a PTH mutant, a mutant of truncated PTH, a truncated mature human parathyroid hormone-related protein (PTHrP), a mutant of truncated PTHrP; the truncated PTH is preferably obtained by truncating no less than 11 amino acid residues from the N-terminus of mature human PTH; and the truncated PTH is more preferably obtained by truncating no less than 34 amino acid residues from the N-terminus of the mature human PTH; furthermore preferably, the PTH functional region is PTH (1-34), PTH (1-37), PTH (1-38), PTH (1-41), PTH (1-84), PTHrP (1-34), PTHrP (1-36); furthermore preferably, the PTH functional region comprises an amino acid sequence as shown in any one of SEQ ID NOs: 5, and 33-37 or an amino acid sequence obtained by addition, deletion, modification and/or conservative substitution of at least one amino acid residue such as 1, 2, 3, 4, 5 or 6 amino acid residues in the amino acid sequences as shown in the aforementioned sequences;
and/or,
the sequence of the linker is selected from (GGGGS)n, (GGGS)n, (GGS)n, (G)n, (GS)n, (EAAAK)n or (XP)n, or a combination of the aforementioned sequences, wherein n is a natural number such as 1-20, and preferably the length of the linker is no more than 20 amino acids; preferably, the sequence of the linker comprises an amino acid sequence selected from any one of SEQ ID NOs: 9-12; more preferably, the sequence of the linker comprises the amino acid sequence of SEQ ID NO: 9.

5. The long-acting parathyroid hormone receptor agonist fusion protein according to any one of claims 1 to 4, wherein the first arm comprises a first arm A chain and a first arm B chain;
the structure of the first arm A chain from the N-terminus to the C-terminus is VL-CL;
the structure of the first arm B chain from the N-terminus to the C-terminus is VH-CH1-FcA; optionally, the VL and VH or CL and CH1 are interchanged by a CrossMab technology;
and/or,
the structure of the second arm from the N-terminus to the C-terminus is the functional region capable of activating PTHR-FcB or the functional region capable of activating PTHR-linker-FcB.

6. The long-acting parathyroid hormone receptor agonist fusion protein according to any one of claims 1 to 5, wherein the Fab or ScFab targeting RANKL comprises 1) a VH comprising heavy chain complementary determining regions HCDR1-3, 2) a VL comprising light chain complementary determining regions LCDR1-3, 3) a CL and 4) a CH1, wherein the amino acid sequences of HCDR1-3 are respectively shown in SEQ ID NOs: 16-18 or SEQ ID NOs: 22-24, and the amino acid sequences of LCDR1-3 are respectively shown in SEQ ID NOs: 19-21 or SEQ ID NOs: 25-27; optionally, and the Fab or ScFab exchanges the VH and the VL, or exchanges CH1 and CL through the CrossMab technology.

7. The long-acting parathyroid hormone receptor agonist fusion protein according to claim 5 or 6, wherein the amino acid sequence of the VH comprises the amino acid sequence shown in SEQ ID NO: 1 or 3, and the amino acid sequence of the VL comprises the amino acid sequence as shown in SEQ ID NO: 2 or 4;
and/or,
the amino acid sequence of the CH1 comprises the amino acid sequence of SEQ ID NO: 14, and the amino acid sequence of the CL comprises the amino acid sequence of SEQ ID NO: 15.

8. A long-acting parathyroid hormone receptor agonist fusion protein, comprising a first arm and a second arm, the first arm comprises a first arm A chain and a first arm B chain; wherein:
when the amino acid sequence of the first arm A chain comprises SEQ ID NO: 2 and SEQ ID NO: 15 connected sequentially, the amino acid sequence of the first arm B chain comprises SEQ ID NO: 1, SEQ ID NO: 14 and SEQ ID NO: 6 connected sequentially, or SEQ ID NO: 1, SEQ ID NO: 14 and SEQ ID NO: 7 connected sequentially, or SEQ ID NO: 1, SEQ ID NO: 14, and SEQ ID NO: 8 connected sequentially, or SEQ ID NO: 1, SEQ ID NO: 14 and SEQ ID NO: 39 connected sequentially, or SEQ ID NO: 1, SEQ ID NO: 14 and SEQ ID NO: 40 connected sequentially; or,
when the amino acid sequence of the first arm A chain comprises SEQ ID NO: 1 and SEQ ID NO: 15 connected sequentially, the amino acid sequence of the first arm B chain comprises SEQ ID NO: 2, SEQ ID NO: 14 and SEQ ID NO: 6 connected sequentially, or SEQ ID NO: 2, SEQ ID NO: 14, and SEQ ID NO: 7 connected sequentially, or SEQ ID NO: 2, SEQ ID NO: 14 and SEQ ID NO: 8 connected sequentially, or SEQ ID NO: 2, SEQ ID NO: 14 and SEQ ID NO: 39 connected sequentially, or SEQ ID NO: 2, SEQ ID NO: 14 and SEQ ID NO: 40 connected sequentially; or,
when the amino acid sequence of the first arm A chain comprises SEQ ID NO: 2 and SEQ ID NO: 14 connected sequentially, the amino acid sequence of the first arm B chain comprises SEQ ID NO: 1, SEQ ID NO: 15 and SEQ ID NO: 6 connected sequentially, or SEQ ID NO: 1, SEQ ID NO: 15 and SEQ ID NO: 7 connected sequentially, or SEQ ID NO: 1, SEQ ID NO: 15 and SEQ ID NO: 8 connected sequentially, or SEQ ID NO: 1, SEQ ID NO: 15, and SEQ ID NO: 39 connected sequentially, or SEQ ID NO: 1, SEQ ID NO: 15 and SEQ ID NO: 40 connected sequentially; or,
when the amino acid sequence of the first arm A chain comprises SEQ ID NO: 1 and SEQ ID NO: 14 connected sequentially, the amino acid sequence of the first arm B chain comprises SEQ ID NO: 2, SEQ ID NO: 15 and SEQ ID NO: 6 connected sequentially, or SEQ ID NO: 2, SEQ ID NO: 15 and SEQ ID NO: 7 connected sequentially, or SEQ ID NO: 2, SEQ ID NO: 15, and SEQ ID NO: 8 connected sequentially, or SEQ ID NO: 2, SEQ ID NO: 15 and SEQ ID NO: 39 connected sequentially, or SEQ ID NO: 2, SEQ ID NO: 15 and SEQ ID NO: 40 connected sequentially; or,
when the amino acid sequence of the first arm A chain comprises SEQ ID NO: 4 and SEQ ID NO: 15 connected sequentially, the amino acid sequence of the first arm B chain comprises SEQ ID NO: 3, SEQ ID NO: 14 and SEQ ID NO: 6 connected sequentially, or SEQ ID NO: 3, SEQ ID NO: 14 and SEQ ID NO: 7 connected sequentially, or SEQ ID NO: 3, SEQ ID NO: 14 and SEQ ID NO: 8 connected sequentially, or SEQ ID NO: 3, SEQ ID NO: 14 and SEQ ID NO: 39 connected sequentially, or SEQ ID NO: 3, SEQ ID NO: 14 and SEQ ID NO: 40 connected sequentially; or,
when the amino acid sequence of the first arm A chain comprises SEQ ID NO: 3 and SEQ ID NO: 15 connected sequentially, the amino acid sequence of the first arm B chain comprises SEQ ID NO: 4, SEQ ID NO: 14, and SEQ ID NO: 6 connected sequentially, or SEQ ID NO: 4, SEQ ID NO: 14, and SEQ ID NO: 7 connected sequentially, or SEQ ID NO: 4, SEQ ID NO: 14 and SEQ ID NO: 8 connected sequentially, or SEQ ID NO: 4, SEQ ID NO: 14 and SEQ ID NO: 39 connected sequentially, or SEQ ID NO: 4, SEQ ID NO: 14 and SEQ ID NO: 40 connected sequentially; or,
when the amino acid sequence of the first arm A chain comprises SEQ ID NO: 4 and SEQ ID NO: 14 connected sequentially, the amino acid sequence of the first arm B chain comprises SEQ ID NO: 3, SEQ ID NO: 15 and SEQ ID NO: 6 connected sequentially, or SEQ ID NO: 3, SEQ ID NO: 15 and SEQ ID NO: 7 connected sequentially, or SEQ ID NO: 3, SEQ ID NO: 15 and SEQ ID NO: 8 connected sequentially, or SEQ ID NO: 3, SEQ ID NO: 15 and SEQ ID NO: 39 connected sequentially, or SEQ ID NO: 3, SEQ ID NO: 15 and SEQ ID NO: 40 connected sequentially; or,
when the amino acid sequence of the first arm A chain comprises SEQ ID NO: 3 and SEQ ID NO: 14 connected sequentially, the amino acid sequence of the first arm B chain comprises SEQ ID NO: 4, SEQ ID NO: 15 and SEQ ID NO: 6 connected sequentially, or SEQ ID NO: 4, SEQ ID NO: 15 and SEQ ID NO: 7 connected sequentially, or SEQ ID NO: 4, SEQ ID NO: 15 and SEQ ID NO: 8 connected sequentially, or SEQ ID NO: 4, SEQ ID NO: 15 and SEQ ID NO: 39 connected sequentially, or SEQ ID NO: 4, SEQ ID NO: 15 and SEQ ID NO: 40 connected sequentially;
preferably,
when the amino acid sequence of the first arm B chain comprises SEQ ID NO: 7, the amino acid sequence of the second arm comprises a PTH functional region, a linker and an Fc region connected sequentially, the PTH functional region is selected from SEQ ID NOs: 5 and 33-37, the linker is selected from SEQ ID NOs: 9-12, and the Fc region sequence is SEQ ID NO: 8; or,
when the amino acid sequence of the first arm B chain comprises SEQ ID NO: 8, the amino acid sequence of the second arm comprises a PTH functional region, a linker and an Fc region connected sequentially, the PTH functional region is selected from SEQ ID NOs: 5 and 33-37, the linker is selected from SEQ ID NOs: 9-12, and the Fc region sequence is SEQ ID NO: 7; or,
when the amino acid sequence of the first arm B chain comprises SEQ ID NO: 39, the amino acid sequence of the second arm comprises a PTH functional region, a linker and an Fc region connected sequentially, the PTH functional region is selected from SEQ ID NOs: 5 and 33-37, the linker is selected from SEQ ID NOs: 9-12, and the Fc region sequence is SEQ ID NO: 40; or,
when the amino acid sequence of the first arm B chain comprises SEQ ID NO: 40, the amino acid sequence of the second arm comprises a PTH functional region, a linker and an Fc region connected sequentially, the PTH functional region is selected from SEQ ID NOs: 5 and 33-37, the linker is selected from SEQ ID NOs: 9-12, and the Fc region sequence is SEQ ID NO: 39.

9. The long-acting parathyroid hormone receptor agonist fusion protein according to claim 8, wherein,
when the amino acid sequence of the first arm A chain comprises SEQ ID NO: 2 and SEQ ID NO: 15 connected sequentially, the amino acid sequence of the first arm B chain comprises SEQ ID NO: 1, SEQ ID NO: 14 and SEQ ID NO: 7 connected sequentially; or, the amino acid sequence of the first arm A chain comprises SEQ ID NO: 2 and SEQ ID NO: 14 connected sequentially, and the amino acid sequence of the first arm B chain comprises SEQ ID NO: 1, SEQ ID NO: 15 and SEQ ID NO: 7 connected sequentially; or, the amino acid sequence of the first arm A chain comprises SEQ ID NO: 1 and SEQ ID NO: 14 connected sequentially, and the amino acid sequence of the first arm B chain comprises SEQ ID NO: 2, SEQ ID NO: 15 and SEQ ID NO: 7 connected sequentially; or, the amino acid sequence of the first arm A chain comprises SEQ ID NO: 1 and SEQ ID NO: 15 connected sequentially, and the amino acid sequence of the first arm B chain comprises SEQ ID NO: 2, SEQ ID NO: 14 and SEQ ID NO: 7 connected sequentially;
the amino acid sequence of the second arm comprises SEQ ID NO: 5, SEQ ID NO: 9 and SEQ ID NO: 8 connected sequentially, or SEQ ID NO: 5 and SEQ ID NO: 8 connected sequentially, or SEQ ID NO: 5, SEQ ID NO: 10 and SEQ ID NO: 8 connected sequentially, or SEQ ID NO: 5, SEQ ID NO: 11 and SEQ ID NO: 8 connected sequentially, or SEQ ID NO: 5, SEQ ID NO: 12 and SEQ ID NO: 8 connected sequentially, or SEQ ID NO: 33 and SEQ ID NO: 8 connected sequentially, or SEQ ID NO: 33, SEQ ID NO: 9 and SEQ ID NO: 8 connected sequentially, or SEQ ID NO: 33, SEQ ID NO: 10 and SEQ ID NO: 8 connected sequentially, or SEQ ID NO: 33, SEQ ID NO: 11 and SEQ ID NO: 8 connected sequentially, or SEQ ID NO: 33, SEQ ID NO: 12 and SEQ ID NO: 8 connected sequentially, or SEQ ID NO: 34 and SEQ ID NO: 8 connected sequentially, or SEQ ID NO: 34, SEQ ID NO: 9 and SEQ ID NO: 8 connected sequentially, or SEQ ID NO: 34, SEQ ID NO: 10 and SEQ ID NO: 8 connected sequentially, or SEQ ID NO: 34, SEQ ID NO: 11 and SEQ ID NO: 8 connected sequentially, or SEQ ID NO: 34, SEQ ID NO: 12 and SEQ ID NO: 8 connected sequentially, or SEQ ID NO: 35 and SEQ ID NO: 8 connected sequentially, or SEQ ID NO: 35, SEQ ID NO: 9 and SEQ ID NO: 8 connected sequentially, or SEQ ID NO: 35, SEQ ID NO: 10 and SEQ ID NO: 8 connected sequentially, or SEQ ID NO: 35, SEQ ID NO: 11 and SEQ ID NO: 8 connected sequentially, or SEQ ID NO: 35, SEQ ID NO: 12 and SEQ ID NO: 8 connected sequentially, or SEQ ID NO: 36 and SEQ ID NO: 8 connected sequentially, or SEQ ID NO: 36, SEQ ID NO: 9 and SEQ ID NO: 8 connected sequentially, or SEQ ID NO: 36, SEQ ID NO: 10 and SEQ ID NO: 8 connected sequentially, or SEQ ID NO: 36, SEQ ID NO: 11 and SEQ ID NO: 8 connected sequentially, or SEQ ID NO: 36, SEQ ID NO: 12 and SEQ ID NO: 8 connected sequentially, or SEQ ID NO: 37 and SEQ ID NO: 8 connected sequentially, or SEQ ID NO: 37, SEQ ID NO: 9 and SEQ ID NO: 8 connected sequentially, or SEQ ID NO: 37, SEQ ID NO: 10 and SEQ ID NO: 8 connected sequentially, or SEQ ID NO: 37, SEQ ID NO: 11 and SEQ ID NO: 8 connected sequentially, or SEQ ID NO: 37, SEQ ID NO: 12 and SEQ ID NO: 8 connected sequentially;
when the amino acid sequence of the first arm A chain comprises SEQ ID NO: 2 and SEQ ID NO: 15 connected sequentially, the amino acid sequence of the first arm B chain comprises SEQ ID NO: 1, SEQ ID NO: 14 and SEQ ID NO: 8 connected sequentially; or, the amino acid sequence of the first arm A chain comprises SEQ ID NO: 2 and SEQ ID NO: 14 connected sequentially, and the amino acid sequence of the first arm B chain comprises SEQ ID NO: 1, SEQ ID NO: 15 and SEQ ID NO: 8 connected sequentially; or, the amino acid sequence of the first arm A chain comprises SEQ ID NO: 1 and SEQ ID NO: 14 connected sequentially, and the amino acid sequence of the first arm B chain comprises SEQ ID NO: 2, SEQ ID NO: 15 and SEQ ID NO: 8 connected sequentially; or, the amino acid sequence of the first arm A chain comprises SEQ ID NO: 1 and SEQ ID NO: 15 connected sequentially, and the amino acid sequence of the first arm B chain comprises SEQ ID NO: 2, SEQ ID NO: 14 and SEQ ID NO: 8 connected sequentially;
the amino acid sequence of the second arm comprises SEQ ID NO: 5, SEQ ID NO: 9 and SEQ ID NO: 7 connected sequentially, or SEQ ID NO: 5 and SEQ ID NO: 7 connected sequentially, or SEQ ID NO: 5, SEQ ID NO: 10 and SEQ ID NO: 7 connected sequentially, or SEQ ID NO: 5, SEQ ID NO: 11 and SEQ ID NO: 7 connected sequentially, or SEQ ID NO: 33 and SEQ ID NO: 7 connected sequentially, or SEQ ID NO: 33, SEQ ID NO: 9 and SEQ ID NO: 7 connected sequentially, or SEQ ID NO: 33, SEQ ID NO: 10 and SEQ ID NO: 7 connected sequentially, or SEQ ID NO: 33, SEQ ID NO: 11 and SEQ ID NO: 7 connected sequentially, or SEQ ID NO: 33, SEQ ID NO: 12 and SEQ ID NO: 7 connected sequentially, or SEQ ID NO: 34 and SEQ ID NO: 7 connected sequentially, or SEQ ID NO: 34, SEQ ID NO: 9 and SEQ ID NO: 7 connected sequentially, or SEQ ID NO: 34, SEQ ID NO: 10, and SEQ ID NO: 7 connected sequentially, or SEQ ID NO: 34, SEQ ID NO: 11 and SEQ ID NO: 7 connected sequentially, or SEQ ID NO: 34, SEQ ID NO: 12 and SEQ ID NO: 7 connected sequentially, or SEQ ID NO: 35 and SEQ ID NO: 7 connected sequentially, or SEQ ID NO: 35, SEQ ID NO: 9 and SEQ ID NO: 7 connected sequentially, or SEQ ID NO: 35, SEQ ID NO: 10 and SEQ ID NO: 7 connected sequentially, or SEQ ID NO: 35, SEQ ID NO: 11 and SEQ ID NO: 7 connected sequentially, or SEQ ID NO: 35, SEQ ID NO: 12 and SEQ ID NO: 7 connected sequentially, or SEQ ID NO: 36 and SEQ ID NO: 7 connected sequentially, or SEQ ID NO: 36, SEQ ID NO: 9 and SEQ ID NO: 7 connected sequentially, or SEQ ID NO: 36, SEQ ID NO: 10 and SEQ ID NO: 7 connected sequentially, or SEQ ID NO: 36, SEQ ID NO: 11 and SEQ ID NO: 7 connected sequentially, or SEQ ID NO: 36, SEQ ID NO: 12 and SEQ ID NO: 7 connected sequentially, or SEQ ID NO: 37 and SEQ ID NO: 7 connected sequentially, or SEQ ID NO: 37, SEQ ID NO: 9 and SEQ ID NO: 7 connected sequentially, or SEQ ID NO: 37, SEQ ID NO: 10 and SEQ ID NO: 7 connected sequentially, or SEQ ID NO: 37, SEQ ID NO: 11 and SEQ ID NO: 7 connected sequentially, or SEQ ID NO: 37, SEQ ID NO: 12 and SEQ ID NO: 7 connected sequentially;
when the amino acid sequence of the first arm A chain comprises SEQ ID NO: 2 and SEQ ID NO: 15 connected sequentially, the amino acid sequence of the first arm B chain comprises SEQ ID NO: 1, SEQ ID NO: 14 and SEQ ID NO: 39 connected sequentially; or, the amino acid sequence of the first arm A chain comprises SEQ ID NO: 2 and SEQ ID NO: 14 connected sequentially, and the amino acid sequence of the first arm B chain comprises SEQ ID NO: 1, SEQ ID NO: 15 and SEQ ID NO: 39 connected sequentially; or, the amino acid sequence of the first arm A chain comprises SEQ ID NO: 1 and SEQ ID NO: 14 connected sequentially, and the amino acid sequence of the first arm B chain comprises SEQ ID NO: 2, SEQ ID NO: 15 and SEQ ID NO: 39 connected sequentially; or, the amino acid sequence of the first arm A chain comprises SEQ ID NO: 1 and SEQ ID NO: 15 connected sequentially, and the amino acid sequence of the first arm B chain comprises SEQ ID NO: 2, SEQ ID NO: 14 and SEQ ID NO: 39 connected sequentially;
the amino acid sequence of the second arm comprises SEQ ID NO: 5, SEQ ID NO: 9 and SEQ ID NO: 40 connected sequentially, or SEQ ID NO: 5 and SEQ ID NO: 40 connected sequentially, or SEQ ID NO: 5, SEQ ID NO: 10 and SEQ ID NO: 40 connected sequentially, or SEQ ID NO: 5, SEQ ID NO: 11, and SEQ ID NO: 40 connected sequentially, or SEQ ID NO: 5, SEQ ID NO: 12, and SEQ ID NO: 40 connected sequentially, or SEQ ID NO: 33 and SEQ ID NO: 40 connected sequentially, or SEQ ID NO: 33, SEQ ID NO: 9 and SEQ ID NO: 40 connected sequentially, or SEQ ID NO: 33, SEQ ID NO: 10 and SEQ ID NO: 40 connected sequentially, or SEQ ID NO: 33, SEQ ID NO: 11 and SEQ ID NO: 40 connected sequentially, or SEQ ID NO: 33, SEQ ID NO: 12 and SEQ ID NO: 40 connected sequentially, or SEQ ID NO: 34 and SEQ ID NO: 40 connected sequentially, or SEQ ID NO: 34, SEQ ID NO: 9 and SEQ ID NO: 40 connected sequentially, or SEQ ID NO: 34, SEQ ID NO: 10 and SEQ ID NO: 40 connected sequentially, or SEQ ID NO: 34, SEQ ID NO: 11 and SEQ ID NO: 40 connected sequentially, or SEQ ID NO: 34, SEQ ID NO: 12 and SEQ ID NO: 40 connected sequentially, or SEQ ID NO: 35 and SEQ ID NO: 40 connected sequentially, or SEQ ID NO: 35, SEQ ID NO: 9 and SEQ ID NO: 40 connected sequentially, or SEQ ID NO: 35, SEQ ID NO: 10 and SEQ ID NO: 40 connected sequentially, or SEQ ID NO: 35, SEQ ID NO: 11 and SEQ ID NO: 40 connected sequentially, or SEQ ID NO: 35, SEQ ID NO: 12 and SEQ ID NO: 40 connected sequentially, or SEQ ID NO: 36 and SEQ ID NO: 40 connected sequentially, or SEQ ID NO: 36, SEQ ID NO: 9 and SEQ ID NO: 40 connected sequentially, or SEQ ID NO: 36, SEQ ID NO: 10 and SEQ ID NO: 40 connected sequentially, or SEQ ID NO: 36, SEQ ID NO: 11 and SEQ ID NO: 40 connected sequentially, or SEQ ID NO: 36, SEQ ID NO: 12 and SEQ ID NO: 40 connected sequentially, or SEQ ID NO: 37 and SEQ ID NO: 40 connected sequentially, or SEQ ID NO: 37, SEQ ID NO: 9 and SEQ ID NO: 40 connected sequentially, or SEQ ID NO: 37, SEQ ID NO: 10 and SEQ ID NO: 40 connected sequentially, or SEQ ID NO: 37, SEQ ID NO: 11 and SEQ ID NO: 40 connected sequentially, or SEQ ID NO: 37, SEQ ID NO: 12 and SEQ ID NO: 40 connected sequentially;
when the amino acid sequence of the first arm A chain comprises SEQ ID NO: 2 and SEQ ID NO: 15 connected sequentially, the amino acid sequence of the first arm B chain comprises SEQ ID NO: 1, SEQ ID NO: 14 and SEQ ID NO: 40 connected sequentially; or, the amino acid sequence of the first arm A chain comprises SEQ ID NO: 2 and SEQ ID NO: 14 connected sequentially, and the amino acid sequence of the first arm B chain comprises SEQ ID NO: 1, SEQ ID NO: 15 and SEQ ID NO: 40 connected sequentially; or, the amino acid sequence of the first arm A chain comprises SEQ ID NO: 1 and SEQ ID NO: 14 connected sequentially, and the amino acid sequence of the first arm B chain comprises SEQ ID NO: 2, SEQ ID NO: 15 and SEQ ID NO: 40 connected sequentially; or, the amino acid sequence of the first arm A chain comprises SEQ ID NO: 1 and SEQ ID NO: 15 connected sequentially, and the amino acid sequence of the first arm B chain comprises SEQ ID NO: 2, SEQ ID NO: 14 and SEQ ID NO: 40 connected sequentially;
the amino acid sequence of the second arm comprises SEQ ID NO: 5, SEQ ID NO: 9, and SEQ ID NO: 39 connected sequentially, or SEQ ID NO: 5 and SEQ ID NO: 39 connected sequentially, or SEQ ID NO: 5, SEQ ID NO: 10 and SEQ ID NO: 39 connected sequentially, or SEQ ID NO: 5, SEQ ID NO: 11 and SEQ ID NO: 39 connected sequentially, or SEQ ID NO: 5, SEQ ID NO: 12 and SEQ ID NO: 39 connected sequentially, or SEQ ID NO: 33 and SEQ ID NO: 39 connected sequentially, or SEQ ID NO: 33, SEQ ID NO: 9 and SEQ ID NO: 39 connected sequentially, or SEQ ID NO: 33, SEQ ID NO: 10 and SEQ ID NO: 39 connected sequentially, or SEQ ID NO: 33, SEQ ID NO: 11 and SEQ ID NO: 39 connected sequentially, or SEQ ID NO: 33, SEQ ID NO: 12 and SEQ ID NO: 39 connected sequentially, or SEQ ID NO: 34 and SEQ ID NO: 39 connected sequentially, or SEQ ID NO: 34, SEQ ID NO: 9 and SEQ ID NO: 39 connected sequentially, or SEQ ID NO: 34, SEQ ID NO: 10 and SEQ ID NO: 39 connected sequentially, or SEQ ID NO: 34, SEQ ID NO: 11 and SEQ ID NO: 39 connected sequentially, or SEQ ID NO: 34, SEQ ID NO: 12 and SEQ ID NO: 39 connected sequentially, or SEQ ID NO: 35 and SEQ ID NO: 39 connected sequentially, or SEQ ID NO: 35, SEQ ID NO: 9 and SEQ ID NO: 39 connected sequentially, or SEQ ID NO: 35, SEQ ID NO: 10 and SEQ ID NO: 39 connected sequentially, or SEQ ID NO: 35, SEQ ID NO: 11 and SEQ ID NO: 39 connected sequentially, or SEQ ID NO: 35, SEQ ID NO: 12 and SEQ ID NO: 39 connected sequentially, or SEQ ID NO: 36 and SEQ ID NO: 39 connected sequentially, or SEQ ID NO: 36, SEQ ID NO: 9 and SEQ ID NO: 39 connected sequentially, or SEQ ID NO: 36, SEQ ID NO: 10 and SEQ ID NO: 39 connected sequentially, or SEQ ID NO: 36, SEQ ID NO: 11 and SEQ ID NO: 39 connected sequentially, or SEQ ID NO: 36, SEQ ID NO: 12 and SEQ ID NO: 39 connected sequentially, or SEQ ID NO: 37 and SEQ ID NO: 39 connected sequentially, or SEQ ID NO: 37, SEQ ID NO: 9 and SEQ ID NO: 39 connected sequentially, or SEQ ID NO: 37, SEQ ID NO: 10 and SEQ ID NO: 39 connected sequentially, or SEQ ID NO: 37, SEQ ID NO: 11 and SEQ ID NO: 39 connected sequentially, or SEQ ID NO: 37, SEQ ID NO: 12 and SEQ ID NO: 39 connected sequentially.

10. An isolated nucleic acid molecule encoding the long-acting parathyroid hormone receptor agonist fusion protein according to any one of claims 1 to 9.

11. A recombinant expression vector comprising the nucleic acid molecule according to claim 10.

12. An engineered cell comprising the recombinant expression vector according to claim 11; preferably, a host cell of the engineered cell is a eukaryotic cell; more preferably, the eukaryotic cell is a yeast cell or a mammalian cell; furthermore preferably, the mammalian cell is a CHO cell or a HEK293 cell.

13. A pharmaceutical composition comprising the long-acting parathyroid hormone receptor agonist fusion protein according to any one of claims 1 to 9, the nucleic acid molecule according to claim 10, the recombinant expression vector according to claim 11, and/or the engineered cell according to claim 12;
optionally, the pharmaceutical composition further comprises a pharmaceutically acceptable carrier and/or excipients.

14. A use of the long-acting parathyroid hormone receptor agonist fusion protein according to any one of claims 1 to 9, the nucleic acid molecule according to claim 10, the recombinant expression vector according to claim 11, the engineered cell according to claim 12 or the pharmaceutical composition according to claim 13 in the manufacture of a medicament for treatment, prevention, adjuvant treatment or neoadjuvant treatment of a bone disorder, wherein the bone disorder is a disease requiring treatment, prevention, adjuvant treatment or neoadjuvant treatment by regulating bone formation and/or bone resorption at lesion-associated sites;
preferably, the bone disorder is osteoporosis, osteopenia, osteogenesis imperfecta, transplantation-associated bone loss, autoimmune-induced bone loss, disuse-induced bone loss, bone injury, degenerative lumbar spondylolisthesis, or a degenerative intervertebral disc disease; the bone injury is preferably bone fracture, bone fissure, cartilage injury, osteonecrosis, or bone injury caused by arthritis or tumors; the bone fracture is preferably osteoporotic fracture, fracture nonunion, or delayed fracture union; the arthritis comprises arthritis caused by degeneration, trauma, overuse, infection, autoimmune diseases or inflammations, such as rheumatoid arthritis, osteoarthritis and infectious arthritis; and the tumors comprise bone tumors and tumor bone metastasis.

15. A method for preparing a long-acting parathyroid hormone receptor agonist fusion protein comprising a step of culturing the engineered cell according to claim 12.

16. A method for treatment, prevention, adjuvant treatment or neoadjuvant treatment of a bone disorder by regulating bone formation and/or bone resorption at lesion-associated sites, comprising administering to a subject in need thereof an effective amount of the long-acting parathyroid hormone receptor agonist fusion protein according to any one of claims 1 to 9, the nucleic acid molecule according to claim 10, the recombinant expression vector according to claim 11, the engineered cell according to claim 12 or the pharmaceutical composition according to claim 13;
preferably, the bone disorder is osteoporosis, osteopenia, osteogenesis imperfecta, transplantation-associated bone loss, autoimmune-induced bone loss, disuse-induced bone loss, bone injury, degenerative lumbar spondylolisthesis, or a degenerative intervertebral disc disease; the bone injury is preferably bone fracture, bone fissure, cartilage injury, osteonecrosis, or bone injury caused by arthritis or tumors; the bone fracture is preferably osteoporotic fracture, fracture nonunion, or delayed fracture union; the arthritis comprises arthritis caused by degeneration, trauma, overuse, infection, autoimmune diseases or inflammations, such as rheumatoid arthritis, osteoarthritis, and infectious arthritis; and the tumors comprise bone tumors and tumor bone metastasis;
and/or, the method of administration is selected from oral administration (e.g., administration via an oral delivery device) or injection; and the injection is preferably intravenous injection, subcutaneous injection, intramuscular injection, or local injection.

17. A use of the long-acting parathyroid hormone receptor agonist fusion protein according to any one of claims 1 to 9, the nucleic acid molecule according to claim 10, the recombinant expression vector according to claim 11, the engineered cell according to claim 12 or the pharmaceutical composition according to claim 13 as a medicament.

18. The long-acting parathyroid hormone receptor agonist fusion protein according to any one of claims 1 to 9, the nucleic acid molecule according to claim 10, the recombinant expression vector according to claim 11, the engineered cell according to claim 12 or the pharmaceutical composition according to claim 13 for use in treatment, prevention, adjuvant treatment or neoadjuvant treatment of a bone disorder;
preferably, the bone disorder is osteoporosis, osteopenia, osteogenesis imperfecta, transplantation-associated bone loss, autoimmune-induced bone loss, disuse-induced bone loss, bone injury, degenerative lumbar spondylolisthesis or a degenerative intervertebral disc disease; the bone injury is preferably bone fracture, bone fissure, cartilage injury, osteonecrosis, or bone injury caused by arthritis or tumors; the bone fracture is preferably osteoporotic fracture, fracture nonunion, or delayed fracture union; the arthritis comprises arthritis caused by degeneration, trauma, overuse, infection, autoimmune diseases or inflammations, such as rheumatoid arthritis, osteoarthritis and infectious arthritis; and the tumors comprise bone tumors and tumor bone metastasis.
